# FASCICULE DE BREVET EUROPEEN

(11) **EP 1 309 575 B1**
(45) Date de publication et mention de la délivrance du brevet: **08.06.2005**
(21) Numéro de dépôt: 01965314.6
(22) Date de dépôt: 03.08.2001
(51) Int. Cl.: C07D 277/34, C07C 235/52, A61K 31/195, C07D 207/40, C07C 237/22, A61K 31/21, C07D 417/12, A61K 31/426, A61K 31/16, C07C 233/87, A61K 31/427, A61P 17/00, C07C 233/51, A61K 31/4015

(54) **DERIVES DE BIPHENYLE ET LEUR UTILISATION COMME ACTIVATEURS RES RECEPTEURS PPAR-GAMMA**
BIPHENYLDERIVATE UND IHRE VERWENDUNG ALS AKTIVATOREN DES PPAR-GAMMA-REZEPTORS
BIPHENYL DERIVATIVES AND THEIR USE AS PPAR-GAMMA RECEPTOR ACTIVATORS

(30) Priorité: 08.08.2000 FR 0010447
(43) Date de publication de la demande: 14.05.2003
(73) Titulaire: Galderma Research & Development, 06560 Valbonne (FR)
(72) Inventeur: BERNARDON, Jean-Michel, F-06650 Le Rouret (FR); CLARY, Laurence, F-06700 St Laurent du Var (FR)
(74) Mandataire: Allab, Myriam
(86) Numéro de dépôt international: PCT/FR2001/002543
(87) Numéro de publication internationale: WO 2002/012210

(56) Documents cités:
- WO-A-99/46232
- TOMKINSON N C O ET AL: "Solid-phase synthesis of hybrid thiazolidinedione- fatty acid PPAR gamma ligands" BIOORGANIC & MEDICINAL CHEMISTRY LETTERS, vol. 7, no. 19, 7 octobre 1997 (1997-10-07), pages 2491-2496, XP004136471

## Description

L'invention concerne, à titre de produits industriels nouveaux et utiles, une nouvelle classe de composés bi-aromatiques activateurs des récepteurs de type Peroxisome Proliferator-Activated Receptor de sous-type γ (PPAR γ). Elle concerne également leur procédé de préparation et leur utilisation dans des compositions pharmaceutiques destinées à un usage en médecine humaine ou vétérinaire, ou bien encore dans des compositions cosmétiques.

L'activité des récepteurs de type PPARs a fait l'objet de nombreuses études. On peut citer à titre indicatif la publication intitulée "Differential Expression of Peroxisome Proliferator-Activated Receptor Subtypes During the Differentiation of Human Keratinocytes", Michel Rivier et al., J. Invest. Dermatol 111, 1998, p 1116-1121, dans laquelle est répertorié un grand nombre de références bibliographiques concernant les récepteurs de type PPARs. On peut également citer à titre indicatif, le dossier intitulé "The PPARs : From orphan receptors to Drug Discovery", Timothy M. Willson, Peter J. Brown, Daniel D.Sternbach, et Brad R. Henke, J. Med.Chem., 2000, Vol.43, p. 527-550.

Les récepteurs PPARs activent la transcription en se liant à des éléments de séquences d'ADN, appelés les éléments de réponse des proliférateurs de peroxysome (PPRE), sous forme d'un hétérodimère avec les récepteurs X des rétinoides (appelés les RXRs).

Trois sous-types de PPARs humains ont été identifiés et décrits : les PPARα, PPARγ et PPARδ (ou NUC1).
PPAR α est principalement exprimé dans le foie alors que PPAR δ est ubiquitaire.

PPAR γ est le plus étudié des trois sous-types. L'ensemble des références suggèrent un rôle critique des PPAR γ dans la régulation de la différentiation des adipocytes, où il est fortement exprimé. Il joue également un rôle clé dans l'homéostasie lipidique systémique.

Il a été notamment décrit dans la demande de brevet WO 96/33724 que des composés sélectifs des PPARγ, tels qu'une prostaglandine-J2 ou -D2, sont des actifs potentiels pour le traitement de l'obésité et du diabète.

Tomkinson et al., Bioorg. Med. Chem. Lett., 1997, vol. 7, p. 2491-6, décrivent des dérivés d'acides gras de 5-benzyl-2,4-thiazolidinedione comme ligands de PPARγ.
Par ailleurs, la Demanderesse a déjà décrit dans la demande de brevet FR98/02894 l'utilisation de composés activateurs de PPARγ dans la préparation d'une composition pharmaceutique, la composition étant destinée à traiter les désordres cutanés liés à une anomalie de la différentiation des cellules épidermiques.

Un des buts de la présente invention est de proposer une nouvelle classe de composés activateurs des PPARγ.

Ainsi, la présente invention concerne des composés répondant à la formule générale suivante : 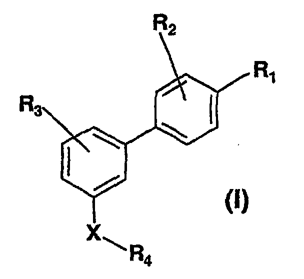 dans laquelle
- **R**_{**1**} représente un radical de formules **(a)** ou **(b)** suivantes: 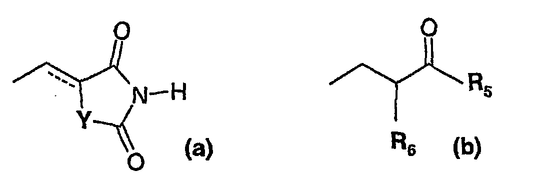 Y, R₅ et R₆ ayant les significations données ci-après,
- **R**_{**2**} et **R**_{**3**} identiques ou différents représentent un atome d'hydrogène, un radical alkyle ayant de 1 à 12 atomes de carbone, un radical aryle, un atome d'halogène, un radical - OR₇, un radical polyéther, un radical nitro, ou un radical amino pouvant éventuellement être substitué par un (ou plusieurs) radical alkyle ayant de 1 à 12 atomes de carbone(s),
   R₇ ayant la signification donnée ci-après,
- X représente une liaison de structure suivante:

   -(CH₂)ₘ-(Z)ₙ-(CO)ₚ-(W)_{q}-

   ladite liaison de structure pouvant être lue de gauche à droite ou inversement,
   Z, W, m, n, p, q ayant les significations données ci-après,
- **R**_{**4**} représente un radical alkyle ayant de 1 à 12 atomes de carbone, un radical aryle, un radical aralkyle, un radical hétéroaryle, ou un radical 9-fluorenylméthyle,
- Y représente un radical CH₂ ou un atome de soufre,
- R₅ représente un radical hydroxy, un radical alkoxy ayant de 1 à 6 atomes de carbone, un radical NH-OH, ou un radical N(R₈)(R₉),
   R₈ et R₉ ayant les significations données ci-après,
- R₆ représente un radical alkyle ayant de 1 à 12 atomes de carbone , un radical OR₁₀, un radical SR₁₀, ou un radical (CH₂)ᵣ-COR₁₁,
   r, R₁₀ et R₁₁ ayant les significations données ci-après,
- R₇ représente un atome d'hydrogène, un radical alkyle ayant de 1 à 12 atomes de carbone, ou un radical aralkyle,
- Z représente un atome d'oxygène, de soufre ou un radical N-R₁₂,
   R₁₂ ayant la signification donnée ci-après,
- W représente un atome d'oxygène, de soufre, un radical NR₁₃ ou un radical CH₂,
   R₁₃ ayant la signification donnée ci-après,
- m, n, p, q, identiques ou différents, peuvent prendre les valeurs 0 ou 1,
   étant entendu que la somme m+n+p+q est supérieure ou égale à 2 et que lorsque p prend la valeur 0 alors n ou q est égal à 0,
- R₈ représente un atome d'hydrogène ou un radical alkyle ayant de 1 à 12 atomes de carbone,
- R₉ représente un atome d'hydrogène, un radical alkyle ayant de 1 à 12 atomes de carbone ou un radical aryle,
- r représente 0 ou 1,
- R₁₀ représente un radical alkyle ayant de 1 à 12 atomes de carbone, un radical aryle, ou un radical aralkyle,
- R₁₁ représente un radical hydroxy, un radical OR₁₄, ou un radical N(R₁₅)(R₁₆),
- R₁₂ représente un atome d'hydrogène ou un radical alkyle ayant de 1 à 12 atomes de carbone,
- R₁₃ représente un atome d'hydrogène ou un radical alkyle ayant de 1 à 12 atomes de carbone,
- R₁₄ représente un radical alkyle ayant de 1 à 12 atomes de carbone, un radical aryle ou un radical aralkyle,
- R₁₅ représente un atome d'hydrogène ou un radical alkyle ayant de 1 à 12 atomes de carbone,
- R₁₆ représente un atome d'hydrogène, un radical alkyle ayant de 1 à 12 atomes de carbone, un radical aryle, un radical aralkyle ou un radical hétéroalkyle,
ainsi que les sels des composés de formule (I) lorsque R₁ contient une fonction acide carboxylique ainsi que les isomères optiques et géométriques desdits composés de formule (I).

Lorsque les composés selon l'invention se présentent sous forme d'un sel, il s'agit de préférence d'un sel d'un métal alcalin ou alcalino-terreux, ou encore d'un sel de zinc ou d'une amine organique.

Selon la présente invention, par radical alkyle ayant de 1 à 12 atomes de carbone, on entend un radical contenant 1 à 12 atomes de carbone et de préférence les radicaux méthyle, éthyle, isopropyle, butyle, tertiobutyle, hexyle, octyle, décyle, ou dodécyle.

Par radical polyéther, on entend un radical polyéther ayant de 1 à 6 atomes de carbone interrompu par au moins un atome d'oxygène tel que les radicaux méthoxyméthoxy, éthoxyméthoxy, ou méthoxyéthoxyméthoxy.

Par atome d'halogène, on entend un atome de fluor, de chlore, ou de brome.

Par radical alkoxy ayant de 1 à 6 atomes de carbone, on entend un radical contenant de un à six atomes de carbones tel que les radicaux méthoxy, éthoxy, isopropyloxy, tertio-butoxy, ou hexyloxy,

Par radical aryle, on entend un radical phényle pouvant être mono ou di-substitué par un atome d'halogène, un radical CF₃, un radical alkyle ayant de 1 à 12 atomes de carbone, un radical alkoxy ayant de 1 à 6 atomes de carbone, une fonction nitro, un radical polyéther, un radical hydroxyle éventuellement protégé par un groupe acétyle, benzoyle ou une fonction amino éventuellement protégée par un groupe acétyle, benzoyle ou éventuellement substituée par au moins un alkyle ayant de 1 à 12 atomes de carbone.
Par radical aralkyle, on entend un radical benzyle ou phénéthyle pouvant être mono ou di-substitué par un atome d'halogène, un radical CF₃, un radical alkyle ayant de 1 à 12 atomes de carbone, un radical hydroxyle, un radical alkoxy ayant de 1 à 6 atomes de carbone, une fonction nitro, un radical polyéther, un radical hydroxyle éventuellement protégé par un groupe acétyle, benzoyle ou une fonction amino éventuellement protégée par un groupe acétyle, benzoyle ou éventuellement substituée par au moins un alkyle ayant de 1 à 12 atomes de carbone.

Par radical hétéroaryle, on entend un radical pyridyle, furyle, thiényle, isoxazolyle éventuellement substitué par au moins un halogène, un alkyle ayant de 1 à 12 atomes de carbone , un alkoxy ayant de 1 à 6 atomes de carbone, une fonction nitro, un radical polyéther, un hydroxyle éventuellement protégé par un groupe acétyle, benzoyle ou une fonction amino éventuellement protégée par un groupe acétyle, benzoyle ou éventuellement substituée par au moins un alkyle ayant de 1 à 12 atomes de carbone.

Parmi les composés de formule (I) ci-dessus rentrant dans le cadre de la présente invention, on peut notamment citer les suivants (seuls ou en mélange) :
N-[4'-(2,4-Dioxo-thiazolidin-5-ylmethyl)-biphenyl-3-ylmethyl]-N-methyl-benzamide
5-{3'-[(Methyl-pyridin-2-yl-amino)-methyl]-biphenyl-4-ylmethyl}-thiazolidine-2,4-dione
N-[4'-(2,4-Dioxo-thiazolidin-5-ylmethyl)-biphenyl-3-ylmethyl]-benzamide
3-{3'-[(Benzoyl-methyl-amino)-methyl]-biphenyl-4-yl}-2-methyl-propionate d'éthyle
Acide 2-methyl-3-(3'-{[methyl-(1-phenyl-methanoyl)-amino]-methyl}-biphenyl-4-yl)-propionique
N-[4'-(2-Carbamoyl-propyl)-biphenyl-3-ylmethyl]-N-methyl-benzamide
N-Methyl-N-[4'-(2-phenylcarbamoyl-propyl)-biphenyl-3-ylmethyl]-benzamide
N-[4'-(2-Hydroxycarbamoyl-propyl)-biphenyl-3-ylmethyl]-N-methyl-benzamide
1-[4'-(2,4-Dioxo-thiazolidin-5-ylmethyl)-biphenyl-3-ylmethyl)-3-phenyl-urée
1-[4'-(2,4-Dioxo-thiazolidin-5-ylmethyl)-biphenyl-3-ylmethyl]-1-methyl-3-phenyl-urée
[4'-(2,4-Dioxo-thiazolidin-5-ylmethyl)-biphenyl-3-ylmethyl]-methyl-carbamate de tert-butyle
N-[4'-(2,4-dioxo-thiazolidin-5-ylmethyl)-biphenyl-3-ylmethyl]-N-methyl-nonanamide
Acide (S)-2-ethoxy-3-(3'-{[methyl-(1-phenyl-methanoyl)-amino]-methyl}-biphenyl-4-yl)-propionique
2-(3'-{[methyl-(1-phenyl-methanoyl)-amino]-methyl}-biphenyl-4-ylmethyl)-malonate de monométhyle
2-(3'-{[Methyl-(1-phenyl-methanoyl)-amino]-methyl}-biphenyl-4-ylmethyl)-malonate de diméthyle
2-(3'-{[Methyl-(1-phenyl-methanoyl)-amino]-methyl}-biphenyl-4-ylmethyl)-malonamate de méthyle
N-[4'-(2,4-Dioxo-thiazolidin-5-ylmethyl)-biphenyl-3-ylmethyl]-N-ethyl-benzamide
N-[4'-(2,4-Dioxo-thiazolidin-5-ylmethyl)-biphenyl-3-ylmethyl]-N-pentyl-benzamide
[4'-(2,4-Dioxo-thiazolidin-5-ylmethyl)-biphenyl-3-ylmethyl]-ethyl-carbamate de tert-butyle
[4'-(2,4-Dioxo-thiazolidin-5-ylmethyl)-biphenyl-3-ylmethyl]-propyl- carbamate de tert-butyle
[4'-(2,4-Dioxo-thiazolidin-5-ylmethyl)-biphenyl-3-ylmethyl]-methyl-carbamate de 9H-fluoren-9-ylmethyle
N-[4'-(2,4-Dioxo-thiazolidin-5-ylmethyl)-biphenyl-3-ylmethyl]-2,2,N-trimethyl-propionamide
N-octyl-4'-(2,4-Dioxo-thiazolidin-5-ylmethyl)-biphenyl-3-carboxamide
N-[4'-(2,4-Dioxo-thiazolidin-5-ylmethyl)-biphenyl-3-ylmethyl]-N-methyl-3-phenylpropionamide
2-[4'-(2,4-Dioxo-thiazolidin-5-ylmethyl)-biphenyl-3-yl]-N-methyl-N-phenyl-acetamide
N-[4'-(2,4-Dioxo-thiazolidin-5-ylidenemethyl)-biphenyl-3-ylmethyl]-N-propyl-benzamide
[4'-(2,4-dioxo-thiazolidin-5-ylméthyl)-biphenyl-3-yl]-carbamate de tert-butyle
N-[4'-(2,4-Dioxo-thiazolidin-5-ylmethyl)-biphenyl-3-ylmethyl]-3,4-diethoxy-N-methylbenzamide
Acide 2-(3'-{[Methyl-(1-phenyl-methanoyl)-amino]-methyl}-biphenyl-4-ylmethyl)-malonamique
N-benzyl-N-méthyl-4'-(2,4-Dioxo-thiazolidin-5-ylmethyl)-biphenyl-3-carboxamide
N-benzyl-4'-(2,4-Dioxo-thiazolidin-5-ylmethyl)-biphenyl-3-carboxamide
N-[4'-(2,4-dioxo-thiazolidin-5-ylmethyl)-biphenyl-3-ylmethyl]-N-methyl-décanamide
N-[4'-(2,4-Dioxo-thiazolidin-5-ylmethyl)-biphenyl-3-yl]-2-phenyl-acetamide
N-[4'-(2,4-dioxo-thiazolidin-5-ylmethyl)-biphenyl-3-ylmethyl]-N-methyl-octanamide
N-[4'-(2,4-dioxo-thiazolidin-5-ylmethyl)-biphenyl-3-ylmethyl]-N-methyl-heptanamide
Acide N-Hydroxy-2-(3'-{[methyl-(1-phenyl-methanoyl)-amino]-methyl}-biphenyl-4-ylmethyl)-malonamique
N-[4'-(2,4-Dioxo-thiazolidin-5-ylmethyl)-biphenyl-3-ylmethyl]-N-methyl-2-phenylacetamide
N-[4'-(2,4-Dioxo-thiazolidin-5-ylmethyl)-biphenyl-3-ylmethyl]-4-methoxy-N-methylbenzamide
N-[4'-(2,4-Dioxo-thiazolidin-5-ylmethyl)-biphenyl-3-ylmethyl]-3-methoxy-N-methylbenzamide
N-[4'-(2,4-Dioxo-thiazolidin-5-ylmethyl)-biphenyl-3-ylmethyl]-3 -N-dimethyl-benzamide
N-[4'-(2,4-Dioxo-thiazolidin-5-ylmethyl)-biphenyl-3-ylmethyl]-N-methyl-4-propylbenzamide
N-[4'-(2,4-Dioxo-thiazolidin-5-ylmethyl)-biphenyl-3-ylmethyl]-4,N-dimethyl-benzamide.
N- [4'-(2,4-dioxo-thiazolidin-5-ylmethyl)-biphenyl-3-ylmethyl]-N-methyl- lsoxazole-5-carboxamide
N-[4'-(2,4-Dioxo-thiazolidin-5-ylmethyl)-biphenyl-3-ylmethyl]-4-ethoxy-N-methylbenzamide
N-[4'-(2,4-Dioxo-thiazolidin-5-ylmethyl)-biphenyl-3-ylmethyl]-4-fluoro-N-methyl-benzamide
4-Dimethylamino-N-[4'-(2,4-dioxo-thiazolidin-5-ylmethyl)-biphenyl-3-ylmethyl]-N-methylbenzamide
N-[4'-(2,4-Dioxo-thiazolidin-5-ylmethyl)-biphenyl-3-ylmethyl]-N-methyl-nicotinamide
3,5-Dichloro-N-[4'-(2,4-dioxo-thiazolidin-5-ylmethyl)-biphenyl-3-ylmethyl]-N-methylbenzamide
N- [4'-(2,4-dioxo-thiazolidin-5-ylmethyl)-biphenyl-3-ylmethyl]-N-methyl-Thiophene-2-carboxamide
N- [4'-(2,4-dioxo-thiazolidin-5-ylmethyl)-biphenyl-3-ylmethyl]-N-methyl- Hexanamide
N-[4'-(2,4-Dioxo-thiazolidin-5-ylmethyl)-biphenyl-3-ylmethyl]-2-methoxy-N-methylbenzamide
N-[4'-(2,4-dioxo-thiazolidin-5-ylmethyl)-biphenyl-3-ylmethyl]-N-methyl- pyridine-2-carboxamide
N- [4'-(2,4-dioxo-thiazolidin-5-ylmethyl)-biphenyl-3-ylmethyl]-N-methyl- furan-2-carboxamide
4-Butoxy-N-[4'-(2,4-dioxo-thiazolidin-5-ylmethyl)-biphenyl-3-ylmethyl]-N-methylbenzamide
N- [4'-(2,4-dioxo-thiazolidin-5-ylmethyl)-biphenyl-3-ylmethyl]-N-methyl- Thiophene-3-carboxamide
Acetate de 4-{[4'-(2,4-dioxo-thiazolidin-5-ylmethyl)-biphenyl-3-ylmethyl]-methylcarbamoyl}-phenyl
N-[4'-(2,4-Dioxo-thiazolidin-5-ylmethyl)-biphenyl-3-ylmethyl]-4-hydroxy-N-methylbenzamide
N-[4'-(2,4-Dioxo-thiazolidin-5-ylmethyl)-biphenyl-3-ylmethyl]-2 N-dimethyl-benzamide
2-Butyl-N- [4'-(2,4-dioxo-thiazolidin-5-ylmethyl)-biphenyl-3-ylmethyl]-N-methyloctanamide
4-Acetylamino-N-[4'-(2,4-dioxo-thiazolidin-5-ylmethyl)-biphenyl-3-ylmethyl]-N-methylbenzamide
N-[4'-(2,4-Dioxo-thiazolidin-5-ylmethyl)-biphenyl-3-ylmethyl]-N-methyl-carbamate d'hexyle
1-[4'-(2,4-Dioxo-thiazolidin-5-ylmethyl)-biphenyl-3-yl]-3-phenyl-uree
N-[4'-(2,4-Dioxo-thiazolidin-5-ylmethyl)-biphenyl-3-yl]-N-methyl-2-phenyl-acetamide
1-[4'-(2,4-Dioxo-thiazolidin-5-ylmethyl)-biphenyl-3-yl]-1-methyl-3-phenyl-uree
1-[4'-(2,4-Dioxo-thiazolidin-5-ylmethyl)-biphenyl-3-yl]-3-heptyl-1-methyl-uree
Ester 4-monométhylique de l'acide 2-{3'-[(Heptanoyl-methyl-amino)-methyl]-biphenyl-4-ylmethyl}-succinique
Acide 2-{3'-[(Methyl-octanoyl-amino)-methyl]-biphenyl-4-ylmethyl}-succinique
N- [4'-(2,5-dioxo-pyrrolidin-3-ylmethyl)-biphenyl-3-ylmethyl]-N-methyl-heptanamide.
N-Phenyl-carbamate de 3-[4'-(2,4-dioxo-thiazolidin-5-ylidenemethyl)]biphenyle
N-Heptyl-carbamate de 3-[4'-(2,4-dioxo-thiazolidin-5-ylidenemethyl)]biphenyle
Phenyl-acetate de 3-[4'-(2,4-dioxo-thiazolidin-5-ylmethyl)]biphenyle
Nonanoate de 3-[4'-(2,4-dioxo-thiazolidin-5-ylmethyl)]biphenyle
N-Heptyl-carbamate de 3-[4'-(2,4-dioxo-thiazolidin-5-ylmethyl)]biphenyle
N-Phenyl-carbamate de 3-[4'-(2,4-dioxo-thiazolidin-5-ylmethyl)]biphenyle
N-[6-benzyloxy-4'-(2,4-dioxo-thiazolidin-5-ylidenemethyl)-biphenyl-3-ylmethyl]-N-methyloctanamide
N-[4'-(2,4-dioxo-thiazolidin-5-ylmethyl)-6-hydroxy-biphenyl-3-ylmethyl]-N-methyloctanamide
N-[4-benzyloxy-4'-(2,4-dioxo-thiazolidin-5-ylidenemethyl)-biphenyl-3-ylmethyl]-N-methyloctanamide
N-[4"-(2,4-dioxo-thiazolidin-5-ylmethyl)-[1,1';3',1"]terphenyl-5'-ylmethyl]-N-methyloctanamide
N-[4'-(2,4-dioxo-thiazolidin-5-ylmethyl)-2'-methyl-biphenyl-3-ylmethyl]-N-methyloctanamide
N-[4'-(2,4-dioxo-thiazolidin-5-ylmethyl)-3'-methyl-biphenyl-3-ylmethyl]-N-methyloctanamide
Acide (S)-2-Ethoxy-3-{3'-[(methyl-octanoyl-amino)-methyl]-biphenyl-4-yl}-propionique
1-[4'-(2,4-Dioxo-thiazolidin-5-ylmethyl)-biphenyl-3-yl]-1-ethyl-3-phenyl-urée
N-[4'-(2,4-dioxo-thiazolidin-5-ylmethyl)-biphenyl-3-yl]-N-methyl-docanamide
N-[4'-(2,4-dioxo-thiazolidin-5-ylmethyl)-biphenyl-3-yl]-N-methyl-nonanamide
N-[4'-(2,4-Dioxo-thiazolidin-5-ylmethyl)-biphenyl-3-yl]-N-methyl-2-(4-butoxy-phenyl)-acetamide
N-[4'-(2,4-Dioxo-thiazolidin-5-ylmethyl)-biphenyl-3-yl]-N-methyl-2-(4-methoxy-phenyl)-acetamide
N-[4'-(2,4-Dioxo-thiazolidin-5-ylmethyl)-biphenyl-3-yl]-N-methyl-2-(4-ethoxy-phenyl)-acetamide
N-[4'-(2,4-Dioxo-thiazolidin-5-ylmethyl)-biphenyl-3-yl]-N-methyl-2-(4-hydroxy-phenyl)-acetamide
1-[4'-(2,4-Dioxo-thiazolidin-5-ylmethyl)-biphenyl-3-yl]-1-methyl-3-(4-butoxy-phenyl)-uree
1-[4'-(2,4-Dioxo-thiazolidin-5-ylmethyl)-biphenyl-3-yl]-1-methyl-3-(4-methoxy-phenyl)-uree
1-[4'-(2,4-Dioxo-thiazolidin-5-ylmethyl)-biphenyl-3-yl]-1-methyl-3-(4-ethoxy-phenyl)-uree
1-[4'-(2,4-Dioxo-thiazolidin-5-ylmethyl)-biphenyl-3-yl]-1-methyl-3-(4-hydroxy-phenyl)-uree
Acide (S)-2-ethoxy-3-(3'-{[methyl-(1-(4-butoxy)-phenylmethanoyl)-amino]-methyl}biphenyl-4-yl)-propionique
Acide (S)-2-ethoxy-3-(3'-{[methyl-(1-(4-methoxy)-phenylmethanoyl)-amino]-methyl}biphenyl-4-yl)-propionique
Acide (S)-2-ethoxy-3-(3'-{[methyl-(1-(4-ethoxy-phenyl)-methanoyl)-amino]-methyl}biphenyl-4-yl)-propionique
Acide (S)-2-ethoxy-3-(3'-{[methyl-(1-(4-hydroxy-phenyl)-methanoyl)-amino]-methyl}biphenyl-4-yl)-propionique
Acide (S)-2-phenoxy-3-(3'-{[methyl-(1-(4-butoxy-phenyl)-methanoyl)-amino]-methyl}biphenyl-4-yl)-propionique
Acide (S)-2-phenoxy-3-(3'-{[methyl-(1-(4-methoxy-phenyl)-methanoyl)-amino]-methyl}biphenyl-4-yl)-propionique
Acide (S)-2-phenoxy-3-(3'-{[methyl-(1-(4-ethoxy-phenyl)-methanoyl)-amino]-methyl}biphenyl-4-yl)-propionique
Acide (S)-2-phenoxy-3-(3'-{[methyl-(1-(4-hydroxy-phenyl)-methanoyl)-amino]-methyl}biphenyl-4-yl)-propionique
Acide (S)-2-phenoxy-3-(3'-{[methyl-(1-phenyl-methanoyl)-amino]-methyl}-biphenyl-4-yl)-propionique
Acide (S)-2-phenoxy-3-{3'-[(methyl-octanoyl-amino)-methyl]-biphenyl-4-yl}-propionique
5-{3'-[Methyl-(2-oxo-2-phenyl-ethyl)-amino]-biphenyl-4-ylmethyl}-thiazolidine-2,4-dione
5-[3'-(Methyl-phenethyl-amino)-biphenyl-4-ylmethyl]-thiazolidine-2,4-dione
[4'-(2,4-Dioxo-thiazolidin-5-ylmethyl)-biphenyl-3-yl]-methyl-carbamate de phenyle
[4'-(2,4-Dioxo-thiazolidin-5-ylmethyl)-biphenyl-3-yl]-methyl-carbamate de tert-butyle

Selon la présente invention les composés de formule (I) plus particulièrement préférés sont ceux qui présentent l'une au moins des caractéristiques suivantes :
- R₁ représente le radical de formule (a) ou le radical de formule (b) où R₅ représente un radical hydroxy et R₆ représente le radical OR_{10,}
- X représente une liaison de structure choisie parmi -CH₂-N(R₁₂)-CO-, ou -NR12-(CO)-NR13, ou -NR12-(CO)-CH2-, ces liaisons étant lues de gauche à droite ou inversement.

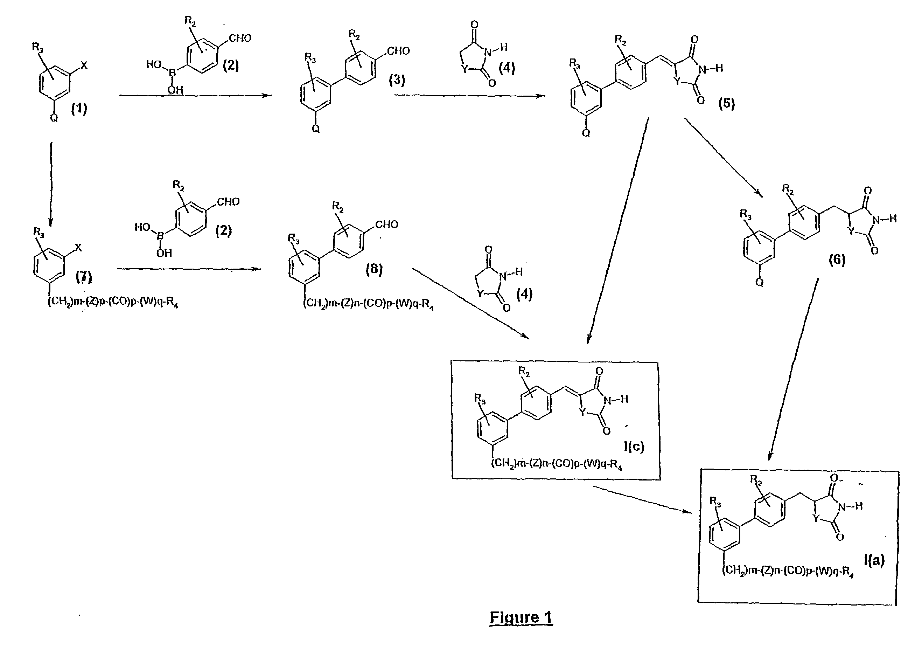

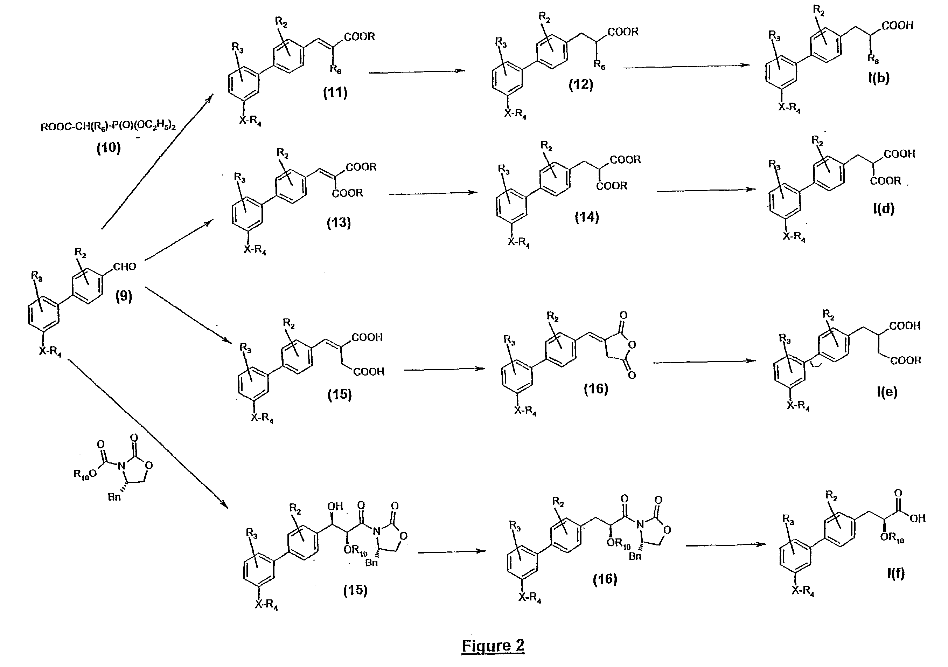

La présente invention a également pour objet les procédés de préparation des composés de formule **(I)**, en particulier selon les schémas réactionnels donnés aux figures 1 et 2.
Les dérivés de formule **(la)** peuvent être obtenus **(Figure 1)** à partir des dérivés **(Ic)** par hydrogénation en présence de Palladium sur charbon dans un solvant tel le dioxanne, l'acétate d'éthyle, le DMF ou l'alcool éthylique.

Les dérivés de formule **(Ia)** peuvent être aussi obtenus **(Figure 1)** à partir des dérivés thiazolidine-2,4-dione **(6)** :
- lorsque Q représente -(CH₂)ₘ-ZH par réaction avec un halogénure d'acyle de formule Cl-CO-(W)_{q}-R₄ en présence d'une base telle la triéthylamine dans un solvant tel le THF ou le dichlorométhane ou par réaction avec un isocyanate de formule O=C=N-R₄.
lorsque Q représente -(CH₂)ₘ-COOH en faisant réagir en milieu anhydre dans un solvant organique de préférence le THF ou le dichlorométhane et en présence d'une amine tertiaire (par exemple triéthylamine ou pyridine) une forme activée de la fonction acide par exemple un chlorure d'acide sur un dérivé amine, hydroxy ou thiol de formule HW-R₄
Les dérivés de formule **(6)** peuvent être obtenus **(Figure 1)** à partir des dérivés de formule **(5)** par hydrogénation en présence de Palladium sur charbon dans un solvant tel le dioxanne, l'acétate d'éthyle, le DMF ou l'alcool éthylique.
Les composés **(5)** et **(Ic)** peuvent être respectivement obtenus **(Figure 1)** à partir des composés **(3)** et **(8)** par réaction avec la 2,4-thiazolidinedione en présence d'acétate de pipéridine dans un solvant alcoolique tel l'éthanol ou dans le toluène. Les composés **(3)** et **(8)** peuvent être respectivement obtenus **(Figure 1)** à partir des dérivés halogénés **(1)** et **(7 )**, de préférence iodés ou bromés par une réaction de couplage de type Suzuki avec un acide boronique **(2)**. Cette réaction est effectuée en présence d'un catalyseur au palladium, par exemple le tétrakis(triphénylphosphine)palladium selon les conditions décrites par N. Miyaura et al., Synthetic Communications (1981) 11(7), 513-519.
Les dérivés boroniques **(2)** pouvant être obtenus à partir des dérivés halogénés correspondants (de préférence iodés ou bromés) tout d'abord par protection de la fonction aldéhyde sous forme d'acétal puis transformation en lithien, réaction avec le triméthyl borate et hydrolyse.
Les dérivés de formule **(Ib)** peuvent être obtenus **(Figure 2)** à partir des dérivés aldéhydiques **(9)**, selon une réaction de type Horner avec un lithio ou sodio dérivé d'un phosphonate **(10)** puis hydrogénation en présence de palladium sur charbon et saponification de l'ester en acide.
Les dérivés de formule **(Id)** peuvent être obtenus **(Figure 2)** par une suite de réactions selon les conditions décrites par S. Doulut et al., J. Med. Chem. (1993) 36, 1369-1379.
Les dérivés de formule **(Ie)** peuvent être obtenus **(Figure 2)** par une suite de réactions selon les conditions décrites par H. Shinkai et al., J. Med. Chem. (1998) 41, 1927-1933.
Les dérivés de formule **(If)** peuvent être obtenus **(Figure 2)** par une suite de réactions selon les conditions décrites par B. Hulin et al., J. Med. Chem. (1996) 39, 3897-3907.
Lorsque R₁ comporte une fonction acide, les composés sont préparés en protégeant R₁ par un groupe protecteur de type alkyle ayant de 1 à 12 atomes de carbone, allylique, benzylique ou tert-butylique.
Le passage à la forme libre peut-être effectué:
- dans le cas d'un groupe protecteur alkyle ayant de 1 à 12 atomes de carbone, au moyen de soude ou d'hydroxyde de lithium dans un solvant alcoolique tel le méthanol ou dans le THF.
- dans le cas d'un groupe protecteur allylique, au moyen d'un catalyseur tel certains complexes de métaux de transition en présence d'une amine secondaire telle la morpholine.
- dans le cas d'un groupe protecteur benzylique, par débenzylation en présence d'hydrogène au moyen d'un catalyseur tel que le palladium sur charbon.
- dans le cas d'un groupe protecteur de type tert-butylique au moyen d'iodure de triméthylsilane.

Les composés selon l'invention présentent des propriétés d'activation des récepteurs de type PPARγ.

Par activateur des récepteurs de type PPARγ, on entend selon l'invention tout composé qui présente, à la concentration de 1 µM, dans un test de transactivation, tel que décrit dans Kliewer et al., Nature 358, 771-774, 1992, un pourcentage d'activation des récepteurs PPARγ d'au moins 20 %, calculé par rapport à un composé de référence,le SB 219994, activant les PPARγ de 100%.

De préférence, l'activateur des récepteurs de type PPARγ présente un pourcentage d'activation supérieur ou égal à 40% et avantageusement supérieur ou égal à 70%.

De préférence, l'activateur des récepteurs de type PPAR-γ est spécifique, c'est à dire qu'il présente un rapport du pourcentage d'activation des récepteurs PPARγ, au pourcentage d'activation des récepteurs PPAR α (calculé par rapport à un composé de référence,le Wy 14643, activant les PPARα de 100%) supérieur ou égal à 3. De préférence, ce rapport est supérieur ou égal à 5 et plus avantageusement supérieur ou égal à 10 .

L'affinité des dérivés PPARs pour le récepteur PPARγ humain a également été déterminée dans un test de binding, par compétition de la fixation d'un agoniste de référence, le BRL 49,653 tritié. La technique d'adsorption sur gel d'hydroxylapatite a été utilisée pour séparer le ligand lié au récepteur du ligand libre. Le récepteur PPARγ humain a été préparé à partir de cellules d'insecte Sf9 infectées avec un baculovirus recombinant. Les résultats sont exprimés en valeur de Kd (nM) qui représente la constante de dissociation à l'équilibre obtenue pour chaque composé. Par ligand des récepteur PPARγ, on entend tout composé selon l'invention présentant une valeur de Kd inférieure à 10000 nM. De préférence, les composés selon l'invention présentent une valeur de Kd inférieure à 1000 nM et avantageusement inférieure à 100 nM.

La présente invention a également pour objet à titre de médicament les composés de formule (I) tels que décrits ci-dessus.

Les composés selon l'invention conviennent particulièrement bien dans les domaines de traitement suivants :
1) pour traiter les affections dermatologiques liées à un désordre de la kératinisation portant sur la différenciation et sur la prolifération, notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides, le lymphome **T**, et les proliférations pouvant être induites par les ultra-violets notamment dans le cas des épithélioma baso et spinocellulaires, ainsi que toute lésion précancéreuse cutanées telles que les kératoacanthomes;
5) pour traiter d'autres désordres dermatologiques tels que les dermatoses immunes telles le lupus érythémateux, les maladies immunes bulleuses et les maladies du collagène, telle la sclérodermie,
6) dans le traitement d'affections dermatologiques ou générales à composante immunologique,
7) dans le traitement de désordres cutanés dus à une exposition aux rayonnements U.V. ainsi que pour réparer ou lutter contre le vieillissement de la peau, qu'il soit photo-induit ou chronologique, ou pour réduire les pigmentations et les kératoses actiniques, ou toutes pathologies associées au vieillissement chronologique ou actinique, telle la xérose,
8) pour lutter contre les troubles de la fonction sébacée tels que l'hyperséborrhée de l'acné ou la séborrhée simple,
9) pour prévenir ou traiter les troubles de la cicatrisation, ou pour prévenir ou pour réparer les vergetures,
10) dans le traitement des désordres de la pigmentation, tel l'hyperpigmentation, le mélasma, l'hypopigmentation ou le vitiligo.
11 ) dans le traitement des affections du métabolisme des lipides, tel l'obésité, l'hyperlipidémie, ou le diabète non insulino-dépendant.
12) dans le traitement d'affections inflammatoires telles que l'arthrite,
13) dans le traitement ou la prévention des états cancéreux ou précancéreux,
14) dans la prévention ou le traitement de l'alopécie de différentes origines, notamment l'alopécie due à la chimiothérapie ou aux rayonnements,
15) dans le traitement des troubles du systèmes immunitaire, tel l'asthme, le diabète sucré de type I, la sclérose en plaque, ou autres disfonctionnements sélectifs du système immunitaire.
16) dans le traitement d'affections du système cardiovasculaire telles que l'artériosclérose ou l'hypertension.

La présente invention a également pour objet une composition pharmaceutique ou cosmétique comprenant, dans un milieu physiologiquement acceptable, au moins un composé de formule (I) tel que défini ci-dessus.

La présente invention a aussi pour objet l'utilisation des composés de formule (I) pour fabriquer une composition destinée au traitement des affections susmentionées, en particulier pour réguler et/ou restaurer le métabolisme des lipides cutanés.

L'administration de la composition selon l'invention peut être effectuée par voie entérale, parentérale topique ou oculaire. De préférence, la composition pharmaceutique est conditionnée sous une forme convenant à une application par voie topique.

Par voie entérale, la composition, plus particulièrement la composition pharmaceutique, peut se présenter sous formes de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, la composition peut se présenter sous forme de solutions ou suspensions pour perfusion ou pour injection.

Les composés selon l'invention sont généralement administrés à une dose journalière d'environ 0,001 mg/kg à 100 mg/kg de poids corporel, en 1 à 3 prises.

Les composés sont utilisés par voie systémique à une concentration généralement comprise entre 0,001% et 10% en poids, de préférence entre 0,01 et 1 % en poids, par rapport au poids de la composition.

Par voie topique, la composition pharmaceutique selon l'invention est plus particulièrement destinée au traitement de la peau et des muqueuses et peut se présenter sous forme d'onguents, de crèmes, de laits, de pommades, de poudres, de tampons imbibés, de solutions, de gels, de sprays, de lotions ou de suspensions. Elle peut également se présenter sous forme de microsphères ou nanosphères ou vésicules lipidiques ou polymériques ou de patches polymériques et d'hydrogels permettant une libération contrôlée. Cette composition par voie topique peut se présenter sous forme anhydre, sous forme aqueuse ou sous la forme d'une émulsion.

Les composés sont utilisés par voie topique à une concentration généralement comprise entre 0,001 % et 10 % en poids, de préférence entre 0,01 et 1 % en poids, par rapport au poids total de la composition.

Les composés de formule (I) selon l'invention trouvent également une application dans le domaine cosmétique, en particulier dans l'hygiène corporelle et capillaire et plus particulièrement pour réguler et/ou restaurer le métabolisme des lipides cutanés. Par rapport aux produits connus antérieurement, ces composés de formule (I) ont l'avantage de présenter en plus d'autres propriétés intéressantes, notamment des propriétés anti-inflammatoires ou apaisantes, ce qui en fait des composés moins irritants et donc mieux tolérés.

L'invention a donc également pour objet l'utilisation cosmétique d'une composition comprenant, dans un support physiologiquement acceptable, au moins un des composés de formule (I) pour l'hygiène corporelle ou capillaire.

La composition cosmétique selon l'invention contenant, dans un support cosmétiquement acceptable, au moins un composé de formule (I) ou l'un de ses isomères optiques ou géométriques ou l'un de ses sels, peut se présenter notamment sous forme d'une crème, d'un lait, d'une lotion, d'un gel, de microsphères ou nanosphères ou vésicules lipidiques ou polymériques, d'un savon ou d'un shampooing.

La concentration en composé de formule (I) dans la composition cosmétique est comprise entre 0,001 et 3 % en poids, par rapport au poids total de la composition.

Les compositions telles que décrites précédemment peuvent bien entendu en outre contenir des additifs inertes ou même pharmacodynamiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des agents antifongiques tels que le kétoconazole ou les polyméthylène-4,5 isothiazolidones-3; des antibactériens, des caroténoïdes et, notamment, le p-carotène; des agents anti-psoriatiques tels que l'anthraline et ses dérivés; les acides eicosa-5,8,11,14-tétraynoïque et eicosa-5,8,11-triynoïque, leurs esters et amides et enfin les rétinoïdes. Les composés de formule (I) peuvent également être combinés avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques.

Ces compositions peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

On va maintenant donner, à titre d'illustration et sans aucun caractère limitatif, plusieurs exemples d'obtention de composés actifs de formule (I) selon l'invention, ainsi que des résultats d'activité biologiques de tels composés et diverses formulations concrètes à base de ses composés.

### EXEMPLE 1

### N-[4'-(2,4-Dioxo-thiazolidin-5-ylmethyl)-biphenyl-3-ylmethyl]-N-methylbenzamide.

### (a) (3-Bromo-benzyl)-carbamate de tert-butyle.

Dans un ballon et sous courant d'azote, on introduit 15 g (67 mmol) de chlorhydrate de 3-bromobenzylamine, 9,4 ml de triéthylamine (67 mmol) et 150 ml de dichlorométhane. On ajoute par petites quantités à température ambiante 15,5 ml (67 mmol) de dicarbonate de di tert-butyle et agite pendant trois heures. On verse le milieu réactionnel dans l'eau glacée, extrait avec du dichlorométhane, décante la phase organique, sèche sur sulfate de magnésium, évapore. On recueille 19,3 g (100%) de produit attendu.

### (b) (3-Bromo-benzyl)-N-méthylcarbamate de tert-butyle.

Dans un ballon et sous courant d'azote, on introduit 19,3 g (67 mmol) de (3-Bromo-benzyl)-carbamate de tert-butyle et 200 ml de THF. On ajoute par petites quantités 3 g (74 mmol) d'hydrure de sodium (60% dans l'huile) et agite jusqu'à cessation du dégagement gazeux. On ajoute ensuite 4,6 ml (74 mmol) d'iodure de méthyle et agite pendant une heure. On verse le milieu réactionnel dans l'eau glacée, extrait avec de l'acétate d'éthyle, décante la phase organique, sèche sur sulfate de magnésium, évapore. On recueille 20,4 g (100%) de produit attendu.

### (c) (3-Bromo-benzyl)-methyl-amine

Dans un ballon et sous courant d'azote, on introduit 20,2 g (67 mmol) de (3-Bromo-benzyl)-*N*-méthylcarbamate de tert-butyle dans 100 ml de dichlorométhane et ajoute 26 ml (335 mmol) d'acide trifluoroacétique . On agite à température ambiante pendant huit heures et hydrolyse le milieu réactionnel avec une solution saturée de carbonate de potassium. On extrait avec du dichlorométhane, décante la phase organique, lave à l'eau, sèche sur sulfate de magnésium, évapore. Le résidu obtenu est purifié par chromatographie sur colonne de silice élué avec un mélange de dichlorométhane et de méthanol (50-50). Après évaporation des solvants, on recueille 9 g (67%) du produit attendu.

### (d) N-(3-Bromo-benzyl)-N-methyl-benzamide

Dans un ballon et sous courant d'azote, on introduit 9 g (45 mmol) de (3-Bromo-benzyl)-methyl-amine, 90 ml de THF et 6,9 ml (50 mmol) de triéthylamine. On ajoute goutte à goutte 5,7 ml (50 mmol) de chlorure de benzoyle et agite pendant une heure. On verse le milieu réactionnel dans l'eau, extrait avec du dichlorométhane, décante la phase organique, lave à l'eau, sèche sur sulfate de magnésium, évapore. Le résidu obtenu est purifié par chromatographie sur colonne de silice élué avec un mélange d'acétate d'éthyle et d'heptane (15-85). Après évaporation des solvants, on recueille 13,7 g (64%) du produit attendu.

### (e) N-(4'-Formyl-biphenyl-3-ylmethyl)-N-methyl-benzamide

Dans un tricol et sous argon, on introduit 8,8 g (29 mmol) de *N*-(3-Bromobenzyl)-*N*-methyl-benzamide, 8,7 g (58 mmol) d'acide 4-formylbenzèneboronique et 125 ml de toluène. On ajoute goutte à goutte 36 ml (72 mmol) d'une solution aqueuse de carbonate de potassium (2M), dégaze le milieu réactionnel avec de l'argon et ajoute 1 g de chlorure de tétrakistriphénylphosphinepalladium(0) et chauffe à 90°C pendant 24 heures. On verse le milieu réactionnel dans l'eau, extrait avec du dichlorométhane, décante la phase organique, sèche sur sulfate de magnésium, évapore. Le résidu obtenu est purifié par chromatographie sur colonne de silice élué avec un mélange d'heptane et d'acétate d'éthyle (50-50). Après évaporation des solvants, on recueille 7,2 g (75%) du produit attendu.

### (f) N-[4'-(2,4-Dioxo-thiazolidin-5-ylidenemethyl)-biphenyl-3-ylmethyl]-N-methylbenzamide

Dans un ballon et sous courant d'azote, on introduit 1,6 g (4,6 mmol) de N-(4'-Formyl-biphenyl-3-ylmethyl)-N-methyl-benzamide, 610 mg (4,6 mmol) de 2,4-thiazolidinedione, 137 mg d'acétate de pipéridine et 60 ml de toluène. On chauffe à reflux pendant cinq heures et sépare l'eau formée à l'aide d'un dean-stark. On refroidit le milieu réactionnel, filtre le précipité formé et le purifie sur colonne de silice avec un mélange éluant d'heptane et d'acétate d'éthyle (70-30). On recueille après évaporation des solvants 1,4 g (70%) du produit attendu.

### (g) N-[4'-(2,4-Dioxo-thiazolidin-5-ylmethyl)-biphenyl-3-ylmethyl]-N-methylbenzamide

Dans un tricol, on introduit 1,4 g (3,3 mmol) de *N*-[4'-(2,4-Dioxo-thiazolidin-5-ylidenemethyl)-biphenyl-3-ylmethyl]-*N*-methyl-benzamide, 30 ml de DMF et 25 ml d'acétate d'éthyle. On dégaze le milieu réactionnel, introduit 1,4 g de palladium sur charbon (10%) et hydrogène sous pression atmosphérique à 60°C. On filtre le milieu réactionnel, évapore et purifie le résidu obtenu par chromatographie sur colonne de silice élué avec un mélange de dichlorométhane et de méthanol (99-1). On recueille après évaporation des solvants 300 mg (21%) de N-[4'-(2,4-Dioxo-thiazolidin-5-ylmethyl)-biphenyl-3-ylmethyl]-N-methyl-benzamide de point de fusion 70-1°C.

### EXEMPLE 2

### 5-{3'-[(Methyl-pyridin-2-yl-amino)-methyl]-biphenyl-4-ylmethyl}-thiazolidine-2,4-dione

### (a) (3-Bromo-benzyl)-methyl-pyridin-2-yl-amine

Dans un ballon et sous courant d'azote, on introduit 1,5 g (7,5 mmol) de (3-Bromo-benzyl)-methyl-amine et 15 ml de 2-fluoropyridine. On chauffe à reflux pendant 8 heures, évapore à sec le milieu réactionnel. Le résidu obtenu est purifié par chromatographie sur colonne de silice élué avec un mélange d'heptane et d'acétate d'éthyle (80-20). Après évaporation des solvants, on recueille 1 g (50%) de produit attendu.

### (b) 3'-[(Methyl-pyridin-2-yl-amino)-methyl]-biphenyl-4-carbaldehyde

De manière analogue à l'exemple 1(e) par réaction de 475 mg (1,7 mmol) de (3-Bromo-benzyl)-methyl-pyridin-2-yl-amine avec 386 mg (2,6 mmol) d'acide 4-formylbenzèneboronique, on obtient 310 mg (60%) de produit attendu.

### (c) 5-{3'-[(Methyl-pyridin-2-yl-amino)-methyl]-biphenyl-4-ylmethylene}-thiazolidine-2,4-dione

De manière analogue à l'exemple 1(f) par réaction de 610 mg (1,85 mmol) de 3'-[(Methyl-pyridin-2-yl-amino)-methyl]-biphenyl-4-carbaldehyde avec 216 mg (1,85 mmol) de 2,4-thiazolidinedione, on obtient 640 mg (81%) de produit attendu.

### (d) 5-{3'-[(Methyl-pyridin-2-yl-amino)-methyl]-biphenyl-4-ylmethyl}-thiazolidine-2,4-dione

De manière analogue à l'exemple 1(g) à partir de 310 mg (0,8 mmol) de 5-{3'-[(Methyl-pyridin-2-yl-amino)-methyl]-biphenyl-4-ylmethylene}-thiazolidine-2,4-dione, on obtient 80 mg (26%) de 5-{3'-[(Methyl-pyridin-2-yl-amino)-methyl]-biphenyl-4-ylmethyl}-thiazolidine-2,4-dione de point de fusion 135-6°C.

### EXEMPLE 3

### N-[4'-(2,4-Dioxo-thiazolidin-5-ylmethyl)-biphenyl-3-ylmethyl]-benzamide

### (a) N-(3-Bromo-benzyl)-benzamide

De manière analogue à l'exemple 1 (d) par réaction de 7 g (32 mmol) de 3-bromobenzylamine avec 4 ml (35 mmol) de chlorure de benzoyle, on obtient 9,1 g (100%) de produit attendu.

### (b) N-(4'-Formyl-biphenyl-3-ylmethyl)-benzamide

De manière analogue à l'exemple 1 (e) par réaction de 2 g (6,9 mmol) de N-(3-Bromo-benzyl)-benzamide avec 1,6 g (10,3 mmol) d'acide 4-formylbenzèneboronique, on obtient 1,4 g (65%) de produit attendu.

### (c) N-[4'-(2,4-Dioxo-thiazolidin-5-ylidenemethyl)-biphenyl-3-ylmethyl]-benzamide

De manière analogue à l'exemple 1(f) par réaction de 1 g (3,2 mmol) de N-(4'-Formyl-biphenyl-3-ylmethyl)-benzamide avec 370 mg (3,2 mmol) de 2,4-thiazolidinedione, on obtient 1,2 g (93%) de produit attendu.

### (d) N-[4'-(2,4-Dioxo-thiazolidin-5-ylmethyl)-biphenyl-3-ylmethyl]-benzamide

De manière analogue à l'exemple 1 (g) à partir de 600 mg (1,45 mmol) de *N*-[4'-(2,4-Dioxo-thiazolidin-5-ylidenemethyl)-biphenyl-3-ylmethyl]-benzamide, on obtient 200 mg (33%) de N-[4'-(2,4-Dioxo-thiazolidin-5-ylmethyl)-biphenyl-3-ylmethyl]-benzamide de point de fusion 225-6°C.

### EXEMPLE 4

### 3-{3'-[(Benzoyl-methyl-amino)-methyl]-biphenyl-4-yl}-2-methyl-propionate d'éthyle

### (a) (E)-2-Methyl-3-(3'-{[(1-phenyl-methanoyl)-amino]-methyl}-biphenyl-4-yl)-acrylate d'éthyle

Dans un tricol et sous courant d'azote, on introduit 440 mg (11 mmol) d'hydrure de sodium (80% dans l'huile) et 10 ml de THF. On ajoute goutte à goutte une solution de 2,2 ml de 2-phosphonopropionate de triéthyle dans 10 ml de THF puis une solution de 3 g (9,1 mmol) de N-(4'-Formyl-biphenyl-3-ylmethyl)-N-methyl-benzamide ( préparé à l'exemple 1 (e) ) et agite à température ambiante pendant 3 heures. On verse le milieu réactionnel dans l'eau, extrait avec du dichlorométhane, décante la phase organique, sèche sur sulfate de magnésium, évapore. Le résidu obtenu est purifié par chromatographie sur colonne de silice élué avec un mélange d'heptane et d'acétate d'éthyle (70-30). On recueille après évaporation des solvants, 3 g (80%) de (E)-2-Methyl-3-(3'-{[(1-phenyl-methanoyl)-amino]-methyl}-biphenyl-4-yl)-acrylate d'éthyle sous forme d'une huile.

### (b) 3-{3'-[(Benzoyl-methyl-amino)-methyl]-biphenyl-4-yl}-2-methyl-propionate d'éthyle

Dans un tricol, on introduit 2,2 g (5,3 mmol) de (E)-2-Methyl-3-(3'-{[(1-phenyl-methanoyl)-amino]-methyl}-biphenyl-4-yl)-acrylate d'éthyle et 100 ml d'acétate d'éthyle. On dégaze le milieu réactionnel, introduit 450 mg de palladium sur charbon (10%) et hydrogène sous pression atmosphérique pendant deux heures. On filtre le milieu réactionnel, évapore et purifie le résidu obtenu par chromatographie sur colonne de silice élué avec un mélange d'heptane et d'acétate d'éthyle (80-20). On recueille après évaporation des solvants 1,45 g (64%) de 3-{3'-[(Benzoyl-methyl-amino)-methyl]-biphenyl-4-yl}-2-methylpropionate d'éthyle sous forme d'une huile.

### EXEMPLE 5

### Acide 2-methyl-3-(3'-{[methyl-(1-phenyl-methanoyl)-amino]-methyl}-biphenyl-4-yl)-propionique

Dans un ballon, on introduit 1,25 g (3 mmol) de 3-{3'-[(Benzoyl-methylamino)-methyl]-biphenyl-4-yl}-2-methyl-propionate d'éthyle, 3 ml d'hydroxyde de sodium (10N), 32 ml de THF et 2 ml de méthanol. On chauffe à reflux pendant 8 heures, verse le milieu réactionnel dans l'eau, acidifie à pH 4 avec de l'acide chlorhydrique 1N, extrait avec du dichlorométhane, décante la phase organique, sèche sur sulfate de magnésium, évapore. Le résidu obtenu est purifié par chromatographie sur colonne de silice élué avec un mélange d'heptane et d'acétate d'éthyle (60-40). On obtient 900 mg (90%) d' acide 2-methyl-3-(3'{[methyl-(1-phenyl-methanoyl)-amino]-methyl}-biphenyl-4-yl)-propionique de point de fusion 60-1 °C.

### EXEMPLE 6

### N-[4'-(2-Carbamoyl-propyl)-biphenyl-3-ylmethyl]-N-methyl-benzamide

### (a) chlorure de 3-(3'-{[methyl-(1-phenyl-methanoyl)-amino]-methyl}-biphenyl-4-yl)-propionyle

Par réaction de 1,6 g (4,1 mmol) d'acide 2-methyl-3-(3'-{[methyl-(1-phenylmethanoyl)-amino]-methyl}-biphenyl-4-yl)-propionique avec 390 µl (4,5 mmol) de chlorure d'oxalyle dans le dichlorométhane, on obtient après évaporation 1,7 g (100%) de chlorure de 3-(3'-{[methyl-(1-phenyl-methanoyl)-amino]-methyl}biphenyl-4-yl)-propionyle.

### (b) N-[4'-(2-Carbamoyl-propyl)-biphenyl-3-ylmethyl]-N-methyl-benzamide

Par réaction d'une solution de 1,7 g (4,1 mmol) de chlorure de 3-(3'-{[methyl-(1-phenyl-methanoyl)-amino]-methyl}-biphenyl-4-yl)-propionyle dans 5 ml de THF avec 10 ml d'ammoniaque, on obtient après purification par chromatographie sur colonne de silice 200 mg (32%) de N-[4'-(2-Carbamoylpropyl)-biphenyl-3-ylmethyl]-N-methyl-benzamide de point de fusion 47-8°C.

### EXEMPLE 7

### N-Methyl-N-[4'-(2-phenylcarbamoyl-propyl)-biphenyl-3-ylmethyl]-benzamide

Par réaction d'une solution de 637 mg (1,6 mmol) de chlorure de 3-(3'-{[methyl-(1-phenyl-methanoyl)-amino]-methyl}-biphenyl-4-yl)-propionyle dans 5 ml de THF avec 146 µl (1,6 mmol) de benzylamine en présence de 250 µl (1,8 mmol) de triéthylamine (1,6 mmol), on obtient après purification par chromatographie sur colonne de silice 370 mg (50%) de N-Methyl-N-[4'-(2-phenylcarbamoyl-propyl)-biphenyl-3-ylmethyl]-benzamide de point de fusion 68-9°C.

### EXEMPLE 8

### N-[4'-(2-Hydroxycarbamoyl-propyl)-biphenyl-3-ylmethyl]-N-methylbenzamide

Par réaction d'une solution de 669 mg (1,65 mmol) de chlorure de 3-(3'-{[methyl-(1-phenyl-methanoyl)-amino]-methyl}-biphenyl-4-yl)-propionyle dans 6 ml de THF avec 243 mg (1,65 mmol) de O-(tert-butyldimethylsilyl)hydroxylamine en présence de 250 µl (1,8 mmol) de triéthylamine puis déprotection avec 530 µl d'une solution de fluorure de tétrabutylammonium (1 N dans le THF), on obtient après purification par chromatographie sur colonne de silice 350 mg (42%) N-[4'-(2-Hydroxycarbamoyl-propyl)-biphenyl-3-ylmethyl]-N-methyl-benzamide de point de fusion 65-6°C.

### EXEMPLE 9

### 1-[4'-(2,4-Dioxo-thiazolidin-5-ylmethyl)-biphenyl-3-ylmethyl]-3-phenyl-urée

### (a) 1-(4'-Bromo-biphenyl-3-ylmethyl)-3-phenyl-urée

Dans un ballon, on introduit 2,9 g (14,5 mmol) de 3-bromobenzylamine, 90 ml de dichlorométhane et ajoute goutte à goutte 1,73 ml (16 mmol) de phenylisocyanate. On agite à température ambiante pendant huit heures, verse le milieu réactionnel dans l'eau, extrait avec du dichlorométhane, décante la phase organique, sèche sur sulfate de magnésium, évapore. Le résidu obtenu est purifié par chromatographie sur colonne de silice élué avec un mélange d'heptane et d'acétate d'éthyle (70-30). On obtient 3,7 g (80%) de produit attendu.

### (b) 1-(4'-Formyl-biphenyl-3-ylmethyl)-3-phenyl-urée

De manière analogue à l'exemple 1(e) par réaction de 2,5 g (8,2 mmol) de 1-(4'-Bromo-biphenyl-3-ylmethyl)-3-phenyl-urée avec 1,8 g (12,3 mmol) d'acide 4-formylbenzèneboronique, on obtient 2,1 g (77%) de produit attendu.

### (c) 1-[4'-(2,4-Dioxo-thiazolidin-5-ylidenemethyl)-biphenyl-3-ylmethyl]-3-phenyl-urée

De manière analogue à l'exemple 1(f) par réaction de 2,1 g (6,4 mmol) de 1-(4'-Formyl-biphenyl-3-ylmethyl)-3-phenyl-urée avec 750 mg (6,4 mmol) de 2,4-thiazolidinedione, on obtient 1,4 g (53%) de produit attendu.

### (d) 1-[4'-(2,4-Dioxo-thiazolidin-5-ylmethyl)-biphenyl-3-ylmethyl]-3-phenyl-urée

De manière analogue à l'exemple 1 (g) à partir de 1,4 g (3,3 mmol) de 1-[4'-(2,4-Dioxo-thiazolidin-5-ylidenemethyl)-biphenyl-3-ylmethyl]-3-phenyl-urée, on obtient 300 mg (21 %) de 1-[4'-(2,4-Dioxo-thiazolidin-5-ylmethyl)-biphenyl-3-ylmethyl]-3-phenyl-urée de point de fusion 70-1°C.

### EXEMPLE 10

### 1-[4'-(2,4-Dioxo-thiazolidin-5-ylmethyl)-biphenyl-3-ylmethyl]-1-methyl-3-phenyl-urée

### (a) (4'-Formyl-biphenyl-3-ylmethyl)-methyl-carbamate de tert-butyle

De manière analogue à l'exemple 1(e) par réaction de 13,7 g (45 mmol) de (3-bromobenzyl)-N-méthylcarbamate de tert-butyle ( préparé à l'exemple 1(b)) avec 10 g (67 mmol) d'acide 4-formylbenzèneboronique, on obtient 11 g (76%) de produit attendu.

### (b) [4'-(2,4-Dioxo-thiazolidin-5-ylidenemethyl)-biphenyl-3-ylmethyl]-methyl-carbamate de tert-butyle

De manière analogue à l'exemple 1(f) par réaction de 8 g (25 mmol) de (4'-Formyl-biphenyl-3-ylmethyl)-methyl-carbamate de tert-butyle avec 2,9 g (25 mmol) de 2,4-thiazolidinedione, on obtient 8,6 g (81 %) de produit attendu.

### (c) 5-(3'-Methylaminomethyl-biphenyl-4-ylidenemethyl)-thiazolidine-2,4-dione

Dans un ballon, on introduit 2 g (4,7 mmol) de [4'-(2,4-Dioxo-thiazolidin-5-ylidenemethyl)-biphenyl-3-ylmethyl]-methyl-carbamate de tert-butyle et 20 ml de dichlorométhane. On ajoute 2,2 ml (28,2 mmol) d'acide trifluoroacétique et agite à température ambiante pendant quatre heures. On verse le milieu réactionnel dans l'eau bicarbonatée, extrait avec du dichlorométhane, décante la phase organique, sèche sur sulfate de magnésium, évapore. On obtient 1,3 g (85%) du produit attendu.

### (d) 1-[4'-(2,4-Dioxo-thiazolidin-5-ylidenemethyl)-biphenyl-3-ylmethyl]-1-methyl-3-phenyl-urée

De manière analogue à l'exemple 9(a) par réaction de 300 mg (0,9 mmol) de 5-(3'-Methylaminomethyl-biphenyl-4-ylmethylene)-thiazolidine-2,4-dione avec 190 µl (1,8 mmol) de phenylisocyanate, on obtient 450 mg (98%) de produit attendu.

### (e) 1-[4'-(2,4-Dioxo-thiazolidin-5-ylmethyl)-biphenyl-3-ylmethyl]-1-methyl-3-phenyl-urée

De manière analogue à l'exemple 1(g) à partir de 450 mg (0,8 mmol) de 1-[4'-(2,4-Dioxo-thiazolidin-5-ylidenemethyl)-biphenyl-3-ylmethyl]-1-methyl-3-phenyl-urée, on obtient 100 mg (28%) de 1-[4'-(2,4-Dioxo-thiazolidin-5-ylmethyl)-biphenyl-3-ylmethyl]-1-methyl-3-phenyl-urée de point de fusion 70-1°C.

### EXEMPLE 11

### [4'-(2,4-Dioxo-thiazolidin-5-ylmethyl)-biphenyl-3-ylmethyl]-methyl-carbamate de tert-butyle

De manière analogue à l'exemple 1(g) à partir de 500 mg (1,2 mmol) de [4'-(2,4-Dioxo-thiazolidin-5-ylidenemethyl)-biphenyl-3-ylmethyl]-methyl-carbamate de tert-butyle ( préparé à l'exemple 10(b)), on obtient 250 mg (49%) de [4'-(2,4-Dioxo-thiazolidin-5-ylmethyl)-biphenyl-3-ylmethyl]-methyl-carbamate de tert-butyle de point de fusion 45-6°C.

### EXEMPLE 12

### N-[4'-(2,4-dioxo-thiazolidin-5-ylmethyl)-biphenyl-3-ylmethyl]-N-methylnonanamide

### (a) N-[4'-(2,4-dioxo-thiazolidin-5-ylidenemethyl)-biphenyl-3-ylmethyl]-N-methylnonanamide

De manière analogue à l'exemple 1(d) par réaction de 500 mg (0,9 mmol) de 5-(3'-Methylaminomethyl-biphenyl-4-ylmethylene)-thiazolidine-2,4-dione avec 330 µl (1,8 mmol) de chlorure de nonanoyle, on obtient 350 mg (66%) de produit attendu.

### (b) N-[4'-(2,4-dioxo-thiazolidin-5-ylmethyl)-biphenyl-3-ylmethyl]-N-methylnonanamide

De manière analogue à l'exemple 1(g) à partir de 330 mg (0,7 mmol) de *N*-[4'-(2,4-dioxo-thiazolidin-5-ylidenemethyl)-biphenyl-3-ylmethyl]-*N*-methylnonanamide, on obtient 70 mg (21 %) de *N*-[4'-(2,4-dioxo-thiazolidin-5-ylmethyl)-biphenyl-3-ylmethyl]-*N*-methyl-nonanamide.

### EXEMPLE 13

### Acide (S)-2-ethoxy-3-(3'-{[methyl-(1-phenyl-methanoyl)-amino]-methyl}biphenyl-4-yl)-propionique

### (a) N-{4'-[(1R,2S)-3-((S)-4-Benzyl-2-oxo-oxazolidin-3-yl)-2-ethoxy-1-hydroxy-3-oxo-propyl]-biphenyl-3-ylmethyl}-N-methyl-benzamide

Dans un tricol et sous argon, on introduit 1,8 g (6,7 mmol) de (S)-4-Benzyl-3-(2-ethoxy-ethanoyl)-oxazolidin-2-one et 15 ml de dichlorométhane. On ajoute à 0°C goutte à goutte successivement 7,3 ml (8 mmol) de trifluorométhanesulfonate de dibutylborane et 1,3 ml (8 mmol) de diisopropyléthylamine et agite pendant une heure. A -78°C, on ajoute une solution de 2 g (6 mmol) de N-(4'-Formylbiphenyl-3-ylmethyl)-*N*-methyl-benzamide ( préparé à l'exemple 1(e)) dans 25 ml de dichlorométhane et agite pendant une heure. On laisse remonter à 0°C et traite avec une solution tampon pH=7 (16 ml) dans 45 ml de méthanol puis avec une solution d'eau oxygénée (16 ml) dans 45 ml de méthanol et agite une heure à 0°C. On verse le milieu réactionnel dans l'eau, extrait avec du dichlorométhane, décante la phase organique, sèche sur sulfate de magnésium, évapore. Le résidu obtenu est purifié par chromatographie sur colonne de silice élué avec un mélange d'heptane et d'acétate d'éthyle (60-40). On obtient 1,7 g (48%) de *N*-{4'-[(1R,2S)-3-((S)-4-Benzyl-2-oxo-oxazolidin-3-yl)-2-ethoxy-1-hydroxy-3-oxo-propyl]-biphenyl-3-ylmethyl}-*N*-methyl-benzamide

### (b) N-{4'-[(S)-3-((S)-4-Benzyl-2-oxo-oxazolidin-3-yl)-2-ethoxy-3-oxo-propyl]-biphenyl-3-ylmethyl}-N-methyl-benzamide.

Dans un tricol et sous courant d'azote, on introduit 320 µl (2,6 mmol) d'éthérate de trifluorure de bore et 5 ml de dichlorométhane. A 0°C, on ajoute goutte à goutte 800 µl (5 mmol) d'hydrure de triméthylsilane puis par petites portions 380 mg (0,64 mmol) de N-{4'-[(1R,2S)-3-((S)-4-Benzyl-2-oxo-oxazolidin-3-yl)-2-ethoxy-1-hydroxy-3-oxo-propyl]-biphenyl-3-ylmethyl}-N-methyl-benzamide. Après une heure à température ambiante, on chauffe à 50°C pendant 20 heures. On verse le milieu réactionnel dans l'eau, extrait avec de l'acétate d'éthyle, décante la phase organique, sèche sur sulfate de magnésium, évapore. Le résidu obtenu est purifié par chromatographie sur colonne de silice élué avec un mélange d'heptane et d'acétate d'éthyle (70-30). On obtient 280 mg (76%) de produit attendu.

### (c) Acide (S)-2-ethoxy-3-(3'-{[methyl-(1-phenyl-methanoyl)-amino]-methyl}-biphenyl-4-yl)-propionique

Dans un ballon, on introduit 600 mg (1 mmol) de N-[4'-[(S)-3-((S)-4-Benzyl-2-oxo-oxazolidin-3-yl)-2-ethoxy-3-oxo-propyl]-biphenyl-3-ylmethyl}-N-methylbenzamide et 10 ml de THF. A 0°C, on ajoute 4,2 ml (2 mmol) d'une solution aqueuse d'hydroxyde de lithium (0,5 M) et agite une heure. On verse le milieu réactionnel dans l'eau, acidifie à pH 1, extrait avec de l'acétate d'éthyle, décante la phase organique, sèche sur sulfate de magnésium, évapore. Le résidu obtenu est purifié par chromatographie sur colonne de silice élué avec un mélange d'heptane et d'acétate d'éthyle (50-50). On obtient 200 mg (47%) d' acide (S)-2-ethoxy-3-(3'-{[methyl-(1-phenyl-methanoyl)-amino]-methyl}-biphenyl-4-yl)-propionique de point de fusion 53-54°C.

### EXEMPLE 14

### 2-(3'-{[methyl-(1-phenyl-methanoyl)-amino]-methyl}-biphenyl-4-ylmethyl)-malonate de monométhyle

### (a) 2-(3'-{[Methyl-(1-phenyl-methanoyl)-amino]-methyl}-biphenyl-4-ylmethylene)-malonate de diéthyle

Dans un tricol, sous courant d'azote, on introduit 600 mg (1,8 mmol) de N-(4'-formyl-biphenyl-3-ylmethyl)-N-methyl-benzamide (préparé comme dans l'exemple 1e), 10 ml de toluène, 300 µl (1,8 mmol) de malonate de diéthyle et 50 mg (0,35 mmol) d'acétate de pipéridine. On chauffe à reflux pendant 6 heures en séparant l'eau formée à l'aide d'un Dean-Stark. Le milieu réactionnel est refroidi, extrait à l'acétate d'éthyle, lavé à l'eau. On décante la phase organique, sèche sur sulfate de magnésium, évapore. Le produit brut est purifié par chromatographie sur colonne de silice élué avec un mélange d'heptane et d'acétate d'éthyle (75-25). Après évaporation des solvants, on recueille 700 mg (84%) du produit attendu.

### (b) 2-(3'-{[Methyl-(1-phenyl-methanoyl)-amino]-methyl}-biphenyl-4-ylmethyl)-malonate de diéthyle

Dans un tricol, sous courant d'azote, on introduit 700 mg (1,5 mmol) de 2-(3'-{[Methyl-(1-phenyl-methanoyl)-amino]-methyl}-biphenyl-4-ylmethylene)-malonate de diéthyle, 7 ml de THF et 1 ml de méthanol. On dégaze le milieu réactionnel, introduit 80 mg de palladium sur charbon (5%) et on hydrogène sous pression atmosphérique à température ambiante pendant 20 heures. Le milieu réactionnel est filtré sur célite, évaporé. Le résidu obtenu est purifié par chromatographie sur colonne de silice élué avec un mélange d'heptane et d'acétate d'éthyle (80-20). On recueille après évaporation des solvants, 450 mg (63%) du produit voulu.

### (c) 2-(3'-{[Methyl-(1-phenyl-methanoyl)-amino]-methyl}-biphenyl-4-ylmethyl)-malonate de monométhyle

Dans un ballon, on place 400 mg (0,85 mmol) de 2-(3'-{[Methyl-(1-phenylmethanoyl)-amino]-methyl}-biphenyl-4-ylmethylene)-malonate de diéthyle, 5 ml de méthanol et 100 mg de carbonate de potassium. On agite à température ambiante pendant 18 heures, acidifie à pH 3 avec de l'acide sulfurique et on extrait à l'acétate d'éthyle. La phase organique est séchée sur sulfate de magnésium et évaporée. Le résidu obtenu est purifié par chromatographie sur colonne de silice élué avec un mélange d'heptane et d'acétate d'éthyle (70-30). On recueille après évaporation des solvants, 160 mg (44%) du produit voulu de point de fusion 67°C.

### EXEMPLE 15

### 2-(3'-{[Methyl-(1-phenyl-methanoyl)-amino]-methyl}-biphenyl-4-ylmethyl)malonate de diméthyle

### (a) 2-(3'-{[Methyl-(1-phenyl-methanoyl)-amino]-methyl}-biphenyl-4-ylmethylene)-malonate de diméthyle

Dans un tricol, sous courant d'azote, on introduit 1,4 g (4,25 mmol) de N-(4'-formyl-biphenyl-3-ylmethyl)-N-methyl-benzamide (préparé comme dans l'exemple 1e), 20 ml de toluène, 560 mg (4,25 mmol) de malonate de diméthyle et 125 mg (0,85 mmol) d'acétate de pipéridine. On chauffe à reflux pendant 6 heures en séparant l'eau formée à l'aide d'un Dean-Stark. Le milieu réactionnel est refroidi, extrait à l'acétate d'éthyle, lavé à l'eau. On décante la phase organique, sèche sur sulfate de magnésium, évapore. Le produit brut est purifié par chromatographie sur colonne de silice élué avec un mélange d'heptane et d'acétate d'éthyle (70-30). Après évaporation des solvants, on recueille 1,4 g (75%) du produit attendu.

### (b) 2-(3'-{[Methyl-(1-phenyl-methanoyl)-amino]-methyl}-biphenyl-4-ylmethyl)-malonate de diméthyle

Dans un tricol, sous courant d'azote, on introduit 1,3 g (2,9 mmol) de 2-(3'-{[Methyl-(1-phenyl-methanoyl)-amino]-methyl}-biphenyl-4-ylmethylene)-malonate de diméthyle, 10 ml de dioxanne et 2 ml de méthanol. On dégaze le milieu réactionnel, introduit 80 mg de palladium sur charbon (5%) et on hydrogène sous pression atmosphérique à température ambiante pendant 8 heures. Le milieu réactionnel est filtré sur célite, évaporé. Le résidu obtenu est purifié par chromatographie sur colonne de silice élué avec un mélange d'heptane et d'acétate d'éthyle (60-40). On recueille après évaporation des solvants, 1 g (78%) du produit voulu sous forme d'une huile.

### EXEMPLE 16

### 2-(3'-{[Methyl-(1-phenyl-methanoyl)-amino]-methyl}-biphenyl-4-ylmethyl)-malonamate de méthyle

### (a) 2-(3'-{[Methyl-(1-phenyl-methanoyl)-amino]-methyl}-biphenyl-4-ylmethylene)-maloriate de diméthyle

Dans un tricol, sous courant d'azote, on introduit 1,54 g (4,25 mmol) de N-(4'-formyl-biphenyl-3-ylmethyl)-N-methyl-benzamide (préparé comme dans l'exemple 1e), 20 ml de toluène, 0,56 g (4,25 mmol) de malonate de diméthyle et 123 mg (0,85 mmol) d'acétate de pipéridine. On chauffe à reflux pendant 5 heures en séparant l'eau formée à l'aide d'un Dean-Stark. Le milieu réactionnel est refroidi, extrait à l'acétate d'éthyle, lavé à l'eau. On décante la phase organique, sèche sur sulfate de magnésium, évapore. Le produit brut est purifié par chromatographie sur colonne de silice élué avec un mélange d'heptane et d'acétate d'éthyle (70-30). Après évaporation des solvants, on recueille 1,4 g (75%) du produit attendu.

### (b) 2-(3'-{[Methyl-(1-phenyl-methanoyl)-amino]-methyl}-biphenyl-4-ylmethyl)-malonate de diméthyle

Dans un tricol, sous courant d'azote, on introduit 1,3 g (2,9 mmol) de 2-(3'-{[Methyl-(1-phenyl-methanoyl)-amino]-methyl}-biphenyl-4-ylmethylene)-malonate de diméthyle, 10 ml de dioxanne et 2 ml de méthanol. On ajoute 76 mg de palladium sur charbon (5%), on hydrogène sous pression atmosphérique pendant 8 heures. Le milieu réactionnel est filtré sur célite, évaporé. Le résidu obtenu est purifié par chromatographie sur colonne de silice élué avec un mélange d'heptane et d'acétate d'éthyle (60-40). On obtient 1 g (78%) du produit voulu.

### (c) 2-(3'-{[Methyl-(1-phenyl-methanoyl)-amino]-methyl}-biphenyl-4-ylmethyl)-malonate monomethylique

Dans un ballon, on introduit 920 mg (2 mmol) de 2-(3'-{[Methyl-(1-phenylmethanoyl)-amino]-methyl}-biphenyl-4-ylmethyl)-malonate de diméthyle ( préparé en 15(b) ), 4 ml de tétrahydrofuranne et 8 ml de méthanol. A 0°C, on additionne goutte à goutte 0,24 ml (2,1 mmol) d'une solution aqueuse de soude 35%. On agite pendant 18 heures, verse le milieu réactionnel dans de l'eau, acidifie à pH 4-5 avec une solution d'acide chlorhydrique 1 N, extrait avec de l'acétate d'éthyle. Après décantation, la phase organique est séchée sur sulfate de magnésium, filtrée, évaporée. On obtient 820 mg (92%) du produit voulu.

### (d) 2-(3'-{[Methyl-(1-phenyl-methanoyl)-amino]-methyl}-biphenyl-4-ylmethyl)-malonamate de methyle

Dans un tricol, sous courant d'azote, on introduit 780 mg (1,8 mmol) de 2-(3'-{[Methyl-(1-phenyl-methanoyl)-amino]-methyl}-biphenyl-4-ylmethyl)-malonate monomethylique, 8 ml de dichlorométhane. A 0°C, on additionne goutte à goutte, 180 µl (2 mmol) de chlorure d'oxalyle puis on agite pendant 4 heures. Le milieu réactionnel est évaporé à sec, placé dans 2 ml d'acétone et 1 ml d'une solution d'ammoniaque à 32%. On agite à température ambiante pendant 2 heures, plonge le milieu réactionnel dans de l'eau, acidifie à pH5, extrait à l'acétate d'éthyle. La phase organique est séchée sur sulfate de magnésium, filtrée, évaporée. Le résidu obtenu est lavé avec un mélange d'heptane et d'éther (50-50). On obtient 400 mg (52%) du produit voulu de point de fusion 62°C.

### EXEMPLE 17

### N-[4'-(2,4-Dioxo-thiazolidin-5-ylmethyl)-biphenyl-3-ylmethyl]-N-ethylbenzamide

### (a) N-(3-Bromo-benzyl)-benzamide

Dans un tricol et sous courant d'azote, on place 7 g (31,6 mmol) de 3-Bromo-benzylamine, 100 ml de THF, 9,6 ml (69,2 mmol) de triéthylamine. On additionne goutte à goutte 4 ml (34,6 mmol) de chlorure de benzoyle et on agite à température pendant 1 heure. Le milieu réactionnel est extrait avec de l'acétate d'éthyle, lavé à l'eau, séché sur sulfate de magnésium, filtré, évaporé. On obtient 9,2 g (100%) du produit voulu.

### (b) N-(4'-Formyl-biphenyl-3-ylmethyl)-benzamide

De manière analogue à l'exemple 1(e) par réaction de 7,4 g (25,4 mmol) de *N*-(3-Bromo-benzyl)-benzamide avec 5,7 g (38,2 mmol) d'acide 4-formylbenzèneboronique, on obtient 4 g (50%) de produit attendu.

### (c) N-(4'-[1,3]Dioxolan-2-yl-biphenyl-3-ylmethyl)-benzamide

Dans un tricol et sous courant d'azote, on introduit 3,2 g (10 mmol) de *N*-(4'-Formyl-biphenyl-3-ylmethyl)-benzamide, 50 ml de toluène, 2,8 ml (50 mmol) d'éthylène glycol et 38 mg (0,2 mmol) d'acide para-toluènesulfonique. Le milieu réactionnel est chauffé à reflux pendant 3 heures et l'eau formée est séparée à l'aide d'un dean-stark. On extrait au dichlorométhane, lave à l'eau, décante la phase organique. La phase organique est séchée sur sulfate de magnésium, filtrée, évaporée. On obtient 3,7 g (100%) du produit voulu.

### (d) N-(4'-[1,3]Dioxolan-2-yl-biphenyl-3-ylmethyl)- N-ethyl-benzamide

Dans un tricol et sous azote, on introduit 600 mg (1,7 mmol) de *N*-(4'-[1,3]Dioxolan-2-yl-biphenyl-3-ylmethyl)-benzamide, 5 ml de THF et 206 mg (1,85 mmol) de tert-butylate de potassium. On ajoute goutte à goutte 300 µl (3,7 mmol) d'iodoéthane. On agite à température ambiante pendant 3 heures, on extrait à l'acétate d'éthyle, lave à l'eau, sèche sur sulfate de magnésium, filtre et évapore. Le résidu obtenu est purifié par chromatographie sur colonne de silice élué avec un mélange d'heptane et d'acétate d'éthyle (70-30). On obtient 400 mg (64%) du produit voulu.

### (e) N-Ethyl-N-(4'-formyl-biphenyl-3-ylmethyl)-benzamide

Dans un ballon, on introduit 400 mg (1 mmol) de *N*-(4'-[1,3]Dioxolan-2-yl-biphenyl-3-ylmethyl)- *N*-ethyl-benzamide, 5 ml de méthanol, 2 g de silice et quelques gouttes d'acide sulfurique. On agite à température ambiante pendant 24 heures. On verse le milieu réactionnel dans l'eau, extrait avec du dichlorométhane, décante la phase organique, sèche sur sulfate de magnésium, évapore. On obtient 320 mg (91 %) du produit voulu.

### (f) N-[4'-(2,4-Dioxo-thiazolidin-5-ylidenemethyl)-biphenyl-3-ylmethyl]-N-ethylbenzamide

De manière analogue à l'exemple 1(f) par réaction de 300 mg (0,9 mmol) de *N*-Ethyl-*N*-(4'-formyl-biphenyl-3-ylmethyl)-benzamide avec 100 mg (0,9 mmol) de 2,4-thiazolidinedione, on obtient 340 mg (88%) de produit attendu.

### (g) N-[4'-(2,4-Dioxo-thiazolidin-5-ylmethyl)-biphenyl-3-ylmethyl]-N-ethyl-benzamide

De manière analogue à l'exemple 1(g) à partir de 300 mg (0,7 mmol) de *N*-[4'-(2,4-Dioxo-thiazolidin-5-ylidenemethyl)-biphenyl-3-ylmethyl]-*N*-ethylbenzamide dans 10 ml THF, on obtient 150 mg (50%) de *N*-[4'-(2,4-Dioxothiazolidin-5-ylmethyl)-biphenyl-3-ylmethyl]-*N*-ethyl-benzamide de point de fusion 157°C..

### EXEMPLE 18

### N-[4'-(2,4-Dioxo-thiazolidin-5-ylmethyl)-biphenyl-3-ylmethyl]-N-pentylbenzamide

### (a) N-(4'-[1,3]Dioxolan-2-yl-biphenyl-3-ylmethyl)- N-pentyl-benzamide

De manière analogue à l'exemple 17(d) à partir de 1 g (2,8 mmol) de *N*-(4'-[1,3]Dioxolan-2-yl-biphenyl-3-ylmethyl)-benzamide( prépéré en 17(c) ) et de 2,4 ml (18,3 mmol) d'iodopentane, on obtient 600 mg (50%) de *N*-(4'-[1,3]Dioxolan-2-yl-biphenyl-3-ylmethyl)- *N*-pentyl-benzamide.

### (b) N-(4'-Formyl-biphenyl-3-ylmethyl)- N-pentyl-benzamide

De manière analogue à l'exemple 17(e) à partir de 550 mg (1,2 mmol) de *N*-(4'-[1,3]Dioxolan-2-yl-biphenyl-3-ylmethyl)-*N*-pentyl-benzamide, on obtient 400 mg (82%) de *N*-(4'-Formyl-biphenyl-3-ylmethyl)- *N*-pentyl-benzamide.

### (c) N-[4'-(2,4-Dioxo-thiazolidin-5-ylidenemethyl)-biphenyl-3-ylmethyl]-N-pentylbenzamide

De manière analogue à l'exemple 1(f) par réaction de 320 mg (0,8 mmol) de N-(4'-Formyl-biphenyl-3-ylmethyl)-benzamide avec 100 mg (0,8 mmol) de 2,4-thiazolidinedione, on obtient 350 mg (87%) de produit attendu.

### (d) N-[4'-(2,4-Dioxo-thiazolidin-5-ylmethyl)-biphenyl-3-ylmethyl]-N-pentylbenzamide

De manière analogue à l'exemple 1(g) à partir de 330 mg (0,7 mmol) de *N*-[4'-(2,4-Dioxo-thiazolidin-5-ylidenemethyl)-biphenyl-3-ylmethyl]-*N*-pentylbenzamide dans 10 ml d'acétate d'éthyle et 10 ml d'éthanol, on obtient 220 mg (65%) de *N*-[4'-(2,4-Dioxo-thiazolidin-5-ylmethyl)-biphenyl-3-ylmethyl]-*N*-pentylbenzamide de point de fusion 57°C.

### EXEMPLE 19

### [4'-(2,4-Dioxo-thiazolidin-5-ylmethyl)-biphenyl-3-ylmethyl]-ethyl-carbamate de tert-butyle

### (a) (3-Bromo-benzyl)-ethyl-carbamate de tert-butyle

De manière analogue à l'exemple 17(d) à partir de 3 g (10,5 mmol) de (3-Bromo-benzyl)-carbamate de tert-butyle (préparé comme en 1(a)) et de 4,2 ml (52,5 mmol) d'iodoéthane, on obtient 3,2 g (97%) du produit voulu.

### (b) Ethyl-(4'-formyl-biphenyl-3-ylmethyl)- carbamate de tert-butyle

De manière analogue à l'exemple 1(e) par réaction de 3,2 g (10 mmol) de (3-Bromo-benzyl)-ethyl- carbamate de tert-butyle avec 2,3 g (15 mmol) d'acide 4-formylbenzèneboronique, on obtient 1,4 g (41%) de produit attendu.

### (c) [4'-(2,4-Dioxo-thiazolidin-5-ylidenemethyl)-biphenyl-3-ylmethyl]-ethylcarbamate de tert-butyle

De manière analogue à l'exemple 1(f) par réaction de 1,35 g (4 mmol) de Ethyl-(4'-formyl-biphenyl-3-ylmethyl)- carbamate de tert-butyle avec 470 mg (4 mmol) de 2,4-thiazolidinedione, on obtient 1,6 g (92%) de produit attendu.

### (d) [4'-(2,4-Dioxo-thiazolidin-5-ylmethyl)-biphenyl-3-ylmethyl]-ethyl- carbamate de tert-butyle

De manière analogue à l'exemple 1(g) à partir de 500 mg (1,15 mmol) de [4'-(2,4-Dioxo-thiazolidin-5-ylidenemethyl)-biphenyl-3-ylmethyl]-ethyl- carbamate de tert-butyle dans 5 ml d'acétate d'éthyle, on obtient 100 mg (20%) du produit voulu de point de fusion 103°C.

### EXEMPLE 20

### [4'-(2,4-Dioxo-thiazolidin-5-ylmethyl)-biphenyl-3-ylmethyl]-propylcarbamate de tert-butyle

### (a) [4'-(2,4-Dioxo-thiazolidin-5-ylidenemethyl)-biphenyl-3-ylmethyl]- carbamate de tert-butyle

De manière analogue à l'exemple 1(f) par réaction de 7,4 g (24 mmol) de (4'-Formyl-biphenyl-3-ylmethyl)-carbamate de *tert*-butyle (préparé comme en 1e à partir du (3-Bromo-benzyl)-carbamate de *tert*-butyle) avec 2,8 g (24 mmol) de 2,4-thiazolidinedione, on obtient 9 g (95 %) de produit attendu.

### (b) [4'-(2,4-Dioxo-thiazolidin-5-ylidenemethyl)-biphenyl-3-ylmethyl]-propylcarbamate de tert-butyle

De manière analogue à l'exemple 17(d) à partir de 700 mg (1,7 mmol) de [4'-(2,4-Dioxo-thiazolidin-5-ylidenemethyl)-biphenyl-3-ylmethyl]- carbamate de tert-butyle et de 350 µl (3,8mmol) de 1-bromopropane, on obtient 600 mg (78%) du produit voulu.

### (c) [4'-(2,4-Dioxo-thiazolidin-5-ylmethyl)-biphenyl-3-ylmethyl]-propyl- carbamate de tert-butyle

De manière analogue à l'exemple 1(g) à partir de 600 mg (1,3 mmol) de [4'-(2,4-Dioxo-thiazolidin-5-ylidenemethyl)-biphenyl-3-ylmethyl]-propyl- carbamate de tert-butyle dans 7 ml d'acétate d'éthyle et 7 ml de diméthylformamide, on obtient 50 mg (10%) du produit voulu.

### EXEMPLE 21

### [4'-(2,4-Dioxo-thiazolidin-5-ylmethyl)-biphenyl-3-ylmethyl]-methylcarbamate de 9H-fluoren-9-ylmethyle

### (a) [4'-(2,4-Dioxo-thiazolidin-5-ylidenemethyl)-biphenyl-3-ylmethyl]-methylcarbamate de 9H-fluoren-9-ylmethyle

Dans un ballon, on introduit 500 mg (0,9 mmol) de 5-(3'-Methylaminomethyl-biphenyl-4-ylmethylene)-thiazolidine-2,4-dione ( préparé en 12(c) ) dans 12 ml d'une solution aqueuse de carbonate de sodium à 10%. A 0°C, on additionne goutte à goutte 240 mg de chlorure de 9H-fluoren-9-ylmethyle dans 5 ml de dioxanne. On agite de 0°C à température ambiante pendant 4 heures. Le milieu réactionnel est plongé dans l'eau, et extrait à l'acétate d'éthyle après acidification à pH4. La phase organique est séchée sur sulfate de magnésium, filtrée, évaporée. On obtient 490 mg (100%) de produit attendu.

### (b) [4'-(2,4-Dioxo-thiazolidin-5-ylmethyl)-biphenyl-3-ylmethyl]- methyl-carbamate de 9H-fluoren-9-ylmethyle

De manière analogue à l'exemple 1(g) à partir de 500 mg (0,9 mmol) de [4'(2,4-Dioxo-thiazolidin-5-ylidenemethyl)-biphenyl-3-ylmethyl]- methyl-carbamate de 9H-fluoren-9-ylmethyle dans 10 ml de dioxanne, on obtient 200 mg (40%) du produit voulu de point de fusion 94°C.

### EXEMPLE 22

### N-[4'-(2,4-Dioxo-thiazolidin-5-ylmethyl)-biphenyl-3-ylmethyl]-2,2,Ntrimethyl-propionamide

### (a) N-[4'-(2,4-Dioxo-thiazolidin-5-ylidenemethyl)-biphenyl-3-ylmethyl]-2,2,N-trimethyl-propionamide

De manière analogue à l'exemple 1(d) par réaction de 500 mg (0,9 mmol) de 5-(3'-Methylaminomethyl-biphenyl-4-ylmethylene)-thiazolidine-2,4-dione ( préparé en 12(c) ) avec 0,3 ml (2,3 mmol) de chlorure de 2,2-dimethyl-propionyle, on obtient 240 mg (65%) de produit attendu.

### (b) N-[4'-(2,4-Dioxo-thiazolidin-5-ylmethyl)-biphenyl-3-ylmethyl]-2,2,N-trimethyl-propionamide

De manière analogue à l'exemple 1(g) à partir de 240 mg (0,6 mmol) de *N*-[4'-(2,4-Dioxo-thiazolidin-5-ylidenemethyl)-biphenyl-3-ylmethyl]-2,2,*N*-trimethylpropionamide dans 20 ml de tétrahydrofuranne, on obtient 100 mg (40%) du produit voulu de point de fusion 78°C.

### EXEMPLE 23

### N-octyl-4'-(2,4-Dioxo-thiazolidin-5-ylmethyl)-biphenyl-3-carboxamide

### (a) 4'-Formyl-biphenyl-3-carboxylate d'éthyle

De manière analogue à l'exemple 1(e) par réaction de 12,6 ml (79 mmol) de 3-Bromobenzoate d'éthyle avec 15 g (100 mmol) d'acide 4-formylbenzèneboronique, on obtient 12 g (60%) de produit attendu.

### (b) 4'-(2,4-Dioxo-thiazolidin-5-ylidenemethyl)-biphenyl-3-carboxylate d'éthyle

De manière analogue à l'exemple 1(f) par réaction de 11,7 g (46 mmol) de 4'-Formyl-biphenyl-3-carboxylate d'éthyle avec 5,4 g (46 mmol) de 2,4-thiazolidinedione, on obtient 13 g (83%) de produit attendu.

### (c) acide 4'-(2,4-Dioxo-thiazolidin-5-ylidenemethyl)-biphenyl-3-carboxylique

Dans un ballon, on introduit 12,6 g (37 mmol) de 4'-(2,4-Dioxo-thiazolidin-5-ylidenemethyl)-biphenyl-3-carboxylate d'éthyle, 150 ml tétrahydrofuranne, 15 ml de méthanol, quelques gouttes d'eau et 7,4 g (186 mmol) d'hydroxyde de sodium en pastilles. On chauffe à reflux pendant 18 heures. Le milieu réactionnel est placé dans de l'eau, acidifié à pH2, extrait avec de l'acétate d'éthyle. La phase organique est séchée sur sulfate de magnésium, filtrée, évaporée. On obtient 11,4 g (94%) du produit voulu.

### (d) N-octyl-4'-(2,4-Dioxo-thiazolidin-5-ylidenemethyl)-biphenyl-3-carboxamide

Dans un tricol, sous courant d'azote, on introduit 1 g (3,1 mmol) de l'acide 4'-(2,4-Dioxo-thiazolidin-5-ylidenemethyl)-biphenyl-3-carboxylique, 15 ml de diméthylformamide, 460 mg (3,4 mmol) de 1-hydroxybenzotriazole et 500 µl (3,1 mmol) de n-octylamine. A 0°C, on additionne par portions 0,59 g (3,4 mmol) de chlorhydrate de 1-(3-dimethylaminopropyl)-3-ethylcarbodiimide. On agite pendant trois jours en laissant remonter la température. Le milieu réactionnel est versé dans l'eau et extrait à l'acétate d'éthyle, séché sur sulfate de magnésium, filtré, évaporé. On obtient 450 mg (33%) du produit voulu.

### (e) N-octyl-4'-(2,4-Dioxo-thiazolidin-5-ylmethyl)-biphenyl-3-carboxamide

Dans un réacteur, sous courant d'azote, on introduit 150 mg (0,35 mmol) de *N*-octyl-4'-(2,4-Dioxo-thiazolidin-5-ylidenemethyl)-biphenyl-3-carboxamide, 20 ml de diméthylformamide et 150 mg de palladium sur charbon (10%). Le milieu est chauffé à 80°C et hydrogéné sous une pression de 3 atm pendant 5 heures. Le milieu réactionnel est filtré sur célite. Le filtrat est placé dans l'eau, extrait avec de l'acétate d'éthyle et abondamment lavé à l'eau. La phase organique est séchée sur sulfate de magnésium, filtrée, évaporée. Le résidu est chromatographié sur colonne de silice, élué avec un mélange d'heptane et d'acétate d'éthyle (30-70). On obtient 100 mg (65%) du produit attendu de point de fusion 137-8°C.

### EXEMPLE 24

### N-[4'-(2,4-Dioxo-thiazolidin-5-ylmethyl)-biphenyl-3-ylmethyl]-N-methyl-3-phenyl-propionamide

### (a) N-[4'-(2,4-Dioxo-thiazolidin-5-ylidenemethyl)-biphenyl-3-ylmethyl]- N-methyl-3-phenyl-propionamide

De manière analogue à l'exemple 1(d) par réaction de 500 mg (0,9 mmol) de 5-(3'-Methylaminomethyl-biphenyl-4-ylmethylene)-thiazolidine-2,4-dione ( préparé en 10(c)) avec 0,35 ml (2,3 mmol) de chlorure de 3-phenyl-propionyle, on obtient 220 mg (54%) de produit attendu.

### (b) N-[4'-(2,4-Dioxo-thiazolidin-5-ylmethyl)-biphenyl-3-ylmethyl]-N-methyl-3-phenyl-propionamide

De manière analogue à l'exemple 1(g) à partir de 210 mg (0,45 mmol) de *N*-[4'-(2,4-Dioxo-thiazolidin-5-ylidenemethyl)-biphenyl-3-ylmethyl]-*N*-methyl-3-phenyl-propionamide dans 15 ml de tétrahydrofuranne, on obtient 95 mg (45%) du produit voulu de point de fusion 325°C.

### EXEMPLE 25

### 2-[4'-(2,4-Dioxo-thiazolidin-5-ylmethyl)-biphenyl-3-yl]-N-methyl-N-phenylacetamide

### (a) 2-(3-Bromo-phenyl)- N-methyl-N-phenyl-acetamide

Dans un tricol sous courant d'azote, on introduit 6 g (28 mmol) d'acide (3-Bromo-phenyl)-acétique dans 50 ml de dichlorométhane. A O°C, on additionne goutte à goutte, 2,7 ml (28 mmol) de chlorure d'oxalyle puis on agite à température ambiante pendant 18 heures. Le milieu réactionnel est évaporé à sec, placé dans 10 ml de tétrahydrofuranne, et additionné goutte à goutte à un mélange de 3,2 g (28 mmol) de *N*-méthyl-aniline, 50 ml de tétrahydrofuranne et 4,6 ml (33 mmol) de triéthylamine. Le milieu réactionnel est agité à température ambiante pendant une nuit, plongé dans de l'eau et extrait avec de l'acétate d'éthyle. Après décantation, la phase organique est séchée sur sulfate de magnésium, filtrée, évaporée. On obtient 5, 7 g (67%) du produit voulu de point de fusion 60°C.

### (b) 2-(4'-Acetyl-biphenyl-3-yl)- N-methyl-N-phenyl-acetamide

De manière analogue à l'exemple 1 (e) par réaction de 2,9 g (9,5 mmol) de 2-(3-Bromo-phenyl)- *N*-methyl-*N*-phenyl-acetamide avec 2,2 g (14,2 mmol) d'acide 4-formylbenzèneboronique, on obtient 2,25 g (95%) de produit attendu.

### (c) 2-[4'-(2,4-Dioxo-thiazolidin-5-ylidenemethyl)-biphenyl-3-yl]-N-methyl-N-phenyl-acetamide

De manière analogue à l'exemple 1 (f) par réaction de 1 g (4 mmol) de 2-(4'-Acetyl-biphenyl-3-yl)- *N*-methyl-*N*-phenyl-acetamide avec 460 mg (4 mmol) de 2,4-thiazolidinedione, on obtient 1,2 g (71 %) de produit attendu.

### (d) 2-[4'-(2,4-Dioxo-thiazolidin-5-ylmethyl)-biphenyl-3-yl]-N-methyl-N-phenylacetamide

De manière analogue à l'exemple 1(g) à partir de 1,1 g (2,6 mmol) de 2-[4'-(2,4-Dioxo-thiazolidin-5-ylidenemethyl)-biphenyl-3-yl]-*N*-methyl-*N*-phenyl-acetamide dans 20 ml d'acétate d'éthyle et 20 ml d'éthanol, on obtient 660 mg (60%) du produit voulu de point de fusion 158°C.

### EXEMPLE 26

### N-[4'-(2,4-Dioxo-thiazolidin-5-ylidenemethyl)-biphenyl-3-ylmethyl]-N-propylbenzamide

### (a) (3-Bromo-benzyl)-propyl-carbamate de tert-butyle

De manière analogue à l'exemple 17(d) à partir de 3 g (10,5 mmol) de (3-Bromo-benzyl)-carbamate de tert-butyle et de 1,15 ml (11,5 mmol) d'iodopropane, on obtient 3,35 g (97%) de produit voulu.

### (b) (3-Bromo-benzyl)-propyl-amine

De manière analogue à l'exemple 10(c) à partir de 2 g (6 mmol) de (3-Bromo-benzyl)-propyl-carbamate de tert-butyle, on obtient 1,3 g (92%) du produit voulu.

### (c) N-(3-Bromo-benzyl)- N-propyl-benzamide

De manière analogue à l'exemple 1(d) par réaction de 1,3 g (5,6 mmol) de (3-Bromo-benzyl)-propyl-amine avec 700 µl (6,2 mmol) de chlorure de benzoyle, on obtient 1,4 g (74%) de produit attendu.

### (d) N-(4'-Formyl-biphenyl-3-ylmethyl)-N-propyl-benzamide

De manière analogue à l'exemple 1 (e) par réaction de 1,4 g (4,2 mmol) de *N*-(3-Bromo-benzyl)-*N*-propyl-benzamide avec 940 mg (6,2 mmol) d'acide 4-formylbenzèneboronique, on obtient 780 mg (53%) de produit attendu.

### (e) N-[4'-(2,4-Dioxo-thiazolidin-5-ylidenemethyl)-biphenyl-3-ylmethyl]-N-propyl-benzamide

De manière analogue à l'exemple 1(f) par réaction de 580 mg (1,6 mmol) de *N*-(4'-Formyl-biphenyl-3-ylmethyl)-*N*-propyl-benzamide avec 190 mg (1,6 mmol) de 2,4-thiazolidinedione, on obtient 530 mg (72%) de produit attendu de point de fusion 250-1°C.

### EXEMPLE 27

### [4'-(2,4-dioxo-thiazolidin-5-ylméthyl)-biphenyl-3-yl]-carbamate de tert-butyle

### (a) (3-Bromo-phenyl)-carbamate de tert-butyle

Dans un tricol sous courant d'azote, on introduit 19 ml de 3-bromoaniline dans 300 ml de tétrahydrofuranne. On additionne par portions 8,4 g (209 mmol) d'hydrure de sodium (60% dans l'huile) et on agite jusqu'à cessation du dégagement gazeux. On additionne alors goutte à goutte, 38 g (174,5 mmol) de dicarbonate de di tert-butyle . On chauffe à reflux pendant 36 heures. Le milieu réactionnel est plongé dans de l'eau, extrait à l'acétate d'éthyle. Après décantation, la phase organique est séchée sur sulfate de magnésium, filtrée, évaporée. On obtient 43 g (92%) du produit voulu.

### (b) (4'-Formyl-biphenyl-3-yl)-carbamate de tert-butyle

De manière analogue à l'exemple 1(e) par réaction de 19,3 g (71 mmol) de (3-Bromo-phenyl)-carbamate de *tert*-butyle avec 16 g (107 mmol) d'acide 4-formylbenzèneboronique, on obtient 21 g (63%) de produit attendu.

### (c) [4'-(2,4-Dioxo-thiazolidin-5-ylidenemethyl)-biphenyl-3-yl]-carbamate de tert-butyle

De manière analogue à l'exemple 1(f) par réaction de 9,5 g (32 mmol) de (4'-Formyl-biphenyl-3-yl)-carbamate de *tert*-butyle avec 3,8 g (32 mmol) de 2,4-thiazolidinedione, on obtient 12,7 g (91%) de produit attendu.

### (d) [4'-(2,4-Dioxo-thiazolidin-5-ylmethyl)-biphenyl-3-yl]-carbamate de tert-butyle

De manière analogue à l'exemple 1(g) à partir de 700 mg (1,8 mmol) de [4'-(2,4-Dioxo-thiazolidin-5-ylidenemethyl)-biphenyl-3-yl]-carbamate de *tert*-butyle dans 10 ml de dioxanne, on obtient 700 mg (60%) du produit voulu de point de fusion 158°C.

### EXEMPLE 28

### N-[4'-(2,4-Dioxo-thiazolidin-5-ylmethyl)-biphenyl-3-ylmethyl]-3,4-diethoxy-N-methyl-benzamide

### (a) N-[4'-(2,4-Dioxo-thiazolidin-5-ylidenemethyl)-biphenyl-3-ylmethyl]-3,4-diethoxy-N-methyl-benzamide

De manière analogue à l'exemple 1(d) par réaction de 500 mg (0,9 mmol) de 5-(3'-Methylaminomethyl-biphenyl-4-ylmethylene)-thiazolidine-2,4-dione ( préparé en 10(c) ) avec 520 mg (2,3 mmol) de chlorure de 3,4-Diethoxybenzoyle, on obtient 370 mg (80%) de produit attendu.

### (b) N-[4'-(2,4-Dioxo-thiazolidin-5-ylmethyl)-biphenyl-3-ylmethyl]-3,4-diethoxy-N-methyl-benzamide

De manière analogue à l'exemple 1(g) à partir de 360 mg (0,7 mmol) de N-[4'-(2,4-Dioxo-thiazolidin-5-ylidenemethyl)-biphenyl-3-ylmethyl]-3,4-diethoxy-N-methyl-benzamide dans 15 ml de méthanol, on obtient 150 mg (40%) du produit voulu de point de fusion 66°C.

### EXEMPLE 29

### Acide 2-(3'-{[Methyl-(1-phenyl-methanoyl)-amino]-methyl}-biphenyl-4-ylmethyl)-malonamique

On introduit 180 mg (0,4 mmol) de 2-(3'-{[Methyl-(1-phenyl-methanoyl)-amino]-methyl}-biphenyl-4-ylmethyl)-malonamate de methyle ( préparé en 16(d) ) dans 2 ml de méthanol, 2 ml de tétrahydrofuranne et 250 µl (0,5 mmol) d'une solution aqueuse d'hydroxyde de sodium 2N. Le milieu réactionnel est agité à température ambiante pendant 36 heures, acidifié à pH 3 avec une solution d'acide chlorhydrique, extrait à l'acétate d'éthyle. La phase organique est séchée sur sulfate de magnésium, filtrée, évaporée. On obtient 80 mg (46%) du produit voulu de point de fusion 117°C.

### EXEMPLE 30

### N-benzyl-N-méthyl-4'-(2,4-Dioxo-thiazolidin-5-ylmethyl)-biphenyl-3-carboxamide

### (a) N-benzyl-N-méthyl-4'-(2,4-Dioxo-thiazolidin-5-ylidenemethyl)-biphenyl-3-carboxamide

Dans un tricol, sous courant d'azote, on introduit 500 mg (1,54 mmol) de l' acide 4'-(2,4-Dioxo-thiazolidin-5-ylidenemethyl)-biphenyl-3-carboxylique ( préparé en 23(c) ), 5 ml de diméthylformamide, 230 mg (1,7 mmol) de 1-hydroxybenzotriazole et 200 µl (1,5 mmol) de benzyl-methyl-amine. A 0°C, on additionne par portions 350 mg (1,7 mmol) de dicyclohexylcarbodiimide. On agite pendant 18 heures en laissant remonter la température. Le milieu réactionnel est plongé dans l'eau et extrait à l'acétate d'éthyle, séché sur sulfate de magnésium, filtré, évaporé. Le résidu obtenu est lavé avec du dichlorométhane, filtré. On obtient 400 mg (61 %) du produit voulu.

### (b) N-benzyl-N-méthyl-4'-(2,4-Dioxo-thiazolidin-5-ylmethyl)-biphenyl-3-carboxamide

De manière analogue à l'exemple 1(g) à partir de 400 mg (0,9 mmol) de *N*-benzyl-*N*-méthyl-4'-(2,4-Dioxo-thiazolidin-5-ylidenemethyl)-biphenyl-3-carboxamide
dans 5 ml de dioxanne, on obtient 170 mg (43%) du produit voulu de point de fusion 66°C.

### EXEMPLE 31

### N-benzyl-4'-(2,4-Dioxo-thiazolidin-5-ylmethyl)-biphenyl-3-carboxamide

### (a) N-benzyl-4'-(2,4-Dioxo-thiazolidin-5-ylidenemethyl)-biphenyl-3-carboxamide

Dans un tricol, sous courant d'azote, on introduit 600 mg (1,84 mmol) de l' acide 4'-(2,4-Dioxo-thiazolidin-5-ylidenemethyl)-biphenyl-3-carboxylique ( préparé en 23(c) ), 5 ml de diméthylformamide, 340 mg (2,5 mmol) de 1-hydroxybenzotriazole et 250 µl (2,3 mmol) de benzylamine. A 0°C, on additionne par portions 480 mg (2,5 mmol) de chlorhydrate de 1-(3-dimethylaminopropyl)-3-ethylcarbodiimide. On agite pendant 24 heures en laissant remonter la température. Le milieu réactionnel est plongé dans l'eau et extrait à l'acétate d'éthyle, séché sur sulfate de magnésium, filtré, évaporé. Le résidu obtenu est lavé avec du dichlorométhane, filtré. On obtient 460mg (60%) du produit voulu.

### (b) N-benzyl-4'-(2,4-Dioxo-thiazolidin-5-ylmethyl)-biphenyl-3-carboxamide

De manière analogue à l'exemple 1(g) à partir de 430 mg (1 mmol) de *N*-benzyl- 4'-(2,4-Dioxo-thiazolidin-5-ylidenemethyl)-biphenyl-3-carboxamide dans 7 ml de diméthylformamide, on obtient 100 mg (23%) du produit voulu de point de fusion 148°C.

### EXEMPLE 32

### N-[4'-(2,4-dioxo-thiazolidin-5-ylmethyl)-biphenyl-3-ylmethyl]-N-methyldécanamide

### (a) N-[4'-(2,4-dioxo-thiazolidin-5-ylidenemethyl)-biphenyl-3-ylmethyl]-N-methyldécanamide

De manière analogue à l'exemple 1(d) par réaction de 1 g (1,8 mmol) de 5-(3'-Methylaminomethyl-biphenyl-4-ylidenemethyl)-thiazolidine-2,4-dione ( préparé en 10(c) )avec 410 µl (2 mmol) de chlorure de décanoyle, on obtient 500 mg (60%) du produit attendu.

### (b) N-[4'-(2,4-dioxo-thiazolidin-5-ylmethyl)-biphenyl-3-ylmethyl]-N-methyldécanamide

De manière analogue à l'exemple 1(g) à partir de 460 mg (1 mmol) de N-[4'-(2,4-dioxo-thiazolidin-5-ylidenemethyl)-biphenyl-3-ylmethyl]-*N*-methyldécanamide
dans 8 ml de dioxanne, on obtient 300 mg (65%) du produit voulu sous la forme d'un film.

### EXEMPLE 33

### N-[4'-(2,4-Dioxo-thiazolidin-5-ylmethyl)-biphenyl-3-yl]-2-phenyl-acetamide

### (a) 5-(3'-Amino-biphenyl-4-ylmethylene)-thiazolidine-2,4-dione

De manière analogue à 10c, à partir de 5 g (13 mmol) de [4'-(2,4-Dioxo-thiazolidin-5-ylidenemethyl)-biphenyl-3-yl]-carbamate de *tert*-butyle ( préparé en 27(c) ), on obtient 5,2 g (100%) du produit attendu.

### (b) N-[4'-(2,4-Dioxo-thiazolidin-5-ylidenemethyl)-biphenyl-3-yl]-2-phenylacetamide

De manière analogue à l'exemple 1 (d) par réaction de 1,2 g (2,3 mmol) de 5-(3'-Amino-biphenyl-4-ylmethylene)-thiazolidine-2,4-dione avec 430 µl (3 mmol) de chlorure de phénylacétyle, on obtient 920 mg (97%) de produit attendu.

### (c) N-[4'-(2,4-Dioxo-thiazolidin-5-ylidenemethyl)-biphenyl-3-yl]-2-phenylacetamide

De manière analogue à l'exemple 1 (g) à partir de 580 mg (1,4 mmol) de *N*-[4'-(2,4-Dioxo-thiazolidin-5-ylidenemethyl)-biphenyl-3-yl]-2-phenyl-acetamide dans 10 ml d'un mélange dioxanne-méthanol (50-50) sous 3 atm, on obtient 140 mg (15%) du produit voulu de point de fusion 165°C.

### EXEMPLE 34

### N-[4'-(2,4-dioxo-thiazolidin-5-ylmethyl)-biphenyl-3-ylmethyl]-N-methyloctanamide

### (a) N-[4'-(2,4-dioxo-thiazolidin-5-ylidenemethyl)-biphenyl-3-ylmethyl]-N-methyloctanamide

De manière analogue à l'exemple 1(d) par réaction de 500 mg (0,9 mmol) de 5-(3'-Methylaminomethyl-biphenyl-4-ylidenemethyl)-thiazolidine-2,4-dione préparé en 10c avec 170 µl (1 mmol) de chlorure d'octanoyle, on obtient 250 mg (62%) de produit attendu.

### (b) N-[4'-(2,4-dioxo-thiazolidin-5-ylmethyl)-biphenyl-3-ylmethyl]-N-methyloctanamide

De manière analogue à l'exemple 1 (g) à partir de 220 mg (0,5 mmol) de *N*-[4'-(2,4-dioxo-thiazolidin-5-ylidenemethyl)-biphenyl-3-ylmethyl]-*N*-methyloctanamide
dans 10 ml de dioxanne, on obtient 120 mg (53%) du produit voulu de point de fusion 36°C.

### EXEMPLE 35

### N-[4'-(2,4-dioxo-thiazolidin-5-ylmethyl)-biphenyl-3-ylmethyl]-N-methylheptanamide

### (a) N-[4'-(2,4-dioxo-thiazolidin-5-ylidenemethyl)-biphenyl-3-ylmethyl]-N-methylheptanamide

De manière analogue à l'exemple 1(d) par réaction de 1 g (1,8 mmol) de 5-(3'-Methylaminomethyl-biphenyl-4-ylidenemethyl)-thiazolidine-2,4-dione ( préparé en 10(c) )avec 300 µl (2 mmol) de chlorure d'heptanoyle, on obtient 450 mg (57%) du produit attendu.

### (b) N-[4'-(2,4-dioxo-thiazolidin-5-ylmethyl)-biphenyl-3-ylmethyl]-N-methylheptanamide

De manière analogue à l'exemple 1(g) à partir de 360 mg (0,8 mmol) de *N*-[4'-(2,4-dioxo-thiazolidin-5-ylidenemethyl)-biphenyl-3-ylmethyl]-*N*-methylheptanamide
dans 15 ml de dioxanne, sous 3 atm, on obtient 230 mg (66%) du produit voulu sous la forme d'un film incolore.

### EXEMPLE 36

### Acide N-Hydroxy-2-(3'-{[methyl-(1-phenyl-methanoyl)-amino]-methyl}-biphenyl-4-ylmethyl)-malonamique

On chauffe à 60°C pendant 18 heures un mélange de 130 mg (0,3 mmol) de 2-(3'-{[Methyl-(1-phenyl-methanoyl)-amino]-methyl}-biphenyl-4-ylmethyl)-malonate de diméthyle ( préparé en 15(b) ), 2 ml de méthanol, 2 ml de tétrahydrofuranne, 480 mg de carbonate de sodium (4,5 mmol) et 200 mg (1,5 mmol) de chlorhydrate d'hydroxylamine. Le milieu réactionnel est neutralisé à pH 6-7 avec une solution d'acide chlorhydrique, extrait à l'acétate d'éthyle. La phase organique est séchée sur sulfate de magnésium, filtrée, évaporée. Le résidu obtenu est purifié par chromatographie sur colonne de silice élué avec un mélange d'heptane et d'acétate d'éthyle (75-25). On obtient 90 mg (72%) du produit voulu de point de fusion 60°C.

### EXEMPLE 37

### N-[4'-(2,4-Dioxo-thiazolidin-5-ylmethyl)-biphenyl-3-ylmethyl]-N-methyl-2-phenyl-acetamic

### (a) (4'-Formyl-biphenyl-3-ylmethyl)-methyl-carbamate de tert-butyle.

De manière analogue à l'exemple 1(e), à partir de 125 g (410 mmol) de (3-Bromo-benzyl)-*N*-méthylcarbamate de tert-butyle ( préparé en 1(b) ) et de 82 g (510 mmol) d'acide 4-formylbenzèneboronique, on obtient 90 g (67%) du produit voulu.

### (b) [4'-(2,4-Dioxo-thiazolidin-5-ylidenemethyl)-biphenyl-3-ylmethyl]-methylcarbamate de tert-butyle.

De manière analogue à l'exemple 1(f), à partir de 75 g (230 mmol) de (4'-Formyl-biphenyl-3-ylmethyl)-methyl-carbamate de tert-butyle et de 35 g (275 mmol) de 2,4 thiazolidinedione, on obtient 84 g (86%) du produit attendu.

### (c) 4'-(2,4-Dioxo-thiazolidin-5-ylmethyl)-biphenyl-3-ylmethyl]-methyl-carbamate de tert-butyle.

Dans un réacteur, on place 30 g (71 mmol) de [4'-(2,4-Dioxo-thiazolidin-5-ylidenemethyl)-biphenyl-3-ylmethyl]-methyl-carbamate de tert-butyle dans 500 ml de dioxanne . Le milieu réactionnel est dégazé puis on ajoute 30 g (un équivalent massique) de palladium sur charbon 10%. Le système est placé sous 3 bars d'hydrogène et chauffé à 50°C pendant sept heures. On filtre le milieu réactionnel sur célite, évapore et purifie le résidu obtenu par chromatographie sur colonne de silice avec un mélange éluant de dichlorométhane et méthanol (98-2). On recueille après évaporation des solvants 18 g (60%) du produit attendu.

### (d) 5-(3'-Methylaminomethyl-biphenyl-4-ylmethyl)-thiazolidine-2,4-dione

A une solution de 18 g (42 mmol) de 4'-(2,4-Dioxo-thiazolidin-5-ylmethyl)-biphenyl-3-ylmethyl]-methyl-carbamate de tert-butyle dans 250 ml de dichlorométhane , on ajoute 16 ml (210 mmol) d'acide trifluoroacétique. Le milieu réactionnel est agité à température ambiante pendant 18 heures puis évaporé à sec. Le résidu obtenu est lavé à l'acétate d'éthyle, séché. On recueille 14 g (78%) de produit sous forme de trifluoroacétate.

### (e) N-[4'-(2,4-Dioxo-thiazolidin-5-ylmethyl)-biphenyl-3-ylmethyl]-N-methyl-2-phenyl-acetamide

Dans un ballon et sous courant d'azote, on introduit 500 mg (1,1 mmol) du trifluoroacétate de 5-(3'-Methylaminomethyl-biphenyl-4-ylmethyl)-thiazolidine-2,4-dione, 2 ml de tétrahydrofuranne et 500 µl (3,3 mmol) de triéthylamine. On ajoute goutte à goutte 230 µl de chlorure de phénylacétyle et agite à température ambiante pendant une heure. On verse le milieu réactionnel dans l'eau, extrait avec de l'acétate d'éthyle, décante la phase organique, lave à l'eau, sèche sur sulfate de magnésium, évapore. Le résidu obtenu est purifié par chromatographie sur colonne de silice élué avec un mélange de dichlorométhane et de méthanol (97-3). On obtient 360 mg (72%) de *N*-[4'-(2,4-Dioxo-thiazolidin-5-ylmethyl)-biphenyl-3-ylmethyl]-*N*-methyl-2-phenyl-acetamide sous la forme d'un solide blanc de point de fusion 73°C.

### EXEMPLE 38

### N-[4'-(2,4-Dioxo-thiazolidin-5-ylmethyl)-biphenyl-3-ylmethyl]-4-methoxy-N-methyl-benzami

De manière analogue à l'exemple 37(e), par réaction de 500 mg (1,1 mmol) du trifluoroacétate de 5-(3'-Methylaminomethyl-biphenyl-4-ylmethyl)-thiazolidine-2,4-dione avec 170 µl (1,25 mmol) de chlorure de 4-méthoxybenzoyle, on obtient après purification 300 mg (50%) de *N*-[4'-(2,4-Dioxothiazolidin-5-ylmethyl)-biphenyl-3-ylmethyl]-4-methoxy-*N*-methyl-benzamide sous la forme d'un solide blanc de point de fusion 170°C.

### EXEMPLE 39

### N-[4'-(2,4-Dioxo-thiazolidin-5-ylmethyl)-biphenyl-3-ylmethyl]-3-methoxy-N-methylbenzamide

De manière analogue à l'exemple 37(e), par réaction de 540 mg (1,2 mmol) du trifluoroacétate de 5-(3'-Methylaminomethyl-biphenyl-4-ylmethyl)-thiazolidine-2,4- dione avec 300 µl (2 mmol) de chlorure de 3-méthoxybenzoyle, on obtient après purification 400 mg (70%) de *N*-[4'-(2,4-Dioxo-thiazolidin-5-ylmethyl)-biphenyl-3-ylmethyl]-3-methoxy-*N*-methyl-benzamide sous la forme d'un solide blanc de point de fusion 173°C.

### EXEMPLE 40

### N-[4'-(2,4-Dioxo-thiazolidin-5-ylmethyl)-biphenyl-3-ylmethyl]-3 -N-dimethyl-benzamide

De manière analogue à l'exemple 37(e), par réaction de 500 mg (1,1 mmol) du trifluoroacétate de 5-(3'-Methylaminomethyl-biphenyl-4-ylmethyl)-thiazolidine-2,4-dione avec 170 µl (1,25 mmol) de chlorure de 3-méthylbenzoyle, on obtient après purification 350 mg (70%) de *N*-[4'-(2,4-Dioxo-thiazolidin-5-ylmethyl)-biphenyl-3-ylmethyl]-3- *N*-dimethyl-benzamide sous la forme d'un solide blanc de point de fusion 98°C.

### EXEMPLE 41

### N-[4'-(2,4-Dioxo-thiazolidin-5-ylmethyl)-biphenyl-3-ylmethyl]-N-methyl-4-propylbenzamide

De manière analogue à l'exemple 37(e), par réaction de 500 mg (1,1 mmol) du trifluoroacétate de 5-(3'-Methylaminomethyl-biphenyl-4-ylmethyl)-thiazolidine-2,4-dione avec 210 µl (1,2 mmol) de chlorure de 4-propylbenzoyle, on obtient après purification 300 mg (55%) de *N*-[4'-(2,4-Dioxo-thiazolidin-5-ylmethyl)-biphenyl-3-ylmethyl]-*N*-methyl-4-propyl-benzamide de point de fusion 280°C.

### EXEMPLE 42

### N-[4'-(2,4-Dioxo-thiazolidin-5-ylmethyl)-biphenyl-3-ylmethyl]-4,N-dimethylbenzamide.

De manière analogue à l'exemple 37(e), par réaction de 500 mg (1,1 mmol) du trifluoroacétate de 5-(3'-Methylaminomethyl-biphenyl-4-ylmethyl)-thiazolidine-2,4-dione avec 170 µl (1,25 mmol) de chlorure de 4-methylbenzoyle, on obtient après purification 350 mg (70%) de *N*-[4'-(2,4-Dioxo-thiazolidin-5-ylmethyl)-biphenyl-3-ylmethyl]-4 *N*-dimethyl-benzamide sous la forme d'un solide blanc de point de fusion 198°C.

### EXEMPLE 43

### N- [4'-(2,4-dioxo-thiazolidin-5-ylmethyl)-biphenyl-3-ylmethyl]-N-methyl- Isoxazole-5-carboxamide

De manière analogue à l'exemple 37(e), par réaction de 500 mg (1,1 mmol) du trifluoroacétate de 5-(3'-Methylaminomethyl-biphenyl-4-ylmethyl)-thiazolidine-2,4-dione avec 170 mg (1,3 mmol) du chlorure de l'acide 5-isoxazolecarboxylique, on obtient après purification 120 mg (26%) de N-[4'-(2,4-dioxo-thiazolidin-5-ylmethyl)-biphenyl-3-ylmethyl]-N-methyl-Isoxazole-5-carboxamide sous la forme d'un solide blanc de point de fusion 160°C.

### EXEMPLE 44

### N-[4'-(2,4-Dioxo-thiazolidin-5-ylmethyl)-biphenyl-3-ylmethyl]-4-ethoxy-N-methylbenzamide

De manière analogue à l'exemple 37(e), par réaction de 1 g (2,2 mmol) du trifluoroacétate de 5-(3'-Methylaminomethyl-biphenyl-4-ylmethyl)-thiazolidine-2,4-dione avec 460 mg (2,5 mmol) de chlorure de 4-ethoxybenzoyle, on obtient après purification 960 mg (88%) de *N*-[4'-(2,4-Dioxo-thiazolidin-5-ylmethyl)-biphenyl-3-ylmethyl]-4-ethoxy-*N*-methyl-benzamide sous la forme d'un solide blanc de point de fusion 182°C.

### EXEMPLE 45

### N-[4'-(2,4-Dioxo-thiazolidin-5-ylmethyl)-biphenyl-3-ylmethyl]-4-fluoro-N-methylbenzamide

De manière analogue à l'exemple 37(e), par réaction de 1 g (2,2 mmol) du trifluoroacétate de 5-(3'-Methylaminomethyl-biphenyl-4-ylmethyl)-thiazolidine-2,4-dione avec 290 µl (2,5 mmol) de chlorure de 4-fluorobenzoyle, on obtient après purification 1 g (98%) de *N*-[4'-(2,4-Dioxo-thiazolidin-5-ylmethyl)-biphenyl-3-ylmethyl]-4-fluoro-*N*-methylbenzamide de point de fusion 212°C.

### EXEMPLE 46

### 4-Dimethylamino-N-[4'-(2,4-dioxo-thiazolidin-5-ylmethyl)-biphenyl-3-ylmethyl]-N-methyl-benzamide

De manière analogue à l'exemple 37(e), par réaction de 1 g (2,2 mmol) du trifluoroacétate de 5-(3'-Methylaminomethyl-biphenyl-4-ylmethyl)-thiazolidine-2,4-dione avec 500 mg (3 mmol) de chlorure de 4-dimethylaminobenzoyle, on obtient après purification 200 mg (20%) de 4-diméthylamino-*N*-[4'-(2,4-dioxo-thiazolidin-5-ylmethyl)-biphenyl-3-ylmethyl]-*N*-methyl-benzamide de point de fusion 100°C.

### EXEMPLE 47

### N-[4'-(2,4-Dioxo-thiazolidin-5-ylmethyl)-biphenyl-3-ylmethyl]- N-methyl-nicotinamide

De manière analogue à l'exemple 37(e), par réaction de 1 g (2,2 mmol) du trifluoroacétate de 5-(3'-Methylaminomethyl-biphenyl-4-ylmethyl)-thiazolidine-2,4-dione avec 450 mg (2,5 mmol) de chlorure de nicotinoyle, on obtient après purification 400 mg (40%) de *N*-[4'-(2,4-Dioxo-thiazolidin-5-ylmethyl)-biphenyl-3-ylmethyl]- *N*-methyl-nicotinamide sous la forme d'un solide blanc de point de fusion 115°C.

### EXEMPLE 48

### 3,5-Dichloro-N-[4'-(2,4-dioxo-thiazolidin-5-ylmethyl)-biphenyl-3-ylmethyl]-N-methyl-benzamide

De manière analogue à l'exemple 37(e), par réaction de 1 g (2,2 mmol) du trifluoroacétate de 5-(3'-Methylaminomethyl-biphenyl-4-ylmethyl)-thiazolidine-2,4-dione avec 350 µl (2,5 mmol) de chlorure de 3,5-dichlorobenzoyle, on obtient après purification 400 mg (50%) de 3,5-dichloro-*N*-[4'-(2,4-dioxo-thiazolidin-5-ylmethyl)-biphenyl-3-ylmethyl]-*N*-methyl-benzamide sous la forme d'un solide blanc de point de fusion 106°C.

### EXEMPLE 49

### N-[4'-(2,4-dioxo-thiazolidin-5-ylmethyl)-biphenyl-3-ylmethyl]-N-methyl-Thiophene-2-carboxamide

De manière analogue à l'exemple 37(e), par réaction de 1 g (2,2 mmol) du trifluoroacétate de 5-(3'-Methylaminomethyl-biphenyl-4-ylmethyl)-thiazolidine-2,4-dione avec 200 µl (2,5 mmol) de chlorure de l'acide 2-thiophènecarboxylique, on obtient après purification 500 mg (50%) de N-[4'-(2,4-dioxo-thiazolidin-5-ylmethyl)-biphenyl-3-ylmethyl]-N-methyl- thiophene-2-carboxamide sous la forme d'un solide blanc de point de fusion 160°C.

### EXEMPLE 50

### N- [4'-(2,4-dioxo-thiazolidin-5-ylmethyl)-biphenyl-3-ylmethyl]-N-methyl-Hexanamide

De manière analogue à l'exemple 37(e), par réaction de 500 mg (1,5 mmol) de 5-(3'-Methylaminomethyl-biphenyl-4-ylmethyl)-thiazolidine-2,4-dione avec 220 µl (1,6 mmol) de chlorure d'hexanoyle, on obtient après purification 430 mg (66%) de N-[4'-(2,4-dioxo-thiazolidin-5-ylmethyl)-biphenyl-3-ylmethyl]-N-methyl-hexanamide de point de fusion 45°C.

### EXEMPLE 51

### N-[4'-(2,4-Dioxo-thiazolidin-5-ylmethyl)-biphenyl-3-ylmethyl]-2-methoxy-N-methylbenzamide

De manière analogue à l'exemple 37(e), par réaction de 500 mg (1,5 mmol) de 5-(3'-Methylaminomethyl-biphenyl-4-ylmethyl)-thiazolidine-2,4-dione avec 230 µl (1,5 mmol) de chlorure de 2-methoxybenzoyle, on obtient après purification 500 mg (70%) de *N*-[4'-(2,4-Dioxo-thiazolidin-5-ylmethyl)-biphenyl-3-ylmethyl]-2-methoxy-*N*-methyl-benzamide sous la forme d'un solide blanc de point de fusion 96°C.

### EXEMPLE 52

### N-[4'-(2,4-dioxo-thiazolidin-5-ylmethyl)-biphenyl-3-ylmethyl]-N-methylpyridine-2-carboxamide

Préparation du chlorure de l'acide picolinique:
A 500 mg (4,1 mmol) d'acide picolinique placés dans 9 ml de dichlorométhane , on additionne 800 µl (4,1 mmol) de dicyclohexylamine et on agite à température ambiante pendant 30 minutes. On additionne ensuite 300 µl (4,1 mmol) de chlorure de thionyle et agite à température ambiante pendant 2 heures. Le milieu
réactionnel est dilué avec de l'éther ethylique ; Le précipité est filtré, rincé à l'éther éthylique. Par concentration du filtrat, on obtient le chlorure d'acide attendu.

De manière analogue à l'exemple 37(e), par réaction de 500 mg (1,5 mmol) de 5-(3'-Methylaminomethyl-biphenyl-4-ylmethyl)-thiazolidine-2,4-dione avec 220 mg (1,6 mmol) du chlorure d'acide précédent, on obtient après purification 330 mg (50%) de N-[4'-(2,4-dioxo-thiazolidin-5-ylmethyl)-biphenyl-3-ylmethyl]-N-methyl- pyridine-2-carboxamide sous forme d'une huile incolore.

### EXEMPLE 53

### N- [4'-(2,4-dioxo-thiazolidin-5-ylmethyl)-biphenyl-3-ylmethyl]-N-methylfuran-2-carboxamide

Préparation du chlorure de l'acide 2-furanoïque:
A 500 mg (4,5 mmol) d'acide 2-furanoique placés dans 5 ml de dichlorométhane , on additionne 900 µl (4,5 mmol) de dicyclohexylamine et on agite à température ambiante pendant 30 minutes. On additionne ensuite 300 µl (4,5 mmol) de chlorure de thionyle et agite à température ambiante pendant 2 heures. Le milieu réactionnel est dilué avec de l'éther ethylique ; Le précipité est filtré, rincé à l'éther ethylique. Par concentration du filtrat, on obtient le chlorure de l'acide 2-furanoïque.

De manière analogue à l'exemple 37(e), par réaction de 500 mg (1,5 mmol) de 5-(3'-Methylaminomethyl-biphenyl-4-ylmethyl)-thiazolidine-2,4-dione avec 200 mg (1,5 mmol) de chlorure de l'acide 2-furanoïque, on obtient après purification 350 mg (50%) de N-[4'-(2,4-dioxo-thiazolidin-5-ylmethyl)-biphenyl-3-ylmethyl]- N-methyl-furan-2-carboxamide sous la forme d'un solide blanc de point de fusion 150°C.

### EXEMPLE 54

### 4-Butoxy-N-[4'-(2,4-dioxo-thiazolidin-5-ylmethyl)-biphenyl-3-ylmethyl]-N-methyl-benzamide

De manière analogue à l'exemple 37(e), par réaction de 500 mg (1,5 mmol) de 5-(3'-Methylaminomethyl-biphenyl-4-ylmethyl)-thiazolidine-2,4-dione avec 290 µl (1,5 mmol) de chlorure de 4-butoxybenzoyle, on obtient après purification 550 mg (83%) de 4-Butoxy-*N*-[4'-(2,4-dioxo-thiazolidin-5-ylmethyl)-biphenyl-3-ylmethyl]-*N*-methylbenzamide sous la forme d'un solide blanc de point de fusion 116°C.

### EXEMPLE 55

### N- [4'-(2,4-dioxo-thiazolidin-5-ylmethyl)-biphenyl-3-ylmethyl]-N-methyl-Thiophene-3-carboxamide

Préparation du chlorure de l'acide 3-thiophènecarboxylique:
A 500 mg (3,9 mmol) d'acide 3-thiophène carboxylique placés dans 5 ml de dichlorométhane , on additionne 800 µl (3,9 mmol) de dicyclohexylamine et on agite à température ambiante pendant 30 minutes. On additionne ensuite 300 µl (3,9 mmol) de chlorure de thionyle et agite à température ambiante pendant 2 heures. Le milieu réactionnel est dilué avec de l'éther ethylique ; Le précipité est filtré, rincé à l'éther ethylique. Par concentration du filtrat, on obtient le chlorure d'acide attendu.

De manière analogue à l'exemple 37(e), par réaction de 500 mg (1,5 mmol) de 5-(3'-Methylaminomethyl-biphenyl-4-ylmethyl)-thiazolidine-2,4-dione avec 200 mg (1,5 mmol) de chlorure de l'acide 3-thiophène carboxylique, on obtient après purification 450 mg (68%) de N-[4'-(2,4-dioxo-thiazolidin-5-ylmethyl)-biphenyl-3-ylmethyl]-N-methyl- thiophene-3-carboxamide sous la forme d'une poudre blanche de point de fusion 150°C.

### EXEMPLE 56

### Acetate de 4-{[4'-(2,4-dioxo-thiazolidin-5-ylmethyl)-biphenyl-3-ylmethyl]-methylcarbamoyl}-phenyle

Préparation du chlorure de 4-acétoxybenzoyle:
A 1 g (5,6 mmol) d'acide 4-acétoxybenzoique placé dans 10 ml de dichlorométhane , on additionne 1,1 ml (5,6 mmol) de dicyclohexylamine et on agite à température ambiante pendant 30 minutes. On additionne ensuite 400 µl (5,6 mmol) de chlorure de thionyle et agite à température ambiante pendant 2 heures. Le milieu réactionnel est dilué avec de l'éther ethylique ; Le précipité est filtré, rincé à l'éther ethylique. Par concentration du filtrat, on obtient le chlorure de 4-acétoxybenzoyle.

De manière analogue à l'exemple 37(e), par réaction de 1 g (3 mmol) de 5-(3'-Methylaminomethyl-biphenyl-4-ylmethyl)-thiazolidine-2,4-dione avec 600 mg (3 mmol) de chlorure de 4-acétoxybenzoyle, on obtient après purification 1,3 g (90%) d'acetate de 4-{[4'-(2,4-dioxo-thiazolidin-5-ylmethyl)-biphenyl-3-ylmethyl]-methyl-carbamoyl}-phenyle sous la forme d'une poudre blanche de point de fusion 167°C.

### EXEMPLE 57

### N-[4'-(2,4-Dioxo-thiazolidin-5-ylmethyl)-biphenyl-3-ylmethyl]-4-hydroxy-N-methylbenzamide

A 350 mg (6,7 mmol) d'acetate de 4-{[4'-(2,4-dioxo-thiazolidin-5-ylmethyl)-biphenyl-3-ylmethyl]-methyl-carbamoyl}-phenyle (obtenu dans l'exemple 56) dans 5 ml de tétrahydrofuranne et 500 µl d'eau, on ajoute 110 mg (1,3 mmol) d'hydrogénocarbonate de sodium. Le milieu réactionnel est agité à température ambiante pendant 48 heures puis dilué à l'acétate d'éthyle, acidifié à pH 3-4 avec une solution d'acide chlorhydrique 1 N. La phase organique est décantée, séchée sur sulfate de sodium, filtrée, évaporée. Le résidu obtenu est purifié par chromatographie sur colonne de silice élué avec un mélange dichlorométhane-méthanol 99-1. Après évaporation des solvants, on recueille 250 mg (83%) de *N*-[4'-(2,4-Dioxo-thiazolidin-5-ylmethyl)-biphenyl-3-ylmethyl]-4-hydroxy-*N*-methyl-benzamide sous la forme d'une poudre blanche de point de fusion 110°C.

### EXEMPLE 58

### N-[4'-(2,4-Dioxo-thiazolidin-5-ylmethyl)-biphenyl-3-ylmethyl]-2 N-dimethyl-benzamide

De manière analogue à l'exemple 37(e), par réaction de 400 mg (1,2 mmol) de 5-(3'-Methylaminomethyl-biphenyl-4-ylmethyl)-thiazolidine-2,4-dione avec 160 µl (1,2 mmol) de chlorure de 2-méthylbenzoyle, on obtient après purification 500 mg (94%) de *N*-[4'-(2,4-Dioxo-thiazolidin-5-ylmethyl)-biphenyl-3-ylmethyl]-2 *N*-dimethyl-benzamide sous la forme d'une poudre blanche de point de fusion 78°C.

### EXEMPLE 59

### 2-Butyl-N- [4'-(2,4-dioxo-thiazolidin-5-ylmethyl)-biphenyl-3-ylmethyl]-N-methyloctanamide

Préparation du chlorure de 2-butyl-octanoyle:
A 500 mg (2,5 mmol) d'acide 2-butyl-octanoique placés dans 5 ml de dichlorométhane , on additionne 500 µl (2,5 mmol) de dicyclohexylamine et on agite à température ambiante pendant 30 minutes. On additionne ensuite 200 µl (2,5 mmol) de chlorure de thionyle et agite à température ambiante pendant 2 heures. Le milieu réactionnel est dilué avec de l'éther ethylique ; Le précipité est filtré, rincé à l'éther ethylique. Par concentration du filtrat, on obtient le chlorure de 2-butyl-octanoyle.

De manière analogue à l'exemple 37(e), par réaction de 500 mg (1,5 mmol) de 5-(3'-Methylaminomethyl-biphenyl-4-ylmethyl)-thiazolidine-2,4-dione avec 340 mg (1,5 mmol) de chlorure de 2-butyl-octanoyle, on obtient après purification 430 mg (55%) de 2-Butyl-N- [4'-(2,4-dioxo-thiazolidin-5-ylmethyl)-biphenyl-3-ylmethyl]-N-methyl-octanamide sous la forme d'une huile incolore.

### EXEMPLE 60

### 4-Acetylamino-N-[4'-(2,4-dioxo-thiazolidin-5-ylmethyl)-biphenyl-3-ylmethyl]-N-methyl-benzamide

A une solution de 270 mg (1,5 mmol) d'acide 4-acétamidobenzoique, 230 µl (1,6 mmol) de triéthylamine, 230 mg (1,7 mmol) de 1-hydroxybenzotriazole et de 500 mg (1,5 mmol) de 5-(3'-Methylaminomethyl-biphenyl-4-ylmethyl)-thiazolidine-2,4-dione dans 5 ml de dichlorométhane, on additionne par fractions, à 0°C, 320 mg (1,7 mmol) du chlorhydrate de 1-(3-dimethylaminopropyl)-3-ethylcarbodiimide. Le milieu réactionnel est agité de 0°C à température ambiante pendant 18 heures, lavé avec une solution saturée d'hydrogénocarbonate de sodium, extrait au dichlorométhane. La phase organique est décantée, séchée sur sulfate de magnésium, filtrée et évaporée. Le résidu obtenu est purifié par chromatographie sur colonne de silice élué avec un mélange dichlorométhane - méthanol 99-1. On recueille après évaporation des solvants 600 mg (80%) de 4-Acetylamino-*N*-[4'-(2,4-dioxo-thiazolidin-5-ylmethyl)-biphenyl-3-ylmethyl]-*N*-methylbenzamide sous la forme d'un solide blanc de point de fusion 207°C.

### EXEMPLE 61

### N-[4'-(2,4-Dioxo-thiazolidin-5-ylmethyl)-biphenyl-3-ylmethyl]-N-methyl-carbamate d'hexyle

A une solution de 1 g (2,3 mmol) du trifluoroacétate de 5-(3'-Methylaminomethyl-biphenyl-4-ylmethyl)-thiazolidine-2,4-dione et de 1 ml (7,0 mmol) de triéthylamine dans 15 ml de dichlorométhane, on ajoute goutte à goutte une solution de 400 µl (2,5 mmol) de chloroformate d'hexyle dans 5 ml de dichlorométhane. Le milieu réactionnel est agité à température ambiante pendant 3 heures, placé dans de l'eau et extrait à l'acétate d'éthyle. La phase organique est décantée, séchée sur sulfate de magnésium, filtrée, évaporée. Le résidu obtenu est purifié par chromatographie sur colonne de silice élué par un mélange d'heptane et d'acétate d'éthyle (8-2). On recueille après évaporation des solvants, 300 mg (30%) de N-[4'-(2,4-Dioxo-thiazolidin-5-ylmethyl)-biphenyl-3-ylmethyl]-N-methyl-carbamate d'hexyle sous forme d'une huile incolore.

### EXEMPLE 62

### 1-[4'-(2,4-Dioxo-thiazolidin-5-ylmethyl)-biphenyl-3-yl]-3-phenyl-uree

### (a) 1-[4'-(2,4-Dioxo-thiazolidin-5-ylidenemethyl)-biphenyl-3-yl]-3-phenyl-uree

A une solution de 1,2 g (2,3 mmol) de 5-(3'-Amino-biphenyl-4-ylmethylene)-thiazolidine-2,4-dione préparée comme en 33(a) et de 0,9 ml (6,4 mmol) de triethylamine dans 12 ml de dichlorométhane, on additionne goutte à goutte 500 µl (4,6 mmol) de phénylisocyanate. Le milieu réactionnel est agité à température ambiante pendant 2 heures. On extrait au dichlorométhane, lave à l'eau, décante la phase organique. La phase organique est évaporée, le résidu obtenu est lavé au dichlorométhane et séché. On recueille 950 mg (50%) du produit attendu.

### (b) 1-[4'-(2,4-Dioxo-thiazolidin-5-ylmethyl)-biphenyl-3-yl]-3-phenyl-uree

Dans un réacteur, on place 460 mg (1,1 mmol) de 1-[4'-(2,4-Dioxo-thiazolidin-5-ylidenemethyl)-biphenyl-3-yl]-3-phenyl-uree dans 20 ml d'un mélange méthanol- dioxanne (1-1). Le milieu réactionnel est dégazé puis on ajoute 560 mg (1,2 équivalent massique) de palladium sur charbon 10%. Le système est placé sous 3 bars d'hydrogène et chauffé à 50°C pendant 5 heures. On filtre le milieu réactionnel sur célite, évapore et purifie le résidu obtenu par chromatographie sur colonne de silice avec une augmentation graduelle de la polarité d'un mélange d'heptane et d'acétate d'éthyle 9-1 vers un mélange d'heptane et d'acétate d'éthyle 4-6. On recueille après évaporation des solvants 150 mg (33%) de 1-[4'-(2,4-Dioxo-thiazolidin-5-ylmethyl)-biphenyl-3-yl]-3-phenyl-uree sous la forme d'un solide blanc de point de fusion 185°C.

### EXEMPLE 63

### N-[4'-(2,4-Dioxo-thiazolidin-5-ylmethyl)-biphenyl-3-yl]-N-methyl-2-phenylacetamide

### (a) (3-Bromo-phenyl)-methyl-amine

Dans un tricol et sous azote, on introduit 10 g (58 mmol) de 3-bromoaniline et 34 ml (203 mmol) de triéthylorthoformate. Le milieu réactionnel est agité et chauffé à reflux pendant 7 heures. L'excès de triéthylorthoformate est évaporé, le milieu réactionnel est refroidi à 0°C, on ajoute 100 ml d'éthanol et 5 g (130 mmol) de borohydrure de sodium. Le milieu réactionnel est agité à température ambiante pendant 20 heures. On évapore l'éthanol, ajoute de l'eau et on extrait à l'acétate d'éthyle. La phase organique est lavée avec une solution saturée de chlorure de sodium, séchée sur sulfate de magnésium, filtrée, évaporée. Le résidu obtenu est purifié par chromatographie sur colonne de silice élué avec un mélange d'heptane et d'acétate d'éthyle (90-10). Après évaporation des solvants, on recueille 5 g (46%) du produit attendu.

### (b) 3'-Methylamino-biphenyl-4-carboxaldehyde

De manière analogue à l'exemple 1(e) par réaction de 4,3 g (23,2 mmol) de (3-Bromo-phenyl)-methyl-amine avec 5,2 g (34,8 mmol) d'acide 4-formylbenzèneboronique, on obtient 2,9 g (60%) de produit attendu.

### (c) 5-(3'-Methylamino-biphenyl-4-ylmethylene)-thiazolidine-2,4-dione

De manière analogue à l'exemple 1(f), à partir de 2,9 g (13,7 mmol) de 3'-Methylamino-biphenyl-4-carboxaldehyde et de 1,6 g (13,7 mmol) de 2,4 thiazolidinedione, on obtient 3,9 g (91%) du produit obtenu.

### (d) N-[4'-(2,4-Dioxo-thiazolidin-5-ylidenemethyl)-biphenyl-3-yl]-N-methyl-2-phenyl-acetamide

De manière analogue à l'exemple 37(e), par réaction de 1 g (3,2 mmol) de 5-(3'-Methylamino-biphenyl-4-ylmethylene)-thiazolidine-2,4-dione avec 470 µl (3,5 mmol) de chlorure de l'acide phenylacetique, on obtient après purification 1,4 g (50%) de *N*-[4'-(2,4-Dioxo-thiazolidin-5-ylidenemethyl)-biphenyl-3-yl]-*N*-methyl-2- phenyl-acetamide.

### (e) N-[4'-(2,4-Dioxo-thiazolidin-5-ylmethyl)-biphenyl-3-yl]-N-methyl-2-phenyl-acetamide

De manière analogue à l'exemple 62(b), à partir de 660 mg (1,5 mmol) de *N*-[4'-(2,4-Dioxo-thiazolidin-5-ylidenemethyl)-biphenyl-3-yl]-*N*-methyl-2-phenyl-acetamide, on obtient 360 mg (54%) de *N*-[4'-(2,4-Dioxo-thiazolidin-5-ylmethyl)-biphenyl-3-yl]-*N*-methyl-2-phenyl-acetamide sous la forme d'un solide blanc de point de fusion 138°C.

### EXEMPLE 64

### 1-[4'-(2,4-Dioxo-thiazolidin-5-ylmethyl)-biphenyl-3-yl]-1-methyl-3-phenyl-uree

### (a) 1-[4'-(2,4-Dioxo-thiazolidin-5-ylidenemethyl)-biphenyl-3-yl]-1-methyl-3-phenyl-uree

De manière analogue à l'exemple 62(a) , à partir de 1 g (3,2 mmol) de 5-(3'-Methylamino-biphenyl-4-ylmethylene)-thiazolidine-2,4-dione et de 700 µl (6,4 mmol) de phénylisocyanate, on obtient 1,2 g (86%) du produit attendu.

### (b) 1-[4'-(2,4-Dioxo-thiazolidin-5-ylmethyl)-biphenyl-3-yl]-1-methyl-3-phenyl-uree

De manière analogue à l'exemple 62(b), à partir de 1,2 g (2,8 mmol) de 1-[4'-(2,4-Dioxo-thiazolidin-5-ylidenemethyl)-biphenyl-3-yl]-1-methyl-3-phenyl-uree, on obtient 700 mg (56%) de 1-[4'-(2,4-Dioxo-thiazolidin-5-ylmethyl)-biphenyl-3-yl]-1-methyl-3-phenyl-uree sous la forme d'un solide blanc de point de fusion 183°C.

### EXEMPLE 65

### 1-[4'-(2,4-Dioxo-thiazolidin-5-ylmethyl)-biphenyl-3-yl]-3-heptyl-1-methyl-uree

### (a) 1-[4'-(2,4-Dioxo-thiazolidin-5-ylidenemethyl)-biphenyl-3-yl]-3-heptyl-1-methyl-uree

De manière analogue à l'exemple 62(a) , à partir de 500 mg (1,6 mmol) de 5-(3'-Methylamino-biphenyl-4-ylmethylene)-thiazolidine-2,4-dione et de 500 µl (3,2 mmol) d'heptylisocyanate, on obtient 500 mg (71%) du produit attendu.

### (b) 1-[4'-(2,4-Dioxo-thiazolidin-5-ylmethyl)-biphenyl-3-yl]-3-heptyl-1-methyl-uree

De manière analogue à l'exemple 62(b), à partir de 500 mg (1,1 mmol) de 1-[4'-(2,4-Dioxo-thiazolidin-5-ylidenemethyl)-biphenyl-3-yl]-3-heptyl-1-methyl-uree, on obtient 300 mg (56%) de 1-[4'-(2,4-Dioxo-thiazolidin-5-ylmethyl)-biphenyl-3-yl]-3-heptyl-1-methyl-uree sous forme d'un solide blanc de point de fusion 55°C.

### EXEMPLE 66

### Ester 4-monométhylique de l'acide 2-{3'-[(Heptanoyl-methyl-amino)-methyl]-biphenyl-4-ylmethyl}-succinique

### (a) Ester 1-monométhylique de l'acide 2-{3'-[(tert-Butoxycarbonyl-methyl-amino)-methyl]-biphenyl-4-ylmethylene}-succinique.

Dans un ballon et sous courant d'azote, on ajoute 3,5 ml (18,5 mmol) d'une solution de méthylate de sodium à 30% dans le méthanol à 2,6 ml (15,4 mmol) de succinate d'éthyle dans 30 ml de méthanol. Le milieu réactionnel est agité pendant 15 minutes puis on additionne goutte à goutte 5 g (15,4 mmol) de (4'-Formyl-biphenyl-3-ylmethyl)-methyl-carbamate de tert-butyle obtenu en 37(a). Le milieu réactionnel est chauffé à 60°C pendant une nuit. On extrait à l'acétate d'éthyle, lave à l'eau, décante la phase organique. La phase organique est séchée sur sulfate de magnésium, filtrée, évaporée. Le résidu obtenu est purifié par chromatographie sur colonne de silice élué avec un mélange d'heptane et d'acétate d'éthyle 50-50. On recueille après évaporation des solvants, 2,8 g (41%) du produit attendu.

### (b) 2-{3'-[(tert-Butoxycarbonyl-methyl-amino)-methyl]-biphenyl-4-ylmethylene}-succinate de méthyle et d'éthyle

A 2,75 g (6,0 mmol) d' ester 1-monométhylique de l'acide 2-{3'-[(*tert*-Butoxycarbonyl-methyl-amino)-methyl]-biphenyl-4-ylmethylene}-succinique dans 30 ml de 2-butanone, on ajoute 1,10 ml (13,6 mmol) d'iodure d'éthyle et 1,88 g (13,6 mmol) de carbonate de potassium. Le milieu réactionnel est chauffé à reflux pendant 1 heure puis filtré. Le résidu obtenu est purifié par chromatographie sur colonne de silice élué avec un mélange d'heptane et d'acétate d'éthyle 75-25. On obtient 2,1 g (75%) du produit attendu.

### (c) 2-(3'-Methylaminomethyl-biphenyl-4-ylmethylene)-succinate de méthyle et d'ethyle.

A 2,1 g (4,5 mmol) de 2-{3'-[(*tert*-Butoxycarbonyl-methyl-amino)-methyl]-biphenyl-4-ylmethylene}-succinate de methyle et d'ethyle dans 25 ml de dichlorométhane, on ajoute 2,8 ml (36,8 mmol) d'acide trifluoroacétique. Le milieu réactionnel est agité à température ambiante pendant 18 heures, lavé avec une solution saturée de carbonate de sodium, extrait au dichlorométhane. La phase organique est séchée sur sulfate de magnésium, filtrée, évaporée. On obtient 1,5 g (95%) du produit attendu.

### (d) 2-{3'-[(Heptanoyl-methyl-amino)-methyl]-biphenyl-4-ylmethylene}-succinate de methyle et d'ethyle.

De manière analogue à l'exemple 37(e), par réaction de 1,5 g (4,2 mmol) de 2-(3'-Methylaminomethyl-biphenyl-4-ylmethylene)-succinate de méthyle et d'ethyle avec 740 µl (4,8 mmol) de chlorure d'heptanoyle, on obtient après purification 1,8 g (89%) du produit attendu.

### (e) 2-{3'-[(Heptanoyl-methyl-amino)-methyl]-biphenyl-4-ylmethyl}-succinate de methyle et d'ethyle.

De manière analogue à l'exemple 1(g) à partir de 1,70 g (3,5 mmol) de 2-{'3'-[(Heptanoyl-methyl-amino)-methyl]-biphenyl-4-ylmethylene}-succinate de methyle et d'ethyle et de 170 mg (10% massique) de palladium sur charbon 10% dans 20 ml d'acétate d'éthyle , on obtient 1,30 g (77%) du produit attendu.

### (f) Ester 4-monométhylique de l'acide 2-{3'-[(Heptanoyl-methyl-amino)-methyl]-biphenyl-4-ylmethyl}-succinique.

A 0,3 g (0,6 mmol) de 2-{3'-[(Heptanoyl-methyl-amino)-methyl]-biphenyl-4-ylmethyl}-succinate de méthyle et d'ethyle dans 3 ml de méthanol et 1,5 ml de tétrahydrofuranne, on ajoute à 0°C, 300 µl (0,7 mmol) d'une solution aqueuse d'hydroxyde de sodium 2M. Le milieu réactionnel est agité à température ambiante pendant 18 heures, acidifié à pH 5, extrait à l'acétate d'éthyle. La phase organique est séchée sur sulfate de magnésium, filtrée, évaporée. Le résidu obtenu est purifié par chromatographie sur colonne de silice élué avec un mélange dichlorométhane - méthanol 99-1. Après évaporation des solvants, on recueille 130 mg (46%) de l'ester 4-monométhylique de l'acide 2-{3'-[(Heptanoyl-methyl-amino)-methyl]-biphenyl-4-ylmethyl}-succinique sous la forme d'un film incolore.

### EXEMPLE 67

### Acide 2-{3'-[(Methyl-octanoyl-amino)-methyl]-biphenyl-4-ylmethyl}-succinique

A 900 mg (1,9 mmol) de 2-{3'-[(Heptanoyl-methyl-amino)-methyl]-biphenyl-4-ylmethyl}-succinate d'éthyle et de méthyle obtenu en 66(e) dans 9 ml de méthanol, 4,5 ml de tétrahydrofuranne, on ajoute 2,4 ml (5,6 mmol) d'une solution aqueuse d'hydroxyde de sodium 2N. Le milieu réactionnel est agité à température ambiante pendant 36 heures puis à 50°C pendant 3 heures, acidifié à pH 5 et extrait à l'acétate d'éthyle. La phase organique est séchée sur sulfate de magnésium, filtrée, évaporée. Le résidu obtenu est purifié par chromatographie sur colonne de silice élué avec un mélange dichlorométhane - méthanol 99-1. Après évaporation des solvants, on recueille 300 mg (35%) d' acide 2-{3'-[(Methyl-octanoyl-amino)-methyl]-biphenyl-4-ylmethyl}-succinique sous la forme d'un solide blanc de point de fusion 57°C.

### EXEMPLE 68

### N- [4'-(2,5-dioxo-pyrrolidin-3-ylmethyl)-biphenyl-3-ylmethyl]-N-methyl-heptanamide.

***A 420 mg (0,97 mmol) d'acide 2-{3'-[(Methyl-octanoyl-amino)-methyl]-biphenyl-4-ylmethyl}-succinique obtenu à l'exemple 67, on ajoute 180 mg (3 mmol) d'urée. Le milieu réactionnel est chauffé à 180°C pendant 4 heures puis refroidi. Le résidu obtenu est purifié par chromatographie sur colonne de silice élué avec un mélange dichlorométhane-méthanol 99-1. Après évaporation des solvants, on recueille 220 mg (55%) de N- [4'-(2,5-dioxo-pyrrolidin-3-ylmethyl)-biphenyl-3-ylmethyl]-N-methyl-heptanamide sous la forme d'un film incolore*.**

### EXEMPLE 69

### N-Phenyl-carbamate de 3-[4'-(2,4-dioxo-thiazolidin-5-ylidenemethyl)]biphenyle

### (a) 3'-Hydroxy-biphenyl-4-carbaldehyde

De manière analogue à l'exemple 1(e), à partir de 20 g (115 mmol) de 3-bromophénol et de 26 g (173 mmol) d'acide 4-formylbenzèneboronique, on obtient 15 g (65%) du produit attendu.

### (b) 5-(3'-Hydroxy-biphenyl-4-ylmethylene)-thiazolidine-2,4-dione

De manière analogue à l'exemple 1(f), à partir de 15 g (75,7 mmol) de 3'-Hydroxy-biphenyl-4-carbaldehyde et de 8,85 g (75,7 mmol) de 2,4-thiazolidinedione, on obtient 22,5 g (100%) du produit attendu.

### (c) N-Phenyl-carbamate de 3-[4'-(2,4-dioxo-thiazolidin-5-ylidenemethyl)]biphenyle

De manière analogue à l'exemple 9(a), à partir de 1,5 g (5 mmol) de 5-(3'-Hydroxy-biphenyl-4-ylmethylene)-thiazolidine-2,4-dione et de 1,1 ml (10,1 mmol) de phenylisocyanate dans 800 µl de pyridine et 10 ml de THF, on obtient 750 mg (36%) de N-Phenyl-carbamate de 3-[4'-(2,4-dioxo-thiazolidin-5-ylidenemethyl)]biphenyle.

### EXEMPLE 70

### N-Heptyl-carbamate de 3-[4'-(2,4-dioxo-thiazolidin-5-ylidenemethyl)]biphenyle

A 2 g (6,7 mmol) de 5-(3'-Hydroxy-biphenyl-4-ylmethylene)-thiazolidine-2,4-dione ( préparé en 69(b) ) placés dans 30 ml d'acétonitrile et 5 ml de triéthylamine, on additionne 1,3 ml (8,1 mmol) de heptylisocyanate et on chauffe à 40°C pendant 5 heures. On additionne ensuite 480 mg (3,9 mmol) de 4-(diméthylamino)pyridine et on chauffe à reflux pendant 24 heures. Le milieu réactionnel est versé dans de l'eau et extrait à l'éther. La phase organique est évaporée. Le résidu obtenu est lavé avec un mélange acétate d'éthyle-méthanol 80-20 et séché.On recueille 1,3 g (44%) de N-Heptyl-carbamate de 3-[4'-(2,4-dioxo-thiazolidin-5-ylidenemethyl)]biphenyle sous la forme d'un solide jaune de point de fusion 316°C.

### EXEMPLE 71

### Phenyl-acetate de 3-[4'-(2,4-dioxo-thiazolidin-5-ylmethyl)]biphenyle.

### (a) Phenyl-acetate de 3-[4'-(2,4-dioxo-thiazolidin-5-ylidenemethyl)]biphenyle

De manière analogue à l'exemple 37(e), par réaction de 2 g (6,7 mmol) de 5-(3'-Hydroxy-biphenyl-4-ylmethylene)-thiazolidine-2,4-dione ( préparé en 69(b) ) avec 1 ml ( 7,4 mmol) de chlorure de phénylacétyle, on obtient 2,1 g ( 75%) du produit attendu sous la forme d'un solide jaune.

### (b) Phenyl-acetate de 3-[4'-(2,4-dioxo-thiazolidin-5-ylmethyl)]biphenyle

De manière analogue à l'exemple 1(g), à partir de 2,1 g (5 mmol) de Phenyl-acetate de 3-[4'-(2,4-dioxo-thiazolidin-5-ylidenemethyl)]biphenyle dans 40 ml d'un mélange dioxanne-méthanol (50-50), on obtient après purification par chromatographie sur colonne de silice élué avec un mélange heptane - acétate d'éthyle (8/2) 810 mg (38%) de Phenyl-acetate de 3-[4'-(2,4-dioxo-thiazolidin-5-ylmethyl)]biphenyle sous la forme d'un solide jaune de point de fusion 147°C.

### EXEMPLE 72

### Nonanoate de 3-[4'-(2,4-dioxo-thiazolidin-5-ylmethyl)]biphenyle

### (a) 5-(3'-Hydroxy-biphenyl-4-ylmethyl)-thiazolidine-2,4-dione

De manière analogue à l'exemple 1(g), à partir de 3 g (10,1 mmol) de 5-(3'-Hydroxy-biphenyl-4-ylmethylene)-thiazolidine-2,4-dione dans un mélange dioxanne-méthanol (50-50), on obtient après purification par chromatographie sur colonne de silice élué avec un mélange heptane - acétate d'éthyle (7/3) 800 mg (27%) du produit attendu.

### (b) Nonanoate de 3-[4'-(2,4-dioxo-thiazolidin-5-ylmethyl)]biphenyle

De manière analogue à l'exemple 37(e), par réaction de 600 mg (2 mmol) de 5-(3'-Hydroxy-biphenyl-4-ylmethyl)-thiazolidine-2,4-dione avec 400 µl ( 2,2 mmol) de chlorure de nonanoyle et 50 mg (0,4 mmol) de 4-(diméthylamino)pyridine, on obtient 780 mg (89%) de Nonanoate de 3-[4'-(2,4-dioxo-thiazolidin-5-ylmethyl)]biphenyle sous la forme d'un solide blanc de point de fusion 70°C.

### EXEMPLE 73

### N-Heptyl-carbamate de 3-[4'-(2,4-dioxo-thiazolidin-5-ylmethyl)]biphenyle

De manière analogue à l'exemple 1 (g), à partir de 950 mg (2,2 mmol) de N-Heptyl-carbamate de 3-[4'-(2,4-dioxo-thiazolidin-5-ylidenemethyl)]biphenyle ( obtenu en 70 ) dans un mélange dioxanne-méthanol (50-50) sous 3 atm d'hydrogène, on obtient 130 mg (13%) de N-Heptyl-carbamate de 3-[4'-(2,4-dioxo-thiazolidin-5-ylmethyl)]biphenyle sous la forme d'un solide blanc de point de fusion de 315°C.

### EXEMPLE 74

### N-Phenyl-carbamate de 3-[4'-(2,4-dioxo-thiazolidin-5-ylmethyl)]biphenyle

De manière analogue à l'exemple 1 (g), à partir de 740 mg (1,78 mmol) de N-Phenyl-carbamate de 3-[4'-(2,4-dioxo-thiazolidin-5-ylidenemethyl)]-biphenyle ( obtenu en 69 ), dans 25 ml de dioxanne sous 3 atm d'hydrogène, on obtient 310 mg (42%) de N-Phenyl-carbamate de 3-[4'-(2,4-dioxo-thiazolidin-5-ylmethyl)]biphenyle sous la forme d'un solide blanc de point de fusion 142°C.

### EXEMPLE 75

### N-[6-benzyloxy-4'-(2,4-dioxo-thiazolidin-5-ylidenemethyl)-biphenyl-3-ylmethyl]-N-methyl-octanamide

### (a) 4-Benzyloxy-3-bromo-benzaldehyde

Dans un ballon et sous courant d'azote, on introduit 25 g (124 mmol) de 3-bromo-4-hydroxybenzaldéhyde, 250 ml de 2-butanone, 14,8 ml (124 mmol) de bromure de benzyle et 42,8 g (310 mmol) de carbonate de potassium. Le milieu réactionnel est chauffé au reflux pendant 4 heures, filtré et évaporé. Le résidu est repris dans de l'éther diisopropylique puis filtré et séché. On obtient 28 g (77%) du produit attendu sous la forme de poudre beige de point de fusion 93°C.

### (b) N-(4-Benzyloxy-3-bromo-benzyl)-N-methyl-amine

Dans un ballon et sous courant d'azote, on introduit 25 g (86 mmol) de 4-Benzyloxy-3-bromo-benzaldehyde, 500 ml de méthanol, 29 g (430 mmol) de chlorhydrate de méthylamine et 8 g (127 mmol) de cyanoborohydrure de sodium. Le milieu réactionnel est agité 48 heures à température ambiante. Le méthanol est évaporé. Le résidu est repris par de l'acétate d'éthyle et de l'eau puis acidifié. Après décantation, la phase aqueuse est ramenée à pH basique avec de la soude et extraite avec de l'acétate d'éthyle. La phase organique est séchée sur sulfate de magnésium, filtrée puis évaporée. Le résidu est purifié par chromatographie sur colonne de silice élué avec un mélange dichlorométhane - éthanol (9/1). On obtient 9,9 g (38%) du produit attendu sous la forme d'une huile incolore.

### (c) N-(4-benzyloxy-3-bromo-benzyl)-N-methyl-octanamide

De manière analogue à l'exemple 37 (e), par réaction de 9,9 g (32 mmol) de N-(4-Benzyloxy-3-bromo-benzyl)-N-methyl-amine avec 5,3 g (32 mmol) de chlorure d'octanoyle dans 90 ml de dichlorométhane, on obtient 13,6 g (97%) du produit attendu sous la forme d'une huile incolore.

### (d) N-(6-benzyloxy-4'-formyl-biphenyl-3-ylmethyl)-N-methyl-octanamide

De manière analogue à l'exemple 1 (e), par réaction de 13,6 g (31 mmol) de N- (4-benzyloxy-3-bromo-benzyl)-N-methyl-octanamide et de 6,1 g (40 mmol) d'acide 4-formylbenzèneboronique, on obtient 11,5 g (69%) du produit attendu sous la forme d'une huile jaune.

### (e) N-[6-benzyloxy-4'-(2,4-dioxo-thiazolidin-5-ylidenemethyl)-biphenyl-3-ylmethyl]-N-methyl-octanamide

De manière analogue à l'exemple 1(f), par réaction de 11,5 g (22 mmol) de N- (6-benzyloxy-4'-formyl-biphenyl-3-ylmethyl)-N-methyl-octanamide et de 2,5 g (22 mmol) de 2,4-thiazolidinedione, on obtient le produit attendu sous la forme d'une poudre jaune pâle de point de fusion 162°C.

### EXEMPLE 76

### N-[4'-(2,4-dioxo-thiazolidin-5-ylmethyl)-6-hydroxy-biphenyl-3-ylmethyl]-N-methyl-octanamide

### (a) N-[6-benzyloxy-4'-(2,4-dioxo-thiazolidin-5-ylmethyl)-biphenyl-3-ylmethyl]-N-methyl-octanamide

De manière analogue à l'exemple 1 (g), à partir de 1 g (1,8 mmol) de N-[6-benzyloxy-4'-(2,4-dioxo-thiazolidin-5-ylidenemethyl)-biphenyl-3-ylmethyl]-N-methyl-octanamide ( obtenu en 75 ) dans 30 ml de dioxanne et 1 ml de triéthylamine sous 3 atm d'hydrogène, on obtient 270 mg (27%) du produit attendu sous la forme d'une huile jaune.

### (b) N-[4'-(2,4-dioxo-thiazolidin-5-ylmethyl)-6-hydroxy-biphenyl-3-ylmethyl]-N-methyl-octanamide

Dans un ballon et sous courant d'azote, on introduit 250 mg (0,45 mmol) de N- [6-benzyloxy-4'-(2,4-dioxo-thiazolidin-5-ylmethyl)-biphenyl-3-ylmethyl]-N-methyl-octanamide, 4 ml d'acétonitrile et 130 mg (0,65 mmol) de iodotriméthylsilane. Le milieu réactionnel est chauffé à 50°C pendant 3 heures. A température ambiante, on rajoute 450 µl d'acide chlorhydrique 1 N et 450 µl de fluorure de tétrabutylammonium. Le milieu est ensuite dilué avec de l'acétate d'éthyle et lavé avec une solution de thiosulfate de sodium. Après décantation, la phase organique est séchée sur sulfate de magnésium, filtrée et évaporée. Le résidu obtenu est purifié par chromatographie sur colonne de silice élué avec un mélange acétate d'éthyle - heptane (6/4). On obtient 150 mg (72%) de N-[4'-(2,4-dioxo-thiazolidin-5-ylmethyl)-6-hydroxy-biphenyl-3-ylmethyl]-N-methyl-octanamide sous la forme d'une mousse jaunâtre.

### EXEMPLE 77

### N-[4-benzyloxy-4'-(2,4-dioxo-thiazolidin-5-ylidenemethyl)-biphenyl-3-ylmethyl]-N-methyl-octanamide

### (a) 2-Benzyloxy-5-bromo-benzaldehyde

De manière analogue à l'exemple 75(a), par réaction de 30 g (150 mmol) de 5-bromo-2-hydroxybenzaldéhyde et de 17,8 ml (150 mmol) de bromure de benzyle dans 300 ml d'acétone, on obtient après purification par chromatographie sur colonne de silice élué avec un mélange heptane - acétate d'éthyle (8/2), 22,8 g (52%) du produit attendu sous la forme d'un solide jaune.

### (b) N-(2-Benzyloxy-5-bromo-benzyl)-N-methyl-amine

De manière analogue à l'exemple 75(b), par réaction de 22,5 g (77 mmol) de 2-Benzyloxy-5-bromo-benzaldehyde, de 26,1 g (386 mmol) de chlorhydrate de méthylamine et de 7,3 g (116 mmol) de cyanoborohydrure de sodium, on obtient après purification par chromatographie sur colonne de silice élué avec un mélange dichlorométhane - méthanol (9/1) et 0,5 % d'isopropylamine, 12,2 g (51%) du produit attendu sous la forme d'une huile jaune pâle.

### (c) N-(2-benzyloxy-5-bromo-benzyl)-N-methyl-octanamide

De manière analogue à l'exemple 37 (e), par réaction de 6 g (19,6 mmol) de N-(2-Benzyloxy-5-bromo-benzyl)-N-methyl-amine avec 3,4 ml (19,6 mmol) de chlorure d'octanoyle, on obtient après purification par chromatographie sur colonne de silice élué avec un mélange heptane - acétate d'éthyle (8/2), 8 g (95%) du produit attendu sous la forme d'une huile incolore.

### (d) N-(4-benzyloxy-4'-formyl-biphenyl-3-ylmethyl)-N-methyl-octanamide

De manière analogue à l'exemple 1(e), par réaction de 8 g (18,5 mmol)de N- (2-benzyloxy-5-bromo-benzyl)-N-methyl-octanamide et de 3,6 g (24 mmol) d'acide 4-formylbenzèneboronique, on obtient après purification par chromatographie sur colonne de silice élué avec un mélange heptane - acétate d'éthyle (8/2), 5,8 g (69%) du produit attendu sous la forme d'un solide blanc.

### (e) N-[4-benzyloxy-4'-(2,4-dioxo-thiazolidin-5-ylidenemethyl)-biphenyl-3-ylmethyl]-N-methyl-octanamide

De manière analogue à l'exemple 1(f), par réaction de 5,8 g (13 mmol) de N- (4-benzyloxy-4'-formyl-biphenyl-3-ylmethyl)-N-methyl-octanamide et de 1,5 g (13 mmol) de 2,4-thiazolidinedione, on obtient 5,2 g (74%) de N-[4-benzyloxy-4'-(2,4-dioxo-thiazolidin-5-ylidenemethyl)-biphenyl-3-ylmethyl]-N-methyl-octanamide sous la forme d'une poudre jaune vif de point de fusion 158°C.

### EXEMPLE 78

### N-[4"-(2,4-dioxo-thiazolidin-5-ylmethyl)-[1,1';3',1"]terphenyl-5'-ylmethyl]-N-methyl-octanamide

### (a) 3-Bromo-5-iodo-N-methyl-benzamide

Dans un ballon et sous courant d'azote, on introduit 37,5 g (115 mmol) d'acide 3-bromo-5-iodobenzoïque dans 300 ml de diméthylformamide. On rajoute 17,6 ml (127 mmol) de triéthylamine, 17g (126 mmol) de 1-hydroxybenzotriazole et 7,75 g (115 mmol) de chlorhydrate de méthylamine. Le milieu réactionnel est refroidi à 0°C puis on ajoute, par portions, 24,2 g (126 mmol) de chlorhydrate de 1-[3-(dimethylamino) propyl]-3-ethylcarbodiimide. On laisse remonter à température ambiante et le milieu est agité pendant 18 heures. Il est, ensuite, versé dans de l'eau et le précipité obtenu est filtré, lavé à l'heptane puis séché. On obtient 37,3 g (96%) du produit attendu sous la forme d'une poudre beige de point de fusion 205°C.

### (b) 5-Bromo-N-methyl-3-biphenyl-carboxamide

De manière analogue à l'exemple 1(e), par réaction de 4,9 g (14 mmol) de 3-Bromo-5-iodo-N-methyl-benzamide et de 1,93 g (15,8 mmol) d'acide phénylboronique, on obtient après purification par chromatographie sur colonne de silice élué avec un mélange heptane - acétate d'éthyle (8/2), 2,1 g (50%) du produit attendu sous la forme d'un solide blanc de point de fusion 132°C.

### (c) 4"-Formyl-N-methyl-[1,1';3',1"]terphenyl-5'-carboxamide

De manière analogue à l'exemple 1(e), par réaction de 2 g (7 mmol) de 5-Bromo-N-methyl-3-biphenyl-carboxamide et de 1,4 g (9,3 mmol) d'acide 4-formylbenzèneboronique, on obtient après purification par recristallisation dans l'acétonitrile, 1,4 g (63%) du produit attendu sous la forme d'un solide beige.

### (d) (5'-Methylaminomethyl-[1,1';3',1"]terphenyl-4"-yl)-methanol

Dans un ballon, sous un courant d'azote, on introduit 1 g (26 mmol) d'aluminohydrure de lithium dans 20 ml de tétrahydrofurane. Le milieu est refroidi à 0°C et on ajoute goutte à goutte, une solution de 1,4 g (4,4 mmol) de 4"-Formyl-N-methyl-[1,1';3',1"]terphenyl-5'-carboxamide dans 30 ml de tétrahydrofurane. Le milieu réactionnel est chauffé au reflux pendant 48 heures. On laisse, alors, revenir à température ambiante et une solution aqueuse de sulfate de sodium est additionnée goutte à goutte. Après 30 minutes, le milieu est acidifié à pH 5 et extrait à l'acétate d'éthyle. La phase organique est séchée sur sulfate de magnésium, filtrée puis évaporée. Le résidu est purifié par chromatographie sur colonne de silice élué avec un mélange dichlorométhane - éthanol (9/1) et on obtient 660 mg (49%) du produit attendu sous la forme d'un solide blanc.

### (e) 5'-Methylaminomethyl-[1,1';3',1"]terphenyl-4"-carbaldehyde

Dans un ballon, on introduit 660 mg ( 2,2 mmol) de (5'-Methylaminomethyl-[1,1';3',1"]terphenyl-4"-yl)-methanol et 1,9 g (22 mmol) de dioxyde de manganèse dans 30 ml de dichlorométhane. Le milieu réactionnel est agité 24 heures à température ambiante. Il est, ensuite, filtré sur célite et évaporé. On obtient 410 mg (62%) du produit attendu sous la forme d'un solide jaune pâle.

### (f) 5-(5'-Methylaminomethyl-[1,1';3',1"]terphenyl-4"-ylmethylene)-thiazolidine-2,4-dione

De manière analogue à l'exemple 1(f), par réaction de 400 mg (1,3 mmol) de 5'-Methylaminomethyl-[1,1';3',1"]terphenyl-4"-carbaldehyde et de 160 mg (1,3 mmol) de 2,4-thiazolidinedione, on obtient 530 mg (100%) du produit attendu sous la forme d'une poudre orange

### (g) N-[4"-(2,4-dioxo-thiazolidin-5-ylidenemethyl)-[1,1';3',1"]terphenyl-5'-ylmethyl]-N-methyl-octanamide

De manière analogue à l'exemple 37 (e), par réaction de 530 mg (1,3 mmol) de 5-(5'-Methylaminomethyl-[1,1';3',1"]terphenyl-4"-ylmethylene)-thiazolidine-2,4-dione avec 230 µl (1,3 mmol) de chlorure d'octanoyle, on obtient après purification par chromatographie sur colonne de silice élué avec un mélange dichlorométhane - méthanol (95/5), 390 mg (56%) du produit attendu sous la forme d'une huile orange.

### (h) N-[4"-(2,4-dioxo-thiazolidin-5-ylmethyl)-[1,1';3',1"]terphenyl-5'-ylmethyl]-N-methyl-octanamide

De manière analogue à l'exemple 1 (g), à partir de 370 mg (7 mmol) de N-[4"-(2,4-dioxo-thiazolidin-5-ylidenemethyl)-[1,1';3',1"]terphenyl-5'-ylmethyl]-N-methyl-octanamide dans 5 ml de dioxanne et sous 3 atm d'hydrogène, on obtient après purification par chromatographie sur colonne de silice élué avec un mélange heptane - acétate d'éthyle (6/4), 170 mg (47%) de N-[4"-(2,4-dioxo-thiazolidin-5-ylmethyl)-[1,1';3',1"]terphenyl-5'-ylmethyl]-N-methyl-octanamide sous la forme d'une meringue jaune pâle.

### EXEMPLE 79

### N-[4'-(2,4-dioxo-thiazolidin-5-ylmethyl)-2'-methyl-biphenyl-3-ylmethyl]-N-methyl-octanamide.

### (a) (3-Bromo-benzyl)-carbamate de tert-butyle

De manière analogue à l'exemple 1 (a), à partir de 50 g (220 mmol) de chlorhydrate de 3-bromobenzylamine, on obtient 65,1 g (100%) du produit attendu sous la forme d'un solide brun clair.

### (b) (3-Bromo-benzyl)-N-méthyl-carbamate de tert-butyle

De manière analogue à l'exemple 1(b), à partir de 85 g (297 mmol) de (3-Bromo-benzyl)-carbamate de tert-butyle, on obtient 92,5 g (100%) du produit attendu

### (c) (4'-Formyl-2'-methyl-biphenyl-3-ylmethyl)-N-méthyl-carbamate de tert-butyle

De manière analogue à l'exemple 1(e), par réaction de 6,1 g (20,3 mmol) de (3-Bromo-benzyl)-N-méthyl-carbamate de tert-butyle avec 4,3 g (26,2 mmol) d'acide 2-méthyl-4-formylbenzèneboronique, on obtient après purification par chromatographie sur colonne de silice élué avec un mélange heptane - acétate d'éthyle (8/2), 4 g (58%) du produit attendu sous la forme d'une huile orange.

### (d) [4'-(2,4-Dioxo-thiazolidin-5-ylidenemethyl)-2'-methyl-biphenyl-3-ylmethyl]-N-méthyl-carbamate de tert-butyle

De manière analogue à l'exemple 1(f), par réaction de 3,95 g (11,6 mmol) de (4'-Formyl-2'-methyl-biphenyl-3-ylmethyl)-N-méthyl-carbamate de tert-butyle avec 1,4 g (11,6 mmol) de 2,4-thiazolidinedione, on obtient après purification par chromatographie sur colonne de silice élué avec un mélange heptane - acétate d'éthyle (8/2), 3 g (59%) du produit attendu sous la forme d'un solide jaune de point de fusion 156°C

### (e) [4'-(2,4-Dioxo-thiazolidin-5-ylmethyl)-2'-methyl-biphenyl-3-ylmethyl]-N-méthyl-carbamate de tert-butyle

De manière analogue à l'exemple 1 (g), à partir de 1,5 g (3,4 mmol) de [4'-(2,4-Dioxo-thiazolidin-5-ylidenemethyl)-2'-methyl-biphenyl-3-ylmethyl]-N-méthyl-carbamate de tert-butyle, on obtient, après purification par chromatographie sur colonne de silice élué avec un mélange heptane - acétate d'éthyle (8/2), 1,2 g (82%) du produit attendu sous la forme d'une huile jaunâtre.

### (f) 5-(2-Methyl-3'-methylaminomethyl-biphenyl-4-ylmethyl)-thiazolidine-2,4-dione

De manière analogue à l'exemple 1 (c), à partir de 1,2 g (2,7 mmol) de [4'-(2,4-Dioxo-thiazolidin-5-ylmethyl)-2'-methyl-biphenyl-3-ylmethyl]-N-méthyl-carbamate de tert-butyle, on obtient 720 mg (78%) du produit attendu sous la forme d'un solide blanc.

### (g) N-[4'-(2,4-dioxo-thiazolidin-5-ylmethyl)-2'-methyl-biphenyl-3-ylmethyl]-N-methyl-octanamide

De manière analogue à l'exemple 37 (e) , par réaction de 300 mg (0,88 mmol) de 5-(2-Methyl-3'-methylaminomethyl-biphenyl-4-ylmethyl)-thiazolidine-2,4-dione avec 140 mg (0,88 mmol) de chlorure d'octanoyle, on obtient après purification par chromatographie sur colonne de silice élué avec un mélange heptane - acétate d'éthyle (6/4), 250 mg (63%) de N-[4'-(2,4-dioxo-thiazolidin-5-ylmethyl)-2'-methyl-biphenyl-3-ylmethyl]-N-methyl-octanamide sous la forme d'un solide blanc de point de fusion 130°C.

### EXEMPLE 80

### N-[4'-(2,4-dioxo-thiazolidin-5-ylmethyl)-3'-methyl-biphenyl-3-ylmethyl]-N-methyl-octanamide

### (a) (4'-Formyl-3'-methyl-biphenyl-3-ylmethyl)-N-méthyl-carbamate de tert-butyle

De manière analogue à l'exemple 1(e), par réaction de 1,1 g (3,5 mmol) de (3-Bromo-benzyl)-N-méthyl-carbamate de tert-butyle obtenu en 79(b), avec 750 mg (4,6 mmol) d'acide 3-méthyl-4-formylbenzèneboronique, on obtient après purification par chromatographie sur colonne de silice élué avec un mélange heptane - acétate d'éthyle (7/3), 1 g (87%) du produit attendu sous la forme d'une huile jaune.

### (b) [4'-(2,4-Dioxo-thiazolidin-5-ylidenemethyl)-3'-methyl-biphenyl-3-ylmethyl]-N-methyl-carbamate de tert-butyle

De manière analogue à l'exemple 1(f), par réaction de 1 g (2,9 mmol) de (4'-Formyl-3'-methyl-biphenyl-3-ylmethyl)-N-methyl-carbamate de tert-butyle avec 350 mg (2,9 mmol) de 2,4-thiazolidinedione, on obtient après purification par chromatographie sur colonne de silice élué avec un mélange heptane - acétate d'éthyle (6/4), 740 mg (57%) du produit attendu sous la forme d'une huile jaune.

### (c) [4'-(2,4-Dioxo-thiazolidin-5-ylmethyl)-3'-methyl-biphenyl-3-ylmethyl]-N-methylcarbamate de tert-butyle

De manière analogue à l'exemple 1 (g), à partir de 740 mg (1,7 mmol) de [4'-(2,4-Dioxo-thiazolidin-5-ylidenemethyl)-3'-methyl-biphenyl-3-ylmethyl]-N-methyl-carbamate de tert-butyle, on obtient 720 mg (97%) du produit attendu sous la forme d'une huile jaune.

### (d) 5-(3-Methyl-3'-methylaminomethyl-biphenyl-4-ylmethyl)-thiazolidine-2,4-dione

De manière analogue à l'exemple 1 (c), à partir de 720 mg (1,6 mmol) de [4'-(2,4-Dioxo-thiazolidin-5-ylmethyl)-3'-methyl-biphenyl-3-ylmethyl]-N-methyl-carbamate de tert-butyle, on obtient 320 mg (58%) du produit attendu sous la forme d'une poudre blanche de point de fusion 135°C.

### (e) N-[4'-(2,4-dioxo-thiazolidin-5-ylmethyl)-3'-methyl-biphenyl-3-ylmethyl]-N-methyl-octanamide

De manière analogue à l'exemple 37 (e) , par réaction de 310 mg (0,9 mmol) de 5-(3-Methyl-3'-methylaminomethyl-biphenyl-4-ylmethyl)-thiazolidine-2,4-dione avec 160 mg (0,9 mmol) de chlorure d'octanoyle, on obtient après purification par chromatographie sur colonne de silice élué avec un mélange heptane - acétate d'éthyle (6/4), 230 mg (55%) de N-[4'-(2,4-dioxo-thiazolidin-5-ylmethyl)-3'-methyl-biphenyl-3-ylmethyl]-N-methyl-octanamide sous la forme d'une huile jaunâtre.

### EXEMPLE 81

### Acide (S)-2-Ethoxy-3-{3'-[(methyl-octanoyl-amino)-methyl]-biphenyl-4-yl}-propionique

### (a) (4'-Formyl-biphenyl-3-ylmethyl)-N-methyl-carbamate de tert-butyle

De manière analogue à l'exemple 1(e), par réaction de 38 g (127 mmol) de (3-Bromo-benzyl)-N-methyl-carbamate de tert-butyle obtenu en 79(b), avec 25,6 g (170 mmol) d'acide 4-formylbenzèneboronique, on obtient après purification par chromatographie sur colonne de silice élué avec un mélange heptane - acétate d'éthyle (9/1), 20 g (57%) du produit attendu.

### (b) Chlorure d'ethoxyacetyle

Dans un ballon et sous courant d'azote, on introduit 25 g (240 mmol) d'acide éthoxyacétique dans 300 ml de dichlorométhane. On ajoute 47,6 ml (239 mmol) de dicyclohexylamine. Le milieu est agité 1 heure à température ambiante. On ajoute, alors, 19,2 ml (265 mmol) de chlorure de thionyle et on agite pendant 3 heures. On rajoute au milieu réactionnel de l'éther éthyulique, le précipité formé est filtré et rincé à l'éther. Après évaporation du filtrat, on obtient 29 g (100%) du produit attendu sous la forme d'un liquide marron.

### (c) 3-(2-Ethoxy-ethanoyl)-4-benzyloxazolidin-2-one

Dans un ballon et sous courant d'azote, on introduit 36,7 g (207 mmol) de (S)-4-benzyloxazolidin-2-one dans 800 ml de THF. Le milieu réactionnel est refroidi à -78°C et on ajoute, goutte à goutte, 83 ml (207 mmol) de n-Butyllithium 2,5 M/hexane. 30 minutes après, on ajoute, à - 78°C, 25,4 g (207 mmol) de chlorure d'ethoxyacetyle obtenu en 81 (b). Le milieu réactionnel est agité 24 heures puis versé dans une solution aqueuse saturée en chlorure de sodium et extrait à l'acétate d'éthyle. La phase organique est séchée sur sulfate de magnésium, filtrée et évaporée. On obtient, après purification par chromatographie sur colonne de silice élué avec un mélange heptane - acétate d'éthyle (6/4), 30,6 g (56%) du produit attendu sous la forme d'une huile orangée.

### (d) (2S,3R)-3-{3'-[(tert-Butoxycarbonyl-methyl-amino)-methyl]-biphenyl-4-yl}-2-ethoxy-3-hydroxy-propionate de methyle.

De manière analogue à l'exemple 13(a), par réaction de 23,5 g (69 mmol) de (4'-Formyl-biphenyl-3-ylmethyl)-N-methyl-carbamate de tert-butyle préparé en 81 (a) avec 21,9 g (83 mmol) de 3-(2-Ethoxy-ethanoyl)-4-benzyloxazolidin-2-one, on obtient, après purification par chromatographie sur colonne de silice élué avec un mélange heptane - acétate d'éthyle (7/3), 21 g (51%) du produit attendu.

### (e) (2S,3R)-2-Ethoxy-3-hydroxy-3-(3'-methylaminomethyl-biphenyl-4-yl)-propionate de methyle.

Dans un ballon et sous courant d'azote, on introduit 21 g (47,3 mmol) de (2S,3R)-3-{3'-[(tert-Butoxycarbonyl-methyl-amino)-methyl]-biphenyl-4-yl}-2-ethoxy-3-hydroxy-propionate de methyle, 8,76 ml (54,9 mmol) de triéthylsilane dans 300 ml d'acide trifluoroacétique. Le milieu réactionnel est agité pendant 4 heures à température ambiante. On ajoute, ensuite, de l'acétate d'éthyle et on neutralise avec de la soude. La phase organique est lavée avec une solution aqueuse saturée en chlorure de sodium, séchée sur sulfate de magnésium, filtrée et évaporée. On obtient 19,6 g (100%) de produit brut déprotégé mais toujours hydroxylé.

### (e) (S)-2-Ethoxy-3-(3'-methylaminomethyl-biphenyl-4-yl)-propionate de methyle

19,6 g du produit brut obtenu en 81 (e) sont dissous dans 200 ml d'acide trifluoroacétique et on ajoute 41,7 mi (297 mmol) de triéthylamine. Le milieu réactionnel est agité à température ambiante pendant 48 h puis extrait à l'acétate d'éthyle . La phase organique est décantée, lavée avec une solution de soude puis avec une solution aqueuse saturée en chlorure de sodium, séchée sur sulfate de magnésium, filtrée et évaporée. Le résidu obtenu est purifié par chromatographie sur colonne de silice élué avec un mélange dichlorométhane - méthanol (95-5). On obtient 1,6 g (10%) du produit attendu.

### (f) (S)-2-Ethoxy-3-{3'-[(methyl-octanoyl-amino)-methyl]-biphenyl-4-yl}-propionate de methyle.

De manière analogue à l'exemple 37(e), par réaction de 500 mg (1,5 mmol) de (S)-2-Ethoxy-3-(3'-methylaminomethyl-biphenyl-4-yl)-propionate de methyle avec 550 µl (3,18 mmol) de chlorure d'octanoyle, on obtient après purification par chromatographie sur colonne de silice élué avec un mélange heptane - acétate d'éthyle (70/30), 200 mg (29%) du produit attendu.

### (h) acide (S)-2-Ethoxy-3-{3'-[(methyl-octanoyl-amino)-methyl]-biphenyl-4-yl}-propionique.

De manière analogue à l'exemple 13 (c), par réaction de 180 mg ( 0,4 mmol) de (S)-2-Ethoxy-3-{3'-[(methyl-octanoyl-amino)-methyl]-biphenyl-4-yl}-propionate de méthyle avec 17 mg (0,4 mmol) d'hydroxyde de lithium, monohydraté, on obtient, après purification par chromatographie sur colonne de silice avec un mélange éluant heptane - acétate d'éthyle (50/50), 130 mg (74%) du produit attendu sous la forme d'une huile incolore.

### EXEMPLE 82

### 1-[4'-(2,4-Dioxo-thiazolidin-5-ylmethyl)-biphenyl-3-yl]-1-ethyl-3-phenyl-urée

### (a) (3-Bromo-phenyl)-ethyl-amine

Dans un ballon et sous courant d'azote, on introduit 5 g (29,1 mmol) de 3-bromoaniline dans 500 ml d'éther. On ajoute 4,5 ml (32 mmol) de triéthylamine et 15,2 ml (117 mmol) de diéthylsulfate. Le milieu est chauffé à reflux 24 heures puis 8 jours à température ambiante. Il est, ensuite, versé dans de l'eau et extrait à l'éther. La phase organique est lavée avec une solution aqueuse saturée en chlorure de sodium puis est séchée sur sulfate de magnésium, filtrée et évaporée. Le résidu est purifié par chromatographie sur colonne de silice élué avec un mélange heptane - acétate d'éthyle (95/5) et, on obtient 2,5 g (43%) du produit attendu.

### (b) 3'-Ethylamino-biphenyl-4-carbaldehyde

De manière analogue à l'exemple 1(e), par réaction de 1,25 g (6,25 mmol) de (3-Bromo-phenyl)-ethyl-amine avec 1,4 g (9,4 mmol) d'acide 4-formylbenzèneboronique, on obtient après purification par chromatographie sur colonne de silice avec un mélange éluant heptane - acétate d'éthyle (75/25), 1 g (71 %) du produit attendu sous la forme d'un solide jaune.

### (c) 5-(3'-Ethylamino-biphenyl-4-ylmethylene)-thiazolidine-2,4-dione

De manière analogue à l'exemple 1(f), par réaction de 900 mg (4 mmol) de 3'-Ethylamino-biphenyl-4-carbaldehyde avec 470 mg (4 mmol) de 2,4-thiazolidinedione, on obtient 1 g (77%) du produit attendu sous la forme d'un solide orange.

### (d) 1-[4'-(2,4-Dioxo-thiazolidin-5-ylidenemethyl)-biphenyl-3-yl]-1-ethyl-3-phenyl-uree

De manière analogue à l'exemple 9(a), par réaction de 500 mg (1,54 mmol) de 5-(3'-Ethylamino-biphenyl-4-ylmethylene)-thiazolidine-2,4-dione avec 340 µl (3,1 mmol) de phénylisocyanate, on obtient 650 mg (95%) du produit attendu sous la forme d'un solide jaune.

### (e) 1-[4'-(2,4-Dioxo-thiazolidin-5-ylmethyl)-biphenyl-3-yl]-1-ethyl-3-phenyl-urée

De manière analogue à l'exemple 1(g), à partir de 650 mg (1,4 mmol) de 1-[4'-(2,4-Dioxo-thiazolidin-5-ylidenemethyl)-biphenyl-3-yl]-1-ethyl-3-phenyl-uree, on obtient après purification par chromatographie sur colonne de silice élué avec un mélange heptane - acétate d'éthyle (70/30), 400 mg (61 %) de 1-[4'-(2,4-Dioxo-thiazolidin-5-ylmethyl)-biphenyl-3-yl]-1-ethyl-3-phenyl-urée sous la forme d'une poudre blanche de point de fusion 186°C.

### EXEMPLE 83

L'activité agoniste vis à vis des récepteurs PPARγ des composés selon l'invention peut être évaluée par les tests de binding et de transactivation. Les résultats obtenus pour les composés selon l'invention sont regroupés dans le tableau suivant :

| Composés | % d'activation de PPARα | % d'activation de PPARγ | Binding PPAR-γ Kd en nM |
|---|---|---|---|
| Référence 1 : Wy 14643 | 100* | n.a. | n.a |
| Référence 2 : SB 219994 | n.a. | 100 | 0.5 |
| Référence 3 : BRL 49,653 | 3 | 83 | 23 |
| Exemple 1 | 8.2 | 102.2 | 55 |
| Exemple 2 | 8.2 | 42.9 | 263 |
| Exemple 10 | 0 | 22.1 | 800 |
| Exemple 11 | 2.4 | 74.5 | 289 |
| Exemple 12 | 0 | 60.4 | 7 |
| Exemple 13 | 0 | 70.2 | 24 |
| Exemple 21 | 14.9 | 90 | 190 |
| Exemple 22 | 7.5 | 95.3 | 76.6 |
| Exemple 24 | 8.7 | 95 | 72.3 |
| Exemple 26 | 2.1 | 88.6 | N.T |
| Exemple 28 | 1.1 | 79.8 | N.T |
| Exemple 30 | 8.9 | 80.3 | N.T |
| Exemple 32 | 0 | 74.9 | N.T |
| Exemple 34 | 17.6 | 102.5 | 2 |
| Exemple 35 | 11 | 56 | N.T |

| | | | |
|---|---|---|---|
| n.a signifie non actif N.T signifie non testé *... : % d'activation à la concentration de 1µM | | | |

Ces résultats montrent l'affinité des composés pour PPAR-γ et leur activité de transactivation. Ces résultats montrent plus particulièrement la spécificité de l'activation des composés de l'invention pour le sous-type PPAR-γ, comparé à l'activation des composés pour le sous-type PPAR-α.

### EXEMPLE 84

Dans cet exemple, on a illustré diverses formulations concrètes à base des composés selon l'invention.
A- VOIE ORALE
   (a) Comprimé de 0,2 g
      - Composé 1 0,001 g
      - Amidon 0,114 g
      - Phosphate bicalcique 0,020 g
      - Silice 0,020 g
      - Lactose 0,030 g
      - Talc 0,010 g
      - Stéarate de magnésium 0,005 g
   (b) Suspension buvable en ampoules de 5 ml
      - Composé 5 0,001 g
      - Glycérine 0,500 g
      - Sorbitol à 70% 0,500 g
      - Saccharinate de sodium 0,010 g
      - Parahydroxybenzoate de méthyle 0,040 g
      - Arome qs
      - Eau purifiée qsp5 ml
   (c) Comprimé de 0,8 g
      - Composé 2 0,500 g
      - Amidon prégélatinisé 0,100 g
      - Cellulose microcristalline 0,115 g
      - Lactose 0,075 g
      - Stéarate de magnésium 0,010 g
   (d) Suspension buvable en ampoules de 10 ml
      - Composé 4 0,200 g
      - Glycérine 1,000 g
      - Sorbitol à 70% 1,000 g
      - Saccharinate de sodium 0,010 g
      - Parahydroxybenzoate de méthyle 0,080 g
      - Arome qs
      - Eau purifiée qsp 10 ml
B- VOIE TOPIQUE
   (a) Onguent
      - Composé 6 0,020 g
      - Myristate d'isopropyle 81,700 g
      - Huile de vaseline fluide 9,100 g
      - Silice ("Aérosil 200" vendue par DEGUSSA) 9,180 g
   (b) Onguent
      - Composé 2 0,300 g
      - Vaseline blanche codex qsp 100 g
   (c) Crème Eau-dans-Huile non ionique
      - Composé 1 0,100 g
      - Mélange d'alcools de lanoline émulsifs, de cires et d'huiles ("Eucerine anhydre" vendu par BDF) 39,900 g
      - Parahydroxybenzoate de méthyle 0,075 g
      - Parahydroxybenzoate de propyle 0,075 g
      - Eau déminéralisée stérile qsp 100 g
   (d) Lotion
      - Composé 3 0,100 g
      - Polyéthylène glycol (PEG 400) 69,900 g
      - Ethanol à 95% 30,000 g
   (e) Onguent hydrophobe
      - Composé 5 0,300 g
      - Miristate d'isopropyle 36,400 g
      - Huile de silicone ("Rhodorsil 47 V 300" vendu par RHONE-POULENC) 36,400 g
      - Cire d'abeille 13,600 g
      - Huile de silicone ("Abil 300.000 cst" vendu par GOLDSCHMIDT) qsp 100 g
   (f) Crème Huile-dans-Eau non ionique
      - Composé 2 1,000 g
      - Alcool cétylique 4,000 g
      - Monostéarate de glycérole 2,500 g
      - Stéarate de PEG 50 2,500 g
      - Beurre de karité 9,200 g
      - Propylène glycol 2,000 g
      - Parahydroxybenzoate de méthyle 0,075 g
      - Parahydroxybenzoate de propyle 0,075 g
      - Eau déminéralisée stérile qsp 100 g

## Revendications

1. Composés **caractérisés par le fait qu'**ils répondent à la formule (I) suivante : 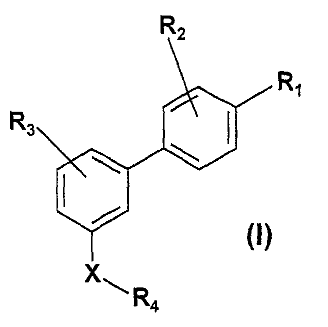 dans laquelle:
- **R**_{**1**} représente un radical de formule **(a)** ou **(b)** suivante: 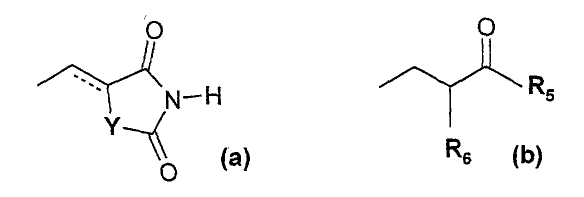 Y, R₅ et R₆ ayant les significations données ci-après,
- **R**_{**2**} et **R**_{**3**} identiques ou différents représentent un atome d'hydrogène, un radical alkyle ayant de 1 à 12 atomes de carbone, un radical aryle, un atome d'halogène, un radical -OR₇, un radical polyéther, un radical nitro, ou un radical amino pouvant éventuellement être substitué par un (ou plusieurs) radical (aux) alkyle(s) ayant de 1 à 12 atomes de carbone,
R₇ ayant la signification donnée ci-après,
- **X** représente une liaison de structure suivante:
-(CH₂)ₘ-(Z)ₙ-(CO)ₚ-(W)_{q}-
ladite liaison de structure pouvant être lue de gauche à droite ou inversement,
Z, W, m, n, p, q ayant les significations données ci-après,
- **R**_{**4**} représente un radical alkyle ayant de 1 à 12 atomes de carbone, un radical aryle, un radical aralkyle, un radical hétéroaryle, ou un radical 9-fluorenylméthyle,
- Y représente un radical CH₂ ou un atome de soufre,
- R₅ représente un radical hydroxy, un radical alkoxy ayant de 1 à 6 atomes de carbone, un radical NH-OH, ou un radical N(R₈)(R₉),
R₈ et R₉ ayant les significations données ci-après,
- R₆ représente un radical alkyle ayant de 1 à 12 atomes de carbone, un radical OR₁₀, un radical SR₁₀, ou un radical (CH₂)ᵣ-COR₁₁,
r, R₁₀ et R₁₁ ayant les significations données ci-après,
- R₇ représente un atome d'hydrogène, un radical alkyle ayant de 1 à 12 atomes de carbone, ou un radical aralkyle,
- Z représente un atome d'oxygène, de soufre ou un radical N-R₁₂,
R₁₂ ayant la signification donnée ci-après,
- W représente un atome d'oxygène, de soufre, un radical NR₁₃ ou un radical CH₂,
R₁₃ ayant la signification donnée ci-après,
- m, n, p, q, identiques ou différents, peuvent prendre les valeurs 0 ou 1,
étant entendu que la somme m+n+p+q est supérieure ou égale à 2 et que lorsque p prend la valeur 0 alors n ou q est égal à 0,
- R₈ représente un atome d'hydrogène ou un radical alkyle ayant de 1 à 12 atomes de carbone,
- R₉ représente un atome d'hydrogène, un radical alkyle ayant de 1 à 12 atomes de carbone ou un radical aryle,
- r représente 0 ou 1,
- R₁₀ représente un radical alkyle ayant de 1 à 12 atomes de carbone, un radical aryle, ou un radical aralkyle,
- R₁₁ représente un radical hydroxy, un radical OR₁₄, ou un radical N(R₁₅)(R₁₆),
- R₁₂ représente un atome d'hydrogène ou un radical alkyle ayant de 1 à 12 atomes de carbone,
- R₁₃ représente un atome d'hydrogène ou un radical alkyle ayant de 1 à 12 atomes de carbone,
- R₁₄ représente un radical alkyle ayant de 1 à 12 atomes de carbone, un radical aryle ou un radical aralkyle,
- R₁₅ représente un atome d'hydrogène ou un radical alkyle ayant de 1 à 12 atomes de carbone,
- R₁₆ représente un atome d'hydrogène, un radical alkyle ayant de 1 à 12 atomes de carbone, un radical aryle, un radical aralkyle ou un radical hétéroalkyle,
et les isomères optiques et géométriques desdits composés de formule (i) ainsi que leurs sels.

2. Composés selon la revendication 1, **caractérisés par le fait qu'**ils se présentent sous forme de sels d'un métal alcalin ou alcalino-terreux, de sels de zinc, ou de sels d'une amine organique.

3. Composés selon l'une des revendications 1 ou 2, **caractérisés par le fait que** les radicaux alkyles ayant de 1 à 12 atomes de carbone sont choisis parmi les radicaux méthyle, éthyle, isopropyle, butyle, tertiobutyle, hexyle, octyle, décyle, ou dodécyle.

4. Composés selon l'une des revendications 1 ou 2, **caractérisés par le fait que** les radicaux polyethers sont choisis parmi les radicaux polyethers ayant de 1 à 6 atomes de carbone interrompus par au moins un atome d'oxygène tel que les radicaux méthoxyméthoxy, éthoxyméthoxy, ou méthoxyéthoxyméthoxy.

5. Composés selon l'une des revendications 1 ou 2, **caractérisés par le fait que** l'atome d'halogène est choisi dans le groupe constitué par un atome de fluor, de chlore, ou de brome.

6. Composés selon l'une des revendications 1 ou 2, **caractérisés par le fait que** le radical alkoxy ayant de 1 à 6 atomes de carbone est choisi dans le groupe constitué par les radicaux méthoxy, éthoxy, isopropyloxy, tertio-butoxy, ou hexyloxy.

7. Composés selon l'une des revendications 1 ou 2, **caractérisés par le fait que** le radical aryle est choisi parmi un radical phényle pouvant être mono ou di-substitué par un atome d'halogène, un radical CF₃, un radical alkyle ayant de 1 à 12 atomes de carbone, un radical alkoxy ayant de 1 à 6 atomes de carbone, une fonction nitro, un radical polyéther, un radical hydroxyle éventuellement protégé par un groupe acétyle, benzoyle ou une fonction amino éventuellement protégée par un groupe acétyle, benzoyle ou éventuellement substituée par au moins un alkyle ayant de 1 à 12 atomes de carbone.

8. Composés selon l'une des revendications 1 ou 2, **caractérisés par le fait que** le radical aralkyle est choisi parmi un radical benzyle ou phénethyle pouvant être mono ou di-substitué par un atome d'halogène, un radical CF₃, un radical alkyle ayant de 1 à 12 atomes de carbone, un radical hydroxyle, un radical alkoxy ayant de 1 à 6 atomes de carbone, une fonction nitro, un radical polyéther, un radical hydroxyle éventuellement protégé par un groupe acétyle, benzoyle ou une fonction amino éventuellement protégée par un groupe acétyle, benzoyle ou éventuellement substituée par au moins un alkyle ayant de 1 à 12 atomes de carbone.

9. Composés selon l'une des revendications 1 ou 2, **caractérisés par le fait que** le radical hétéroaryle est choisi dans le groupe constitué par un radical pyridyle, furyle, thiényle, ou isoxazolyle, éventuellement substitué par au moins un halogène, un alkyle ayant de 1 à 12 atomes de carbone, un alkoxy ayant de 1 à 6 atomes de carbone, une fonction nitro, un radical polyéther, un hydroxyle éventuellement protégé par un groupe acétyle, benzoyle ou une fonction amino éventuellement protégée par un groupe acétyle, benzoyle ou éventuellement substituée par au moins un alkyle ayant de 1 à 12 atomes de carbone.

10. Composés selon la revendication 1, **caractérisés par le fait qu'**ils sont pris, seuls ou en mélanges, dans le groupe constitué par :
N-[4'-(2,4-Dioxo-thiazolidin-5-ylmethyl)-biphenyl-3-ylmethyl]-N-methyl-benzamide
5-{3'-[(Methyl-pyridin-2-yl-amino)-methyl]-biphenyl-4-ylmethyl}-thiazolidine-2,4-dione
N-[4'-(2,4-Dioxo-thiazolidin-5-ylmethyl)-biphenyl-3-ylmethyl]-benzamide
3-{3'-[(Benzoyl-methyl-amino)-methyl]-biphenyl-4-yl}-2-methyl-propionate d'éthyle
Acide 2-methyl-3-(3'-{[methyl-(1-phenyl-methanoyl)-amino]-methyl]-biphenyl-4-yl)-propionique
N-[4'-(2-Carbamoyl-propyl)-biphenyl-3-ylmethyl]-N-methyl-benzamide
N-Methyl-N-[4'-(2-phenylcarbamoyl-propyl)-biphenyl-3-ylmethyl]-benzamide
N-[4'-(2-Hydroxycarbamoyl-propyl)-biphenyl-3-ylmethyl]-N-methyl-benzamide
1-[4'-(2,4-Dioxo-thiazolidin-5-ylmethyl)-biphenyl-3-ylmethyl]-3-phenyl-urée
1-[4'-(2,4-Dioxo-thiazolidin-5-ylmethyl)-biphenyl-3-ylmethyl]-1-methyl-3-phenyl-urée
[4'-(2,4-Dioxo-thiazolidin-5-ylmethyl)-biphenyl-3-ylmethyl]-methyl-carbamate de tert-butyle
N-[4'-(2,4-dioxo-thiazolidin-5-ylmethyl)-biphenyl-3-ylmethyl]-N-methyl-nonanamide
Acide (S)-2-ethoxy-3-(3'-{[methy)-(1-phenyl-methanoyl)-amino]-methyl}-biphenyl-4-yl)-propionique
2-(3'-{[methyl-(1-phenyl-methanoyl)-amino]-methyl}-biphenyl-4-ylmethyl)-malonate de monométhyle
2-(3'-{[Methyl-(1-phenyl-methanoyl)-amino]-methyl}-biphenyl-4-ylmethyl)-malonate de diméthyle
2-(3'-{[Methyl-(1-phenyl-methanoyl)-amino]-methyl}-biphenyl-4-ylmethyl)-malonamate de méthyle
N-[4'-(2,4-Dioxo-thiazolidin-5-ylmethyl)-biphenyl-3-ylmethyl]-N-ethyl-benzamide
N-[4'-(2,4-Dioxo-thiazolidin-5-ylmethyl)-biphenyl-3-ylmethyl]-N-pentyl-benzamide
[4'-(2,4-Dioxo-thiazolidin-5-ylmethyl)-biphenyl-3-ylmethyl]-ethyl-carbamate de tert-butyle
[4'-(2,4-Dioxo-thiazolidin-5-ylmethyl)-biphenyl-3-ylmethyl]-propyl- carbamate de tert-butyle
[4'-(2,4-Dioxo-thiazolidin-5-ylmethyl)-biphenyl-3-ylmethyl]-methyl-carbamate de 9H-fluoren-9-ylmethyle
N-[4'-(2,4-Dioxo-thiazolidin-5-ylmethyl)-biphenyl-3-ylmethyl]-2,2,N-trimethyl-propionamide
N-octyl-4'-(2,4-Dioxo-thiazolidin-5-ylmethyl)-biphenyl-3-carboxamide
N-[4'-(2,4-Dioxo-thiazolidin-5-ylmethyl)-biphenyl-3-ylmethyl]-N-methyl-3-phenyl-propionamide
2-[4'-(2,4-Dioxo-thiazolidin-5-ylmethyl)-biphenyl-3-yl]-N-methyl-N-phenyl-acetamide
N-[4'-(2,4-Dioxo-thiazolidin-5-ylidenemethyl)-biphenyl-3-ylmethyl]-N-propyl-benzamide
[4'-(2,4-dioxo-thiazolidin-5-ylméthyl)-biphenyl-3-yl]-carbamate de tert-butyle
N-[4'-(2,4-Dioxo-thiazolidin-5-ylmethyl)-biphenyl-3-ylmethyl]-3,4-diethoxy-N-methyl-benzamide
Acide 2-(3'-{[Methyl-(1-phenyl-methanoyl)-amino]-methyl}-biphenyl-4-ylmethyl)-malonamique
N-benzyl-N-méthyl-4'-(2,4-Dioxo-thiazolidin-5-ylmethyl)-biphenyl-3-carboxamide
N-benzyl-4'-(2,4-Dioxo-thiazolidin-5-ylmethyl)-biphenyl-3-carboxamide
N-[4'-(2,4-dioxo-thiazolidin-5-ylmethyl)-biphenyl-3-ylmethyl]-N-methyl-décanamide
N-[4'-(2,4-Dioxo-thiazolidin-5-ylmethyl)-biphenyl-3-yl]-2-phenyl-acetamide
N-[4'-(2,4-dioxo-thiazolidin-5-ylmethyl)-biphenyl-3-ylmethyl]-N-methyl-octanamide
N-[4'-(2,4-dioxo-thiazolidin-5-ylmethyl)-biphenyl-3-ylmethyl]-N-methyl-heptanamide
Acide N-Hydroxy-2-(3'-{[methyl-(1-phenyl-methanoyl)-amino]-methyl}-biphenyl-4-ylmethyl)-malonamique
N-[4'-(2,4-Dioxo-thiazolidin-5-ylmethyl)-biphenyl-3-ylmethyl]-N-methyl-2-phenyl-acetamide
N-[4'-(2,4-Dioxo-thiazolidin-5-ylmethyl)-biphenyl-3-ylmethyl]-4-methoxy-N-methyl-benzamide
N-[4'-(2,4-Dioxo-thiazolidin-5-ylmethyl)-biphenyl-3-ylmethyl]-3-methoxy-N-methyl-benzamide
N-[4'-(2,4-Dioxo-thiazolidin-5-ylmethyl)-biphenyl-3-ylmethyl]-3 -N-dimethyl-benzamide
N-[4'-(2,4-Dioxo-thiazolidin-5-ylmethyl)-biphenyl-3-ylmethyl]-N-methyl-4-propyl-benzamide
N-[4'-(2,4-Dioxo-thiazolidin-5-ylmethyl)-biphenyl-3-ylmethyl]-4,N-dimethyl-benzamide.
N- [4'-(2,4-dioxo-thiazolidin-5-ylmethyl)-biphenyl-3-ylmethyl]-N-methyl- Isoxazole-5-carboxamide
N-[4'-(2,4-Dioxo-thiazolidin-5-ylmethyl)-biphenyl-3-ylmethyl]-4-ethoxy-N-methyl-benzamide
N-[4'-(2,4-Dioxo-thiazolidin-5-ylmethyl)-biphenyl-3-ylmethyl]-4-fluoro-N-methyl-benzamide
4-Dimethylamino-N-[4'-(2,4-dioxo-thiazolidin-5-ylmethyl)-biphenyl-3-ylmethyl]-N-methyl-benzamide
N-[4'-(2,4-Dioxo-thiazolidin-5-ylmethyl)-biphenyl-3-ylmethyl]- N-methyl-nicotinamide
3,5-Dichloro-N-[4'-(2,4-dioxo-thiazolidin-5-ylmethyl)-biphenyl-3-ylmethyl]-N-methyl-benzamide
N- [4'-(2,4-dioxo-thiazolidin-5-ylmethyl)-biphenyl-3-ylmethyl]-N-methyl-Thiophene-2-carboxamide
N- [4'-(2,4-dioxo-thiazolidin-5-ylmethyl)-biphenyl-3-ylmethyl]-N-methyl- Hexanamide
N-[4'-(2,4-Dioxo-thiazolidin-5-ylmethyl)-biphenyl-3-ylmethyl]-2-methoxy-N-methyl-benzamide
N-[4'-(2,4-dioxo-thiazolidin-5-ylmethyl)-biphenyl-3-ylmethyl]-N-methyl- pyridine-2-carboxamide
N- [4'-(2,4-dioxo-thiazolidin-5-ylmethyl)-biphenyl-3-ylmethyl]-N-methyl- furan-2-carboxamide
4-Butoxy-N-[4'-(2,4-dioxo-thiazolidin-5-ylmethyl)-biphenyl-3-ylmethyl]-N-methyl-benzamide
N- [4'-(2,4-dioxo-thiazolidin-5-ylmethyl)-biphenyl-3-ylmethyl]-N-methyl- Thiophene-3-carboxamide
Acetate de 4-{[4'-(2,4-dioxo-thiazolidin-5-ylmethyl)-biphenyl-3-ylmethyl]-methyl-carbamoyl}-phenyl
N-[4'-(2,4-Dioxo-thiazolidin-5-ylmethyl)-biphenyl-3-ylmethyl]-4-hydroxy-N-methyl-benzamide
N-[4'-(2,4-Dioxo-thiazolidin-5-ylmethyl)-biphenyl-3-ylmethyl]-2 N-dimethyl-benzamide
2-Butyl-N- [4'-(2,4-dioxo-thiazolidin-5-ylmethyl)-biphenyl-3-ylmethyl]-N-methyl-octanamide
4-Acetylamino-N-[4'-(2,4-dioxo-thiazolidin-5-ylmethyl)-biphenyl-3-ylmethyl]-N-methyl-benzamide
N-[4'-(2,4-Dioxo-thiazolidin-5-ylmethyl)-biphenyl-3-ylmethyl]-N-methyl-carbamate d'hexyle
1-[4'-(2,4-Dioxo-thiazolidin-5-ylmethyl)-biphenyl-3-yl]-3-phenyl-uree
N-[4'-(2,4-Dioxo-thiazolidin-5-ylmethyl)-biphenyl-3-yl]-N-methyl-2-phenyl-acetamide
1-[4'-(2,4-Dioxo-thiazolidin-5-ylmethyl)-biphenyl-3-yl]-1-methyl-3-phenyl-uree
1-[4'-(2,4-Dioxo-thiazolidin-5-ylmethyl)-biphenyl-3-yl]-3-heptyl-1-methyl-uree
Ester 4-monométhylique de l'acide 2-{3'-[(Heptanoyl-methyl-amino)-methyl]-biphenyl-4-ylmethyl}-succinique
Acide 2-{3'-[(Methyl-octanoyl-amino)-methyl]-biphenyl-4-ylmethyl}-succinique
N- [4'-(2,5-dioxo-pyrrolidin-3-ylmethyl)-biphenyl-3-ylmethyl]-N-methyl-heptanamide.
N-Phenyl-carbamate de 3-[4'-(2,4-dioxo-thiazolidin-5-ylidenemethyl)]biphenyle
N-Heptyl-carbamate de 3-[4'-(2,4-dioxo-thiazolidin-5-ylidenemethyl)]biphenyle
Phenyl-acetate de 3-[4'-(2,4-dioxo-thiazolidin-5-ylmethyl)]biphenyle
Nonanoate de 3-[4'-(2,4-dioxo-thiazolidin-5-ylmethyl)]biphenyle
N-Heptyl-carbamate de 3-[4'-(2,4-dioxo-thiazolidin-5-ylmethyl)]biphenyle
N-Phenyl-carbamate de 3-[4'-(2,4-dioxo-thiazolidin-5-ylmethyl)]biphenyle
N-[6-benzyloxy-4'-(2,4-dioxo-thiazolidin-5-ylidenemethyl)-biphenyl-3-ylmethyl]-N-methyl-octanamide
N-[4'-(2,4-dioxo-thiazolidin-5-ylmethyl)-6-hydroxy-biphenyl-3-ylmethyl]-N-methyl-octanamide
N-[4-benzyloxy-4'-(2,4-dioxo-thiazolidin-5-ylidenemethyl)-biphenyl-3-ylmethyl]-N-methyl-octanamide
N-[4"-(2,4-dioxo-thiazolidin-5-ylmethyl)-[1,1';3',1"]terphenyl-5'-ylmethyl]-N-methyl-octanamide
N-[4'-(2,4-dioxo-thiazolidin-5-ylmethyl)-2'-methyl-biphenyl-3-ylmethyl]-N-methyl-octanamide
N-[4'-(2,4-dioxo-thiazolidin-5-ylmethyl)-3'-methyl-biphenyl-3-ylmethyl]-N-methyl-octanamide
Acide (S)-2-Ethoxy-3-{3'-[(methyl-octanoyl-amino)-methyl]-biphenyl-4-yl}-propionique
1-[4'-(2,4-Dioxo-thiazolidin-5-ylmethyl)-biphenyl-3-yl]-1-ethyl-3-phenyl-urée
N-[4'-(2,4-dioxo-thiazolidin-5-ylmethyl)-biphenyl-3-yl]-N-methyl-docanamide
N-[4'-(2,4-dioxo-thiazolidin-5-ylmethyl)-biphenyl-3-yl]-N-methyl-nonanamide
N-[4'-(2,4-Dioxo-thiazolidin-5-ylmethyl)-biphenyl-3-yl]-N-methyl-2-(4-butoxy-phenyl)-acetamide
N-[4'-(2,4-Dioxo-thiazolidin-5-ylmethyl)-biphenyl-3-yl]-N-methyl-2-(4-methoxy-phenyl)-acetamide
N-[4'-(2,4-Dioxo-thiazolidin-5-ylmethyl)-biphenyl-3-yl]-N-methyl-2-(4-ethoxy-phenyl)-acetamide
N-[4'-(2,4-Dioxo-thiazolidin-5-ylmethyl)-biphenyl-3-yl]-N-methyl-2-(4-hydroxy-phenyl)-acetamide
1-[4'-(2,4-Dioxo-thiazolidin-5-ylmethyl)-biphenyl-3-yl]-1-methyl-3-(4-butoxy-phenyl)-uree
1-[4'-(2,4-Dioxo-thiazolidin-5-ylmethyl)-biphenyl-3-yl]-1-methyl-3-(4-methoxy-phenyl)-uree
1-[4'-(2,4-Dioxo-thiazolidin-5-ylmethyl)-biphenyl-3-yl]-1-methyl-3-(4-ethoxy-phenyl)-uree
1-[4'-(2,4-Dioxo-thiazolidin-5-ylmethyl)-biphenyl-3-yl]-1-methyl-3-(4-hydroxy-phenyl)-uree
Acide (S)-2-ethoxy-3-(3'-{[methyl-(1-(4-butoxy)-phenylmethanoyl)-amino]-methyl}-biphenyl-4-yl)-propionique
Acide (S)-2-ethoxy-3-(3'-{[methyl-(1-(4-methoxy)-phenylmethanoyl)-amino]-methyl}-biphenyl-4-yl)-propionique
Acide (S)-2-ethoxy-3-(3'-{[methyl-(1-(4-ethoxy-phenyl)-methanoyl)-amino]-methyl}-biphenyl-4-yl)-propionique
Acide (S)-2-ethoxy-3-(3'-{(methyl-(1-(4-hydroxy-phenyl)-methanoyl)-amino]-methyl}-biphenyl-4-yl)-propionique
Acide (S)-2-phenoxy-3-(3'-{[methyl-(1-(4-butoxy-phenyl)-methanoyl)-amino]-methyl}-biphenyl-4-yl)-propionique
Acide (S)-2-phenoxy-3-(3'-{[methyl-(1-(4-methoxy-phenyl)-methanoyl)-amino]-methyl}-biphenyl-4-yl)-propionique
Acide (S)-2-phenoxy-3-(3'-{[methyl-(1-(4-ethoxy-phenyl)-methanoyl)-amino]-methyl}-biphenyl-4-yl)-propionique
Acide (S)-2-phenoxy-3-(3'-{[methyl-(1-(4-hydroxy-phenyl)-methanoyl)-amino]-methyl}-biphenyl-4-yl)-propionique
Acide (S)-2-phenoxy-3-(3'-{[methyl-(1-phenyl-methanoyl)-amino]-methyl}-biphenyl-4-yl)-propionique
Acide (S)-2-phenoxy-3-{3'-[(methyl-octanoyl-amino)-methyl]-biphenyl-4-yl}-propionique
5-{3'-[Methyl-(2-oxo-2-phenyl-ethyl)-amino]-biphenyl-4-ylmethyl}-thiazolidine-2,4-dione
5-[3'-(Methyl-phenethyl-amino)-biphenyl-4-ylmethyl]-thiazolidine-2,4-dione
[4'-(2,4-Dioxo-thiazolidin-5-ylmethyl)-biphenyl-3-yl]-methyl-carbamate de phenyle
[4'-(2,4-Dioxo-thiazolidin-5-ylmethyl)-biphenyl-3-yl]-methyl-carbamate de tert-butyle

11. Composés selon la revendication 1 ou 2, **caractérisés par le fait qu'**ils présentent l'une au moins des caractéristiques suivantes :
- R₁ représente le radical de formule (a) ou le radical de formule (b) où R₅ représente un radical hydroxy et R₆ représente le radical OR_{10,}
- X représente une liaison de structure choisie parmi -CH₂-N(R₁₂)-CO-, ou -NR12-(CO)-NR13, ou -NR12-(CO)-CH2-, ces liaisons étant lues de gauche à droite ou inversement.

12. Composition cosmétique, **caractérisée par le fait qu'**elle comprend, dans un support physiologiquement acceptable, au moins un des composés tels que définis à l'une quelconque des revendications 1 à 11.

13. Composition selon la revendication 12, **caractérisée en ce que** la concentration en composé(s) selon l'une des revendications 1 à 11 est comprise entre 0,001 % et 3 % en poids par rapport au poids total de la composition.

14. Utilisation cosmétique d'une composition telle que définie à l'une des revendications 12 ou 13 pour l'hygiène corporelle ou capillaire.

15. Composés selon l'une quelconque des revendications 1 à 11 à titre de médicament.

16. Utilisation d'un composé selon l'une quelconque des revendications 1 à 11 dans la fabrication d'une composition destinée à réguler et/ou à restaurer le métabolisme des lipides cutanés.

17. Utilisation d'un composé selon l'une quelconque des revendications 1 à 11 dans la fabrication d'une composition destinée au traitement :
- des affections dermatologiques liées à un désordre de la kératinisation portant sur la différenciation et sur la prolifération notamment les acnés vulgaires, comédoniennes, polymorphes, rosacées, les acnés nodulokystiques, conglobata, les acnés séniles, les acnés secondaires telles que l'acné solaire, médicamenteuse ou professionnelle,
- des ichtyoses, des états ichtyosiformes, de la maladie de Darrier, des kératodermies palmoplantaires, des leucoplasies et des états leucoplasiformes, du lichen cutané ou muqueux (buccal),
- des affections dermatologiques avec une composante immuno-allergique inflammatoire, avec ou sans trouble de la prolifération cellulaire, notamment le psoriasis cutané, muqueux ou unguéal, le rhumatisme psoriasique, l'atopie cutanée, telle que l'eczéma, l'atopie respiratoire ou l'hypertrophie gingivale,
- des proliférations dermiques ou épidermiques bénignes ou malignes, d'origine virale ou non, notamment les verrues vulgaires, les verrues planes l'épidermodysplasie verruciforme, les papillomatoses orales ou florides, le lymphome T,
- des proliférations pouvant être induites par les ultra-violets notamment des épithélioma baso et spinocellulaires,
- des lésions précancéreuses cutanées notamment les kératoacanthomes,
- des dermatoses immunes notamment le lupus érythémateux,
- des maladies immunes bulleuses,
- des maladies du collagène notamment la sclérodermie,
- des affections dermatologiques ou générales à composante immunologique,
- de désordres cutanés dus à une exposition aux rayonnements U.V, du vieillissement de la peau, photo-induit ou chronologique ou des pigmentations et des kératoses actiniques, ou toutes pathologies associées au vieillissement chronologique ou actinique notamment la xérose,
- des troubles de la fonction sébacée notamment l'hyperséborrhée de l'acné ou la séborrhée simple,
- des troubles de la cicatrisation ou des vergetures,
- des désordres de la pigmentation, tel l'hyperpigmentation, le mélasma, l'hypopigmentation ou le vitiligo,
- des affections du métabolisme des lipides, tel l'obésité, l'hyperlipidémie, ou le diabète non insulino-dépendant,
- des affections inflammatoires telles que l'arthrite,
- des états cancéreux ou précancéreux,
- de l'alopécie de différentes origines, notamment l'alopécie due à la chimiothérapie ou aux rayonnements,
- des troubles du systèmes immunitaire, tel l'asthme, le diabète sucré de type I, la sclérose en plaque, ou autres disfonctionnements sélectifs du système immunitaire.
- des affections du système cardiovasculaire telles que l'artériosclérose ou l'hypertension,

18. Composition pharmaceutique, **caractérisée par le fait qu'**elle comprend, dans un support physiologiquement acceptable, au moins l'un des composés tels que définis à l'une quelconque des revendications 1 à 11.

19. Composition selon la revendication 18, **caractérisée en ce que** la concentration en composé(s) selon l'une des revendications 1 à 11 est comprise entre 0,001 % et 10 % en poids par rapport au poids total de la composition.

20. Composition selon la revendication 18, **caractérisée en ce que** la concentration en composé(s) selon l'une des revendications 1 à 11 est comprise entre 0, 01 % et 1 % en poids par rapport au poids total de la composition.

## Patentansprüche

1. Verbindungen, die durch die nachstehende Formel (I) gekennzeichnet sind: 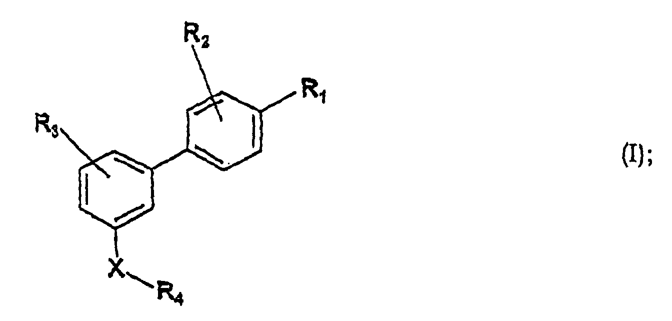 es bedeuten:
- **R**_{**1**} eine Gruppe der folgenden Formel **(a)** oder **(b)**: 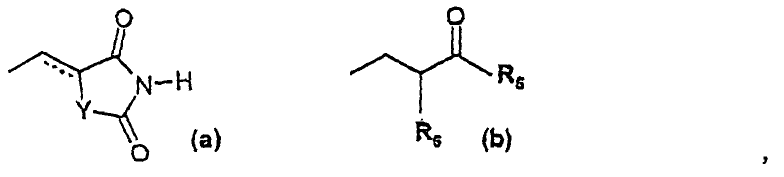
wobei Y, R₅ und R₆ die nachstehend angegebenen Bedeutungen besitzen,
- **R**_{**2**} und **R**_{**3**}**,** die gleich oder verschieden sind, ein Wasserstoffatom, C₁₋₁₂-Alkyl, Aryl, ein Halogenatom, eine Gruppe -OR₇, eine Polyethergruppe, Nitro oder Amino, das gegebenenfalls mit einer oder mehreren C₁₋₁₂-Alkylgruppen substituiert ist,
wobei R₇ die nachstehend angegebene Bedeutung besitzt,
- **X** eine Verknüpfungsgruppe der folgenden Formel:
-(CH₂)ₘ-(Z)ₙ-(CO)ₚ-(W)_{q}-,
die von links nach rechts oder umgekehrt gelesen werden kann,
wobei Z, W, m, n, p und q die nachstehend angegebene Bedeutung besitzen,
- **R**_{**4**} C₁₋₁₂-Alkyl, Aryl, Aralkyl, Heteroaryl oder 9-Fluorenylmethyl,
- Y CH₂ oder ein Schwefelatom,
- R₅ Hydroxy, C₁₋₆-Alkoxy, NH-OH oder N(R₈)(R₉),
wobei R₈ und R₉ die nachstehend angegebenen Bedeutungen besitzen,
- R₆ C₁₋₁₂-Alkyl, OR₁₀, SR₁₀ oder (CH₂)ᵣ-COR₁₁,
wobei R, R₁₀ und R₁₁ die nachstehend angegebenen Bedeutungen besitzen,
- R₇ ein Wasserstoffatom, C₁₋₁₂-Alkyl oder Aralkyl,
- Z ein Sauerstoffatom, ein Schwefelatom oder NR₁₂,
wobei R₁₂ die nachstehend angegebene Bedeutung besitzt,
- W ein Sauerstoffatom, ein Schwefelatom, NR₁₃ oder CH₂,
wobei R₁₃ die nachstehend angegebene Bedeutung besitzt,
- m, n, p, q, die gleich oder verschieden sind, den Wert O oder 1 mit der Maßgabe, dass
die Summe m+n+p+q ≥ 2 ist
und
für p = 0 n oder q gleich 0 ist,
- R₈ ein Wasserstoffatom oder C₁₋₁₂-Alkyl,
- R₉ ein Wasserstoffatom, C₁₋₁₂-Alkyl oder Aryl,
- r 0 oder 1,
- R₁₀ C₁₋₁₂-Alkyl, Aryl oder Aralkyl,
- R₁₁ Hydroxy, OR₁₄ oder N(R₁₅)(R₁₆),
- R₁₂ ein Wasserstoffatom oder C₁₋₁₂-Alkyl,
- R₁₃ ein Wasserstoffatom oder C₁₋₁₂-Alkyl,
- R₁₄ C₁₋₁₂-Alkyl, Aryl oder Aralkyl,
- R₁₅ ein Wasserstoffatom oder C₁₋₁₂-Alkyl
und
- R₁₆ ein Wasserstoffatom, C₁₋₁₂-Alkyl, Aryl, Aralkyl oder
Heteroalkyl,
und die optischen und geometrischen Isomeren dieser Verbindungen der Formel (I) sowie ihre Salze.

2. Verbindungen nach Anspruch 1, **dadurch gekennzeichnet, dass** sie in Form von Salzen eines Alkalimetalls oder Erdalkalimetalls, von Zinksalzen oder von Salzen eines organischen Amins vorliegen.

3. Verbindungen nach Anspruch 1 oder 2, **dadurch gekennzeichnet, dass** die C₁₋₁₂-Alkylgruppen unter Methyl, Ethyl, Isopropyl, Butyl, *t*-Butyl, Hexyl, Octyl, Decyl und Dodecyl ausgewählt sind.

4. Verbindungen nach Anspruch 1 oder 2, **dadurch gekennzeichnet, dass** die Polyethergruppen unter Polyethergruppen mit 1 bis 6 Kohlenstoffatomen, die durch mindestens ein Sauerstoffatom unterbrochen sind, wie Methoxymethoxy, Ethoxymethoxy oder Methoxyethoxymethoxy, ausgewählt sind.

5. Verbindungen nach Anspruch 1 oder 2, **dadurch gekennzeichnet, dass** das Halogenatom unter Fluor, Chlor und Brom ausgewählt ist.

6. Verbindungen nach Anspruch 1 oder 2, **dadurch gekennzeichnet, dass** die C₁₋₆-Alkoxygruppe unter Methoxy, Ethoxy, Isopropyloxy, *t*-Butoxy und Hexyloxy ausgewählt ist.

7. Verbindungen nach Anspruch 1 oder 2, **dadurch gekennzeichnet, dass** die Arylgruppe unter Phenyl ausgewählt ist, das mono- oder disubstituiert sein kann mit einem Halogenatom, CF₃, C₁₋₁₂-Alkyl, C₁₋₆-Alkoxy, Nitro, einer Polyethergruppe, einer Hydroxygruppe, die gegebenenfalls durch eine Acetylgruppe oder eine Benzoylgruppe geschützt ist, oder einer Aminofunktion, die gegebenenfalls durch eine Acetylgruppe oder Benzoylgruppe geschützt oder gegebenenfalls mit mindestens einer C₁₋₁₂-Alkylgruppe substituiert ist.

8. Verbindungen nach Anspruch 1 oder 2, **dadurch gekennzeichnet, dass** die Aralkylgruppe ausgewählt ist unter Benzyl und Phenethyl, die mono- oder disubstituiert sein können mit einem Halogenatom, CF₃, C₁₋₁₂-Alkyl, Hydroxy, C₁₋₆-Alkoxy, Nitro, einer Polyethergruppe, einer Hydroxygruppe, die gegebenenfalls mit einer Acetylgruppe oder einer Benzoylgruppe geschützt ist, oder einer Aminofunktion, die mit einer Acetylgruppe oder einer Benzoylgruppe geschützt oder gegebenenfalls mit mindestens einer C₁₋₁₂-Alkylgruppe substituiert ist.

9. Verbindungen nach Anspruch 1 oder 2, **dadurch gekennzeichnet, dass** die Heteroarylgruppe ausgewählt ist unter Pyridyl, Furyl, Thienyl und Isoxazolyl, das gegebenenfalls mit mindestens einem Halogenatom, einer C₁₋₁₂-Alkylgruppe, einer C₁₋₆-Alkoxygruppe, einer Nitrogruppe, einer Polyethergruppe, einer Hydroxygruppe, die gegebenenfalls mit einer Acetylgruppe oder einer Benzoylgruppe geschützt ist, oder einer Aminofunktion substituiert ist, die gegebenenfalls durch eine Acetylgruppe oder eine Benzoylgruppe geschützt oder gegebenenfalls mit mindestens einer C₁₋₁₂-Alkylgruppe substituiert ist,

10. Verbindungen nach Anspruch 1, **dadurch gekennzeichnet, dass** sie allein oder in Form von Gemischen aus der Gruppe folgender Verbindungen ausgewählt sind:
N-[4'-(2,4-Dioxo-thiazolidin-5-ylmethyl)-biphenyl-3-ylmethyl]-N-methyl-benzamid
5-{3'-[(Methyl-pyridin-2-yl-amino)-methyl]-biphenyl-4-ylmethyl)-thiazolidin-2,4-dion
N-[4'-(2,4-Dioxo-thiazolidin-5-ylmethyl)-biphenyl-3-ylmethyl]-benzamid
3-{3'-[(Benzoyl-methyl-amino)-methyl]-biphenyl-4-yl}-2-methylpropionsäureethylester
2-Methyl-3-(3'-{[methyl-(1-phenyl-methanoyl)-amino]-methyl}biphenyl-4-yl)-propionsäure
N-[4'-(2-Carbamoyl-propyl)-biphenyl-3-ylmethyl]-N-methylbenzamid
N-Methyl-N-[4'-(2-phenylcarbamoyl-propyl)-biphenyl-3-ylmethyl]-benzamid
N-[4'-(2-Hydroxycarbamoyl-propyl)-biphenyl-3-ylmethyl]-N-methyl-benzamid
1-[4'(2,4-Dioxo-thiazolidin-5-ylmethyl)-biphenyl-3-ylmethyl]-3-phenyl-harnstoff
1- [4'-(2,4-Dioxo-thiazolidin-5-ylmethyl)-biphenyl-3-ylmethyl]-1-methyl-3-phenyl-harnstoff
[4'-(2,4-Dioxo-thiazolidin-5-ylmethyl)-biphenyl-3-ylmethyl]-methyl-carbaminsäure-*t*-butylester
N-[4'-(2,4-Dioxo-thiazolidin-5-ylmethyl)-biphenyl-3-ylmethyl]-N-methyl-nonanamid
(S)-2-Ethoxy-3-(3'-{[methyl-(1-phenyl-methanoyl)-amino]-methyl}biphenyl-4-yl)-propionsäure
2-(3'-{[Methyl-(1-phenyl-methanoyl)-amino]-methyl}-biphenyl-4-ylmethyl)-malonsäuremonomethylester
2-(3'-{[Methyl-(1-phenyl-methanoyl)-amino]-methyl}-biphenyl-4-ylmethyl)-malonsäuredimethylester
2-(3'-{[Methyl-(1-phenyl-methanoyl)-amino]-methyl}-biphenyl-4-ylmethyl)-malonamidsäuremethylester
N-[4'-(2,4-Dioxo-thiazolidin-5-ylmethyl)-biphenyl-3-ylmethyl]-N-ethyl-benzamid
N-[4'-(2,4-Dioxo-thiazolidin-5-ylmethyl)-biphenyl-3-ylmethyl]-N-pentyl-benzamid
[4'-(2,4-Dioxo-thiazolidin-5-ylmethyl)-biphenyl-3-ylmethyl]-ethylcarbaminsäure-*t*-butylester
{4'-(2,4-Dioxo-thiazolidin-5-ylmethyl)-biphenyl-3-ylmethyl]-propyl-carbaminsäure-*t*-butylester
[4'-(2,4-Dioxo-thiazolidin-5-ylmethyl)-biphenyl-3-ylmethyl]-methyl-carbaminsäure-fluoren-9-ylmethylester
N-[4'-(2,4-Dioxo-thiazolidin-5-ylmethyl)-biphenyl-3-ylmethyl]-2,2,N-trimethyl-propionamid
N-Octyl-4'-(2,4-dioxo-thiazolidin-5-ylmethyl)-biphenyl-3-carboxamid
N-[4'-(2,4-Dioxo-thiazolidin-5-ylmethyl)-biphenyl-3-ylmethyl]-N-methyl-3-phenyl-propionamid
2-[4'-(2,4-Dioxo-thiazolidin-5-ylmethyl)-biphenyl-3-yl]-N-methyl-N-phenyl-acetamid
N-[4'-(2,4-Dioxo-thiazolidin-5-ylidenmethyl)-biphenyl-3-ylmethyl]-N-propyl-benzamid
[4'-(2,4-Dioxo-thiazolidin-5-ylmethyl)-biphenyl-3-yl]-carbaminsäure-*t*-butylester
N-[4'-(2,4-Dioxo-thiazolidin-5-ylmethyl)-biphenyl-3-ylmethyl]-3,4-diethoxy-N-methylbenzamid
2-(3'-{[Methyl-(1-phenyl-methanoyl)-amino]-methyl}-biphenyl-4-ylmethyl)-malonamidsäure
N-Benzyl-N-methyl-4'-(2,4-dioxo-thiazolidin-5-ylmethyl)-biphenyl-3-carboxamid
N-Benzyl-4'-(2,4-dioxo-thiazolidin-5-ylmethyl)-biphenyl-3-carboxamid
N-[4'-(2,4-Dioxo-thiazolidin-5-ylmethyl)-biphenyl-3-ylmethyl]-N-methyl-decanamid
N-[4'-(2,4-Dioxo-thiazolidin-5-ylmethyl)-biphenyl-3-yl]-2-phenylacetamid
N-[4'-(2,4-Dioxo-thiazolidin-5-ylmethyl)-biphenyl-3-ylmethyl]-N-methyl-octanamid
N-[4'-(2,4-Dioxo-thiazolidin-5-ylmethyl)-biphenyl-3-ylmethyl]-N-methyl-heptanamid
N-Hydroxy-2-(3'-{[methyl-(1-phenyl-methanoyl)-amino]-methyl}biphenyl-4-ylmethyl)-malonamidsäure
N-[4'-(2,4-Dioxo-thiazolidin-5-ylmethyl)-biphenyl-3-ylmethyl]-N-methyl-2-phenylacetamid
N-[4'-(2,4-Dioxo-thiazolidin-5-ylmethyl)-biphenyl-3-ylmethyl]-4-methoxy-N-methylbenzamid
N-[4'-(2,4-Dioxo-thiazolidin-5-ylmethyl)-biphenyl-3-ylmethyl]-3-methoxy-N-methylbenzamid
N-[4'-(2,4-Dioxo-thiazolidin-5-ylmethyl)-biphenyl-3-ylmethyl]-3-N-dimethyl-benzamid
N-[4'-(2,4-Dioxo-thiazolidin-5-ylmethyl)-biphenyl-3-ylmethyl]-N-methyl-4-propylbenzamid
N-[4'-(2,4-Dioxo-thiazolidin-5-ylmethyl)-biphenyl-3-ylmethyl]-4-N-dimethyl-benzamid
N-[4'-(2,4-Dioxo-thiazolidin-5-ylmethyl)-biphenyl-3-ylmethyl]-N-methyl-isoxazol-5-carboxamid
N-[4'-(2,4-Dioxo-thiazolidin-5-ylmethyl)-biphenyl-3-ylmethyl]-4-ethoxy-N-methylbenzamid
N-[4'-(2,4-Dioxo-thiazolidin-5-ylmethyl)-biphenyl-3-ylmethyl]-4-fluor-N-methyl-benzamid
4-Dimethylamino-N-[4'-(2,4-dioxo-thiazolidin-5-ylmethyl)-biphenyl-3-ylmethyl]-N-methyl-benzamid
N-[4'-(2,4-Dioxo-thiazolidin-5-ylmethyl)-biphenyl-3-ylmethyl]-N-methyl-nicotinamid
3,5-Dichlor-N-[4'-(2,4-dioxo-thiazolidin-5-ylmethyl)-biphenyl-3-ylmethyl-N-methyl-benzamid
N-[4'-(2,4-Dioxo-thiazolidin-5-ylmethyl)-biphenyl-3-ylmethyl]-N-methyl-thiophen-2-carboxamid
N- [4'-(2,4-Dioxo-thiazolidin-5-ylmethyl)-biphenyl-3-ylmethyl]-N-methyl-hexanamid
N- [4'-(2,4-Dioxo-thiazolidin-5-ylmethyl)-biphenyl-3-ylmethyl]-2-methoxy-N-methyl-benzamid
N-[4'-(2,4-Dioxo-thiazolidin-5-ylmethyl)-biphenyl-3-ylmethyl]-N-methyl-pyridin-2-carboxamid
N-[4'-(2,4-Dioxo-thiazolidin-5-ylmethyl)-biphenyl-3-ylmethyl]-N-methyl-furan-2-carboxamid
4-Butoxy-N-[4'-(2,4-dioxo-thiazolidin-5-ylmethyl)-biphenyl-3-ylmethyl]-N-methyl-benzamid
N-[4'-(2,4-Dioxo-thiazolidin-5-ylmethyl)-biphenyl-3-ylmethyl]-N-methyl-thiophen-3-carboxamid
4-{[4'-(2,4-Dioxo-thiazolidin-5-ylmethyl)-biphenyl-3-ylmethyl]-methyl-carbamoyl}-phenyl-acetat
N-[4'-(2,4-Dioxo-thiazolidin-5-ylmethyl)-biphenyl-3-ylmethyl]-4-hydroxy-N-methyl-benzamid
N-[4'-(2,4-Dioxo-thiazolidin-5-ylmethyl)-biphenyl-3-ylmethyl]-2-N-dimethyl-benzamid
2-Butyl-N-[4'-(2,4-dioxo-thiazolidin-5-ylmethyl)-biphenyl-3-ylmethyl]-N-methyl-octanamid
4-Acetylamino-N-[4'-(2,4-dioxo-thiazolidin-5-ylmethyl)-biphenyl-3-ylmethyl]-N-methyl-benzamid
N-[4'-(2,4-Dioxo-thiazolidin-5-ylmethyl)-biphenyl-3-ylmethyl]-N-methyl-carbaminsäurehexylester
1-[4'-(2,4-Dioxo-thiazolidin-5-ylmethyl)-biphenyl-3-yl]-3-phenylharnstoff
N-[4'-(2,4-Dioxo-thiazolidin-5-ylmethyl)-biphenyl-3-yl]-N-methyl-2-phenyl-acetamid
1-[4'-(2,4-Dioxo-thiazolidin-5-ylmethyl)-biphenyl-3-yl]-1-methyl-3-phenyl-harnstoff
1-[4'-(2,4-Dioxo-thiazolidin-5-ylmethyl)-biphenyl-3-yl]-3-heptyl-1-methyl-harnstoff
2-{3'-[(Heptanoyl-methyl-amino)-methyl]-biphenyl-4-ylmethyl}bernsteinsäure-4-monomethylester
2-{3'-[(Methyl-octanoyl-amino)-methyl]-biphenyl-4-ylmethyl}bernsteinsäure
N-[4'-(2,5-Dioxo-pyrrolidin-3-ylmethyl)-biphenyl-3-ylmethyl]-N-methyl-heptanamid
3-[4'-(2,4-Dioxo-thiazolidin-5-ylidenmethyl)]-biphenyl-N-phenylcarbamat
3-[4'-(2,4-Dioxo-thiazolidin-5-ylidenmethyl)]-biphenyl-N-heptylcarbamat
3-[4'-(2,4-Dioxo-thiazolidin-5-ylmethyl)]-biphenyl-phenylacetat
3-[4'-(2,4-Dioxo-thiazolidin-5-ylmethyl)]-biphenyl-nonanoat
3-[4'-(2,4-Dioxo-thiazolidin-5-ylmethyl)]-biphenyl-N-heptylcarbamat
3-[4'-(2,4-Dioxo-thiazolidin-5-ylmethyl)]-biphenyl-N-phenylcarbamat
N-[6-Benzyloxy-4'-(2,4-dioxo-thiazolidin-5-ylidenmethyl)-biphenyl-3-ylmethyl]-N-methyloctanamid
N- [4'-(2,4-Dioxo-thiazolidin-5-ylmethyl)-6-hydroxy-biphenyl-3-ylmethyl]-N-methyloctanamid
N-[4-Benzyloxy-4'-(2,4-dioxo-thiazolidin-5-ylidenmethyl)-biphenyl-3 -ylmethyl]-N-methyloctanamid
N-[4"-(2,4-Dioxo-thiazolidin-5-ylmethyl)-[1,1';3',1"]terphenyl-5'ylmethyl]-N-methyloctanamid
N-[4'-(2,4-Dioxo-thiazolidin-5-ylmethyl)-2'-methyl-biphenyl-3-ylmethyl]-N-methyloctanamid
N-[4'-(2,4-Dioxo-thiazolidin-5-ylmethyl)-3'-methyl-biphenyl-3-ylmethyl]-N-methyloctanamid
(S)-2-Ethoxy-3-{3'-[(methyl-octanoyl-amino)-methyl]-biphenyl-4-yl}-propionsäure
1-[4'-(2,4-Dioxo-thiazolidin-5-ylmethyl)-biphenyl-3-yl]-1-ethyl-3-phenyl-harnstoff
N-[4'-(2,4-Dioxo-thiazolidin-5-ylmethyl)-biphenyl-3-yl]-N-methyldocanamid
N-[4'-(2,4-Dioxo-thiazolidin-5-ylinethyl)-biphenyl-3-yl]-N-methylnonanamid
N-[4'-(2,4-Dioxo-thiazolidin-5-ylmethyl)-biphenyl-3-yl]-N-methyl-2-(4-butoxy-phenyl)-acetamid
N-[4'-(2,4-Dioxo-thiazolidin-5-ylmethyl)-biphenyl-3-yl]-N-methyl-2-(4-methoxy-phenyl)-acetamid
N-[4'-(2,4-Dioxo-thiazolidin-5-ylmethyl)-biphenyl-3-yl]-N-methyl-2- (4-ethoxy-phenyl)-acetamid
N-[4'-(2,4-Dioxo-thiazolidin-5-ylmethyl)-biphenyl-3-yl]-N-methyl-2-(4-hydroxy-phenyl)-acetamid
1-[4'-(2,4-Dioxo-thiazolidin-5-ylmethyl)-biphenyl-3-yl]-1-methyl-3-(4-butoxy-phenyl)-harnstoff
1-[4'-(2,4-Dioxo-thiazolidin-5-ylmethyl)-biphenyl-3-yl]-1-methyl-3-(4-methoxy-phenyl)-harnstoff
1-[4'-(2,4-Dioxo-thiazolidin-5-ylmethyl)-biphenyl-3-yl]-1-methyl-3-(4-ethoxy-phenyl)-harnstoff
1-[4'-(2,4-Dioxo-thiazolidin-5-ylmethyl)-bipheny1-3-yl]-1-methyl-3-(4-hydroxyphenyl)-harnstoff
(S)-2-Ethoxy-3-(3'-{[methyl-(1-(4-butoxy)-phenylmethanoyl)-amino]-methyl}-biphenyl-4-yl)-propionsäure
(S)-2-Ethoxy-3-(3'-{[methyl-(1-(4-methoxy)-phenylmethanoyl)-amino]-methyl}-biphenyl-4-yl)-propionsäure
(S)-2-Ethoxy-3-(3'-{[methyl-(1-(4-ethoxy-phenyl)-methanoyl)-amino]-methyl}-biphenyl-4-yl)-propionsäure
(S)-2-Ethoxy-3-(3'-{[methyl-(1-(4-hydroxy-phenyl)-methanoyl)-amino]-methyl}-biphenyl-4-yl)-propionsäure
(S)-2-Phenoxy-3-(3'-{[methyl-(1-(4-butoxy-phenyl)-methanoyl)-amino]-methyl}-biphenyl-4-yl)-propionsäure
(S)-2-Phenoxy-3-(3'-{[methyl-(1-(4-methoxy-phenyl)-methanoyl)-amino]-methyl}-biphenyl-4-yl)-propionsäure
(S)-2-Phenoxy-3-(3'-{[methyl-(1-(4-ethoxy-phenyl)-methanoyl)-amino]-methyl}-biphenyl-4-yl)-propionsäure
(S)-2-Phenoxy-3-(3'-{[methyl-(1-(4-hydroxy-phenyl)-methanoyl)-amino]-methyl}-biphenyl-4-yl)-propionsäure
(S)-2-Phenoxy-3-(3'-{[methyl-(1-phenyl-methanoyl)-amino]-methyl}-biphenyl-4-yl)-propionsäure
(S)-2-Phenoxy-3-{3'-[(methyl-octanoyl-amino)-methyl]-biphenyl-4-yl}-propionsäure
5-{3'-[Methyl-(2-oxo-2-phenyl-ethyl)-amino]-biphenyl-4-ylmethyl}thiazolidin-2,4-dion
5- [3'- (Methyl-phenethyl-amino)-biphenyl-4-ylmethyl]-thiazolidin-2,4-dion
[4'-(2,4-Dioxo-thiazolidin-5-ylmethyl)-biphenyl-3-yl]-methylcarbaminsäurephenylester
[4'-(2,4-Dioxo-thiazolidin-5-ylmethyl)-biphenyl-3-yl]-methylcarbaminsäure-*t*-butylester.

11. Verbindungen nach Anspruch 1 oder 2, **dadurch gekennzeichnet, dass** sie mindestens eines der folgenden Merkmale aufweisen:
- R₁ bedeutet die Gruppe der Formel (a) oder die Gruppe der Formel (b), worin R₅ Hydroxy und R₆ OR₁₀ bedeuten,
- X bedeutet eine Verknüpfungsgruppe, die unter -CH₂-N(R₁₂)-CO-, -NR₁₂-(CO)-NR₁₃ und -NR₁₂-(CO)-CH₂- ausgewählt ist, wobei diese Verknüpfungsgruppen von links nach rechts oder umgekehrt zu lesen sind.

12. Kosmetische Zusammensetzung, **dadurch gekennzeichnet, dass** sie in einem physiologisch akzeptablen Träger mindestens eine der in einem der Ansprüche 1 bis 11 definierten Verbindungen enthält.

13. Zusammensetzung nach Anspruch 12, **dadurch gekennzeichnet, dass** die Konzentration an Verbindung(en) nach einem der Ansprüche 1 bis 11 im Bereich von 0,001 bis 3 Gew.-%, bezogen auf das Gesamtgewicht der Zusammensetzung, liegt.

14. Kosmetische Verwendung einer Zusammensetzung wie in Anspruch 12 oder 13 definiert zur Körper- oder Haarhygiene.

15. Verbindungen nach einem der Ansprüche 1 bis 11 als Arzneimittel.

16. Verwendung einer Verbindung nach einem der Ansprüche 1 bis 11 zur Herstellung einer Zusammensetzung zur Regulierung und/oder zur Wiederherstellung des Metabolismus der Hautlipide.

17. Verwendung einer Verbindung nach einem der Ansprüche 1 bis 11 zur Herstellung einer Zusammensetzung zur Behandlung von:
- dermatologischen Affektionen, die mit einer sich auf die Differenzierung und die Proliferation beziehenden Störung der Keratinisierung in Zusammenhang stehen, insbesondere bei Acne vulgaris, Komedonenakne, polymorpher Akne, Rosazea, nodulozystischen Aknen, Acne conglobata, senilen Aknen, sekundären Aknen, wie Sonnenakne, medikamentöse Akne oder Berufsakne,
- Ichthyosen, ichthyosiformen Zuständen, Darrier-Krankheit, palmoplantaren Keratodermien, Leukoplasien und leukoplasiformen Zuständen, Lichen der Haut oder der Schleimhäute (bukkal),
- dermatologischen Affektionen mit einer immunoallergischen entzündlichen Komponente mit oder ohne Störung der Zellproliferation, insbesondere bei Psoriasis der Haut, der Schleimhäute oder der Nägel, psoriatischem Rheuma, Hautatopie, wie Ekzem, respiratorischer Atopie oder Zahnfleischhypertrophie,
- benignen oder malignen Proliferationen der Haut oder der Epidermis nichtviralen oder viralen Ursprungs, insbesondere gewöhnlichen Warzen, Flachwarzen und warzenähnlichen Epidermodysplasien, oralen oder floriden Papillomatosen und Lymphom T,
- Proliferationen, die durch UV-Strahlung hervorgerufen werden können, insbesondere Epitheliome der unteren Zellschicht und spinozelluläre Epitheliome,
- präkanzerösen Hautläsionen, insbesondere Keratoakanthomen,
- Immundermatosen, insbesondere Lupus erythematoides,
- bullösen Immunerkrankungen,
- Collagenerkrankungen, insbesondere Sklerodermie,
- dermatologischen Affektionen oder allgemeinen Affektionen mit immunologischer Komponente,
- pathologischen Zuständen der Haut, die durch Exposition gegen UV-Strahlung hervorgerufen sind, lichtinduzierter oder alterungsbedingter Hautalterung oder von aktinischen Pigmentationen und Keratosen oder allen mit altersbedingter oder aktinischer Alterung verbundenen pathologischen Zuständen, insbesondere Xerose,
- Störungen der Talgbildungsfunktion, insbesondere Akne-Hyperseborrhoe oder einfacher Seborrhoe,
- Störungen der Narbenbildung oder Schwangerschaftsstreifen,
- Störungen der Pigmentierung, wie Hyperpigmentation, Melasma, Hyperpigmentation oder Vitiligo,
- Affektionen des Lipidstoffwechsels, wie Adipositas, Hyperlipidämie oder nicht insulinabhängigem Diabetes,
- entzündlichen Affektionen, wie Arthritis,
- kanzerösen oder präkanzerösen Zuständen,
- Alopezie unterschiedlicher Genese, insbesondere Alopezie aufgrund von Chemotherapie oder Bestrahlung,
- Störungen des Immunsystems, wie Asthma, Diabetes Typ 1, Plattensklerose oder anderen selektiven Dysfunktionen des Immunsystems,
- Affektionen des kardiovaskulären Systems, wie Arteriosklerose oder Bluthochdruck.

18. Pharmazeutische Zusammensetzung, **dadurch gekennzeichnet, dass** sie in einem physiologisch akzeptablen Träger mindestens eine der in einem der Ansprüche 1 bis 11 definierten Verbindungen enthält.

19. Zusammensetzung nach Anspruch 18, **dadurch gekennzeichnet, dass** die Konzentration an Verbindung(en) nach einem der Ansprüche 1 bis 11 im Bereich von 0,001 bis 10 Gew.-%, bezogen auf das Gesamtgewicht der Zusammensetzung, liegt.

20. Zusammensetzung nach Anspruch 18, **dadurch gekennzeichnet, dass** die Konzentration an Verbindung(en) nach einem der Ansprüche 1 bis 11 im Bereich von 0,01 bis 1 Gew.-%, bezogen auf das Gesamtgewicht der Zusammensetzung, liegt.

## Claims

1. Compounds, **characterized in that** they correspond to formula (I) below: 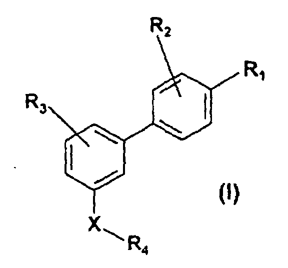 in which:
- **R**_{**1**} represents a radical of formula **(a)** or **(b)** below: 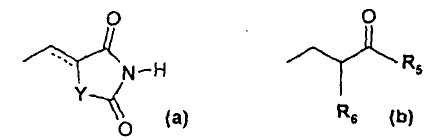 Y, R₅ and R₆ having the meanings given below,
- **R**_{**2**} and **R**_{**3**}**,** which may be identical or different, represent a hydrogen atom, an alkyl radical containing from 1 to 12 carbon atoms, an aryl radical, a halogen atom, a radical -OR₇, a polyether radical, a nitro radical or an amino radical which may optionally be substituted with one (or more) alkyl radical(s) containing from 1 to 12 carbon atoms,
R₇ having the meaning given below,
- **X** represents a structural linkage below:
-(CH₂)ₘ-(Z)ₙ-(CO)ₚ-(W)_{q}-
the said structural linkage being able to be read from left to right or vice versa,
Z, W, m, n, p and q having the meanings given below,
- **R**_{**4**} represents an alkyl radical containing from 1 to 12 carbon atoms, an aryl radical, an aralkyl radical, a heteroaryl radical or a 9-fluorenylmethyl radical,
- Y represents a CH₂ radical or a sulphur atom,
- R₅ represents a hydroxyl radical, an alkoxy radical containing from 1 to 6 carbon atoms, an NH-OH radical or a radical N(R₈)(R₉),
R₈ and R₉ having the meanings given below,
- R₆ represents an alkyl radical containing from 1 to 12 carbon atoms, a radical OR₁₀, a radical SR₁₀ or a radical (CH₂)ᵣ-COR₁₁,
r, R₁₀ and R₁₁ having the meanings given below,
- R₇ represents a hydrogen atom, an alkyl radical containing from 1 to 12 carbon atoms or an aralkyl radical,
- Z represents an oxygen or sulphur atom or a radical N-R₁₂,
R₁₂ having the meaning given below,
- W represents an oxygen or sulphur atom, a radical NR₁₃ or a CH₂ radical,
R₁₃ having the meaning given below,
- m, n, p and q, which may be identical or different, may take the values 0 or 1,
it being understood that the sum m+n+p+q is greater than or equal to 2 and that when p takes the value 0 then n or q is equal to 0,
- R₈ represents a hydrogen atom or an alkyl radical containing from 1 to 12 carbon atoms,
- R₉ represents a hydrogen atom, an alkyl radical containing from 1 to 12 carbon atoms or an aryl radical,
- r represents 0 or 1,
- R₁₀ represents an alkyl radical containing from 1 to 12 carbon atoms, an aryl radical or an aralkyl radical,
- R₁₁ represents a hydroxyl radical, a radical OR₁₄ or a radical N(R₁₅)(R₁₆),
- R₁₂ represents a hydrogen atom or an alkyl radical containing from 1 to 12 carbon atoms,
- R₁₃ represents a hydrogen atom or an alkyl radical containing from 1 to 12 carbon atoms,
- R₁₄ represents an alkyl radical containing from 1 to 12 carbon atoms, an aryl radical or an aralkyl radical,
- R₁₅ represents a hydrogen atom or an alkyl radical containing from 1 to 12 carbon atoms,
- R₁₆ represents a hydrogen atom, an alkyl radical containing from 1 to 12 carbon atoms, an aryl radical, an aralkyl radical or a heteroalkyl radical,
and also the optical and geometrical isomers of the said compounds of formula (I), and also the salts thereof.

2. Compounds according to Claim 1, **characterized in that** they are in the form of alkali metal or alkaline-earth metal salts, zinc salts or organoamine salts.

3. Compounds according to either of Claims 1 and 2, **characterized in that** the alkyl radicals containing from 1 to 12 carbon atoms are chosen from methyl, ethyl, isopropyl, butyl, tert-butyl, hexyl, octyl, decyl and dodecyl radicals.

4. Compounds according to either of Claims 1 and 2, **characterized in that** the polyether radicals are chosen from polyether radicals containing from 1 to 6 carbon atoms interrupted with at least one oxygen atom, such as methoxymethoxy, ethoxymethoxy or methoxyethoxymethoxy radicals.

5. Compounds according to either of Claims 1 and 2, **characterized in that** the halogen atom is chosen from the group consisting of a fluorine atom, a chlorine atom and a bromine atom.

6. Compounds according to either of Claims 1 and 2, **characterized in that** the alkoxy radical containing from 1 to 6 carbon atoms is chosen from the group consisting of methoxy, ethoxy, isopropyloxy, tert-butoxy and hexyloxy radicals.

7. Compounds according to either of Claims 1 and 2, **characterized in that** the aryl radical is chosen from a phenyl radical which may be monosubstituted or disubstituted with a halogen atom, a CF₃ radical, an alkyl radical containing from 1 to 12 carbon atoms, an alkoxy radical containing from 1 to 6 carbon atoms, a nitro function, a polyether radical, a hydroxyl radical optionally protected with an acetyl or benzoyl group or an amino function optionally protected with an acetyl or benzoyl group or optionally substituted with at least one alkyl containing from 1 to 12 carbon atoms.

8. Compounds according to either of Claims 1 and 2, **characterized in that** the aralkyl radical is chosen from a benzyl or phenethyl radical which may be monosubstituted or disubstituted with a halogen atom, a CF₃ radical, an alkyl radical containing from 1 to 12 carbon atoms, a hydroxyl radical, an alkoxy radical containing from 1 to 6 carbon atoms, a nitro function, a polyether radical, a hydroxyl radical optionally protected with an acetyl or benzoyl group or an amino function optionally protected with an acetyl or benzoyl group or optionally substituted with at least one alkyl containing from 1 to 12 carbon atoms.

9. Compounds according to either of Claims 1 and 2, **characterized in that** the heteroaryl radical is chosen from the group consisting of a pyridyl, furyl, thienyl or isoxazolyl radical optionally substituted with at least one halogen, an alkyl containing from 1 to 12 carbon atoms, an alkoxy containing from 1 to 6 carbon atoms, a nitro function, a polyether radical, a hydroxyl optionally protected with an acetyl or benzoyl group or an amino function optionally protected with an acetyl or benzoyl group or optionally substituted with at least one alkyl containing from 1 to 12 carbon atoms.

10. Compounds according to Claim 1, **characterized in that** they are taken, alone or as mixtures, from the group consisting of:
N-[4'-(2,4-Dioxothiazolidin-5-ylmethyl)biphenyl-3-ylmethyl]-N-methylbenzamide
5-{3'-[(Methylpyrid-2-ylamino)methyl]biphenyl-4-yl-methyl}thiazolidine-2,4-dione
N-[4'-(2,4-Dioxothiazolidin-5-ylmethyl)biphenyl-3-ylmethyl]benzamide
Ethyl 3-{3'-[(Benzoylmethylamino)methyl]biphenyl-4-yl}-2-methylpropionate
2-Methyl-3-(3'-{[methyl-(1-phenylmethanoyl)amino]-methyl)biphenyl-4-yl)propionic acid
N-[4'-(2-Carbamoylpropyl)biphenyl-3-ylmethyl]-N-methylbenzamide
N-Methyl-N-[4'-(2-phenylcarbamoylpropyl)biphenyl-3-ylmethyl]benzamide
N-[4'-(2-Hydroxycarbamoylpropyl)biphenyl-3-ylmethyl]-N-methylbenzamide
1-[4'-(2,4-Dioxothiazolidin-5-ylmethyl)biphenyl-3-ylmethyl]-3-phenylurea
1-[4'-(2,4-Dioxothiazolidin-5-ylmethyl)biphenyl-3-ylmethyl]-1-methyl-3-phenylurea
tert-Butyl [4'-(2,4-Dioxothiazolidin-5-ylmethyl)-biphenyl-3-ylmethyl]methylcarbamate
N-[4'-(2,4-Dioxothiazolidin-5-ylmethyl)biphenyl-3-ylmethyl]-N-methylnonanamide
(S)-2-Ethoxy-3-(3'-{[methyl-(1-phenylmethanoyl)amino]-methyl}biphenyl-4-yl)propionic acid
Monomethyl 2-(3'-{[methyl-(1-phenylmethanoyl)amino]-methyl}biphenyl-4-ylmethyl)malonate
Dimethyl 2-(3'-{[methyl-(1-phenylmethanoyl)amino]-methyl}biphenyl-4-ylmethyl)malonate
Methyl 2-(3'-{[methyl-(1-phenylmethanoyl)amino]-methyl}biphenyl-4-ylmethyl)malonamate
N-[4'-(2,4-Dioxothiazolidin-5-ylmethyl)biphenyl-3-ylmethyl]-N-ethylbenzamide
N-[4'-(2,4-Dioxothiazolidin-5-ylmethyl)biphenyl-3-ylmethyl]-N-pentylbenzamide
tert-Butyl [4'-(2,4-dioxothiazolidin-5-ylmethyl)-biphenyl-3-ylmethyl]ethylcarbamate
tert-Butyl [4'-(2,4-dioxothiazolidin-5-ylmethyl)-biphenyl-3-ylmethyl]propylcarbamate
9H-Fluoren-9-ylmethyl [4'-(2,4-dioxothiazolidin-5-ylmethyl)biphenyl-3-ylmethyl]methylcarbamate
N-[4'-(2,4-Dioxothiazolidin-5-ylmethyl)biphenyl-3-ylmethyl]-2,2,N-trimethylpropionamide
N-Octyl-4'-(2,4-dioxothiazolidin-5-ylmethyl)biphenyl-3-carboxamide
N-[4'-(2,4-Dioxothiazolidin-5-ylmethyl)biphenyl-3-ylmethyl]-N-methyl-3-phenylpropionamide
2-[4'-(2,4-Dioxothiazolidin-5-ylmethyl)biphenyl-3-yl]-N-methyl-N-phenylacetamide
N-[4'-(2,4-Dioxothiazolidin-5-ylidenemethyl)biphenyl-3-ylmethyl]-N-propylbenzamide
tert-Butyl [4'-(2,4-Dioxothiazolidin-5-ylmethyl)-biphenyl-3-yl]carbamate
N-[4'-(2,4-Dioxothiazolidin-5-ylmethyl)biphenyl-3-ylmethyl]-3,4-diethoxy-N-methylbenzamide
2-(3'-{[Methyl-(1-phenylmethanoyl)amino]methyl}-biphenyl-4-ylmethyl)malonamic acid
N-Benzyl-N-methyl-4'-(2,4-dioxothiazolidin-5-ylmethyl)-biphenyl-3-carboxamide
N-Benzyl-4'-(2,4-dioxothiazolidin-5-ylmethyl)biphenyl-3-carboxamide
N-[4'-(2,4-Dioxothiazolidin-5-ylmethyl)biphenyl-3-ylmethyl]-N-methyldecanamide
N-[4'-(2,4-Dioxothiazolidin-5-ylmethyl)biphenyl-3-yl]-2-phenylacetamide
N-[4'-(2,4-Dioxothiazolidin-5-ylmethyl)biphenyl-3-ylmethyl]-N-methyloctanamide
N-[4'-(2,4-Dioxothiazolidin-5-ylmethyl)biphenyl-3-ylmethyl]-N-methylheptanamide
N-Hydroxy-2-(3'-{[methyl-(1-phenylmethanoyl)amino]-methyl}biphenyl-4-ylmethyl)malonamic acid
N-[4'-(2,4-Dioxothiazolidin-5-ylmethyl)biphenyl-3-ylmethyl]-N-methyl-2-phenylacetamide
N-[4'-(2,4-Dioxothiazolidin-5-ylmethyl)biphenyl-3-ylmethyl]-4-methoxy-N-methylbenzamide
N-[4'-(2,4-Dioxothiazolidin-5-ylmethyl)biphenyl-3-ylmethyl]-3-methoxy-N-methylbenzamide
N-[4'-(2,4-Dioxothiazolidin-5-ylmethyl)biphenyl-3-ylmethyl]-3,N-dimethylbenzamide
N-[4'-(2,4-Dioxothiazolidin-5-ylmethyl)biphenyl-3-ylmethyl]-N-methyl-4-propylbenzamide
N-[4'-(2,4-Dioxothiazolidin-5-ylmethyl)biphenyl-3-ylmethyl]-4,N-dimethylbenzamide
N-[4'-(2,4-Dioxothiazolidin-5-ylmethyl)biphenyl-3-ylmethyl]-N-methylisoxazole-5-carboxamide
N-[4'-(2,4-Dioxothiazolidin-5-ylmethyl)biphenyl-3-ylmethyl]-4-ethoxy-N-methylbenzamide
N-[4'-(2,4-Dioxothiazolidin-5-ylmethyl)biphenyl-3-ylmethyl]-4-fluoro-N-methylbenzamide
4-Dimethylamino-N-[4'-(2,4-dioxothiazolidin-5-ylmethyl)biphenyl-3-ylmethyl]-N-methylbenzamide
N-[4'-(2,4-Dioxothiazolidin-5-ylmethyl)biphenyl-3-ylmethyl]-N-methylnicotinamide
3,5-Dichloro-N-[4'-(2,4-dioxothiazolidin-5-ylmethyl)-biphenyl-3-ylmethyl]-N-methylbenzamide
N-[4'-(2,4-Dioxothiazolidin-5-ylmethyl)biphenyl-3-ylmethyl]-N-methylthiophene-2-carboxamide
N-[4'-(2,4-Dioxothiazolidin-5-ylmethyl)biphenyl-3-ylmethyl]-N-methylhexanamide
N-[4'-(2,4-Dioxothiazolidin-5-ylmethyl)biphenyl-3-ylmethyl]-2-methoxy-N-methylbenzamide
N-[4'-(2,4-Dioxothiazolidin-5-ylmethyl)biphenyl-3-ylmethyl]-N-methylpyridine-2-carboxamide
N-[4'-(2,4-Dioxothiazolidin-5-ylmethyl)biphenyl-3-ylmethyl]-N-methylfuran-2-carboxamide
4-Butoxy-N-[4'-(2,4-dioxothiazolidin-5-ylmethyl)biphenyl-3-ylmethyl]-N-methylbenzamide
N-[4'-(2,4-Dioxothiazolidin-5-ylmethyl)biphenyl-3-ylmethyl]-N-methylthiophene-3-carboxamide
4-{[4'-(2,4-Dioxothiazolidin-5-ylmethyl)biphenyl-3-ylmethyl]methylcarbamoyl}phenyl acetate
N-[4'-(2,4-Dioxothiazolidin-5-ylmethyl)biphenyl-3-ylmethyl]-4-hydroxy-N-methylbenzamide
N-[4'-(2,4-Dioxothiazolidin-5-ylmethyl)biphenyl-3-ylmethyl]-2N-dimethylbenzamide
2-Butyl-N-[4'-(2,4-dioxothiazolidin-5-ylmethyl)-biphenyl-3-ylmethyl]-N-methyloctanamide
4-Acetylamino-N-[4'-(2,4-dioxothiazolidin-5-ylmethyl)-biphenyl-3-ylmethyl]-N-methylbenzamide
Hexyl N-[4'-(2,4-dioxothiazolidin-5-ylmethyl)biphenyl-3-ylmethyl]-N-methylcarbamate
1-[4'-(2,4-Dioxothiazolidin-5-ylmethyl)biphenyl-3-yl]-3-phenylurea
N-[4'-(2,4-Dioxothiazolidin-5-ylmethyl)biphenyl-3-yl]-N-methyl-2-phenylacetamide
1-[4'-(2,4-Dioxothiazolidin-5-ylmethyl)biphenyl-3-yl]-1-methyl-3-phenylurea
1-[4'-(2,4-Dioxothiazolidin-5-ylmethyl)biphenyl-3-yl]-3-heptyl-1-methylurea
4-Monomethyl ester of 2-{3'-[(heptanoylmethylamino)-methyl]biphenyl-4-ylmethyl}succinic acid
2-{3'-[(Methyloctanoylamino)methyl]biphenyl-4-yl-methyl}succinic acid
N-[4'-(2,5-Dioxopyrrolidin-3-ylmethyl)biphenyl-3-yl-methyl]-N-methylheptanamide
3-[4'-(2,4-Dioxothiazolidin-5-ylidenemethyl))biphenyl N-phenylcarbamate
3-[4'-(2,4-Dioxothiazolidin-5-ylidenemethyl)]biphenyl N-heptylcarbamate
3-[4'-(2,4-Dioxothiazolidin-5-ylmethyl)]biphenyl phenylacetate
3-[4'-(2,4-Dioxothiazolidin-5-ylmethyl)]biphenyl nonanoate
3-[4'-(2,4-Dioxothiazolidin-5-ylmethyl)]biphenyl N-heptylcarbamate
3-[4'-(2,4-Dioxothiazolidin-5-ylmethyl)]biphenyl N-phenylcarbamate
N-[6-Benzyloxy-4'-(2,4-dioxothiazolidin-5-ylidene-methyl)biphenyl-3-ylmethyl]-N-methyloctanamide
N-[4'-(2,4-Dioxothiazolidin-5-ylmethyl)-6-hydroxybiphenyl-3-ylmethyl]-N-methyloctanamide
N-[4-Benzyloxy-4'-(2,4-dioxothiazolidin-5-ylidene-methyl)biphenyl-3-ylmethyl]-N-methyloctanamide
N-[4"-(2,4-Dioxothiazolidin-5-ylmethyl)-[1,1';3',1"]terphenyl-5'-ylmethyl]-N-methyloctanamide
N-[4'-(2,4-Dioxothiazolidin-5-ylmethyl)-2'-methylbiphenyl-3-ylmethyl]-N-methyloctanamide
N-[4'-(2,4-Dioxothiazolidin-5-ylmethyl)-3'-methylbiphenyl-3-ylmethyl]-N-methyloctanamide
(S)-2-Ethoxy-3-(3'-[(methyloctanoylamino)methyl]-biphenyl-4-yl}propionic acid
1-[4'-(2,4-Dioxothiazolidin-5-ylmethyl)biphenyl-3-yl]-1-ethyl-3-phenylurea
N-[4'-(2,4-Dioxothiazolidin-5-ylmethyl)biphenyl-3-yl]-N-methyldecanamide
N-[4'-(2,4-Dioxothiazolidin-5-ylmethyl)biphenyl-3-yl]-N-methylnonanamide
N-[4'-(2,4-Dioxothiazolidin-5-ylmethyl)biphenyl-3-yl]-N-methyl-2-(4-butoxyphenyl)acetamide
N-[4'-(2,4-Dioxothiazolidin-5-ylmethyl)biphenyl-3-yl]-N-methyl-2-(4-methoxyphenyl)acetamide
N-[4'-(2,4-Dioxothiazolidin-5-ylmethyl)biphenyl-3-yl]-N-methyl-2-(4-ethoxyphenyl)acetamide
N-[4'-(2,4-Dioxothiazolidin-5-ylmethyl)biphenyl-3-yl]-N-methyl-2-(4-hydroxyphenyl)acetamide
1-[4'-(2,4-Dioxothiazolidin-5-ylmethyl)biphenyl-3-yl]-1-methyl-3-(4-butoxyphenyl)urea
1-[4'-(2,4-Dioxothiazolidin-5-ylmethyl)biphenyl-3-yl]-1-methyl-3-(4-methoxyphenyl)urea
1-[4'-(2,4-Dioxothiazolidin-5-ylmethyl)biphenyl-3-yl]-1-methyl-3-(4-ethoxyphenyl)urea
1-[4'-(2,4-Dioxothiazolidin-5-ylmethyl)biphenyl-3-yl]-1-methyl-3-(4-hydroxyphenyl)urea
(S)-2-Ethoxy-3-(3'-{[methyl-(1-(4-butoxy)phenyl-methanoyl)amino]methyl}biphenyl-4-yl)propionic acid
(S)-2-Ethoxy-3-(3'-{[methyl-(1-(4-methoxy)phenyl-methanoyl)amino]methyl}biphenyl-4-yl)propionic acid
(S)-2-Ethoxy-3-(3'-{[methyl-(1-(4-ethoxyphenyl)-methanoyl)amino]methyl}biphenyl-4-yl)propionic acid
(S)-2-Ethoxy-3-(3'-{[methyl-(1-(4-hydroxyphenyl)-methanoyl)amino]methyl}biphenyl-4-yl)propionic acid
(S)-2-Phenoxy-3-(3'-{[methyl-(1-(4-butoxyphenyl)-methanoyl)amino]methyl}biphenyl-4-yl)propionic acid
(S)-2-Phenoxy-3-(3'-{[methyl-(1-(4-methoxyphenyl)-methanoyl)amino]methyl}biphenyl-4-yl)propionic acid
(S)-2-Phenoxy-3-(3'-{[methyl-(1-(4-ethoxyphenyl)-methanoyl)amino]methyl}biphenyl-4-yl)propionic acid
(S)-2-Phenoxy-3-(3'-{[methyl-(1-(4-hydroxyphenyl)-methanoyl)amino}methyl)biphenyl-4-yl)propionic acid
(S)-2-Phenoxy-3-(3'-{[methyl-(1-phenylmethanoyl)amino]-methyl}biphenyl-4-yl)propionic acid
(S)-2-Phenoxy-3-{3'-[(methyloctanoylamino)methyl]-biphenyl-4-yl}propionic acid
5-{3'-[Methyl-(2-oxo-2-phenylethyl)amino]biphenyl-4-ylmethyl}thiazolidine-2,4-dione
5-[3'-(Methylphenethylamino)biphenyl-4-ylmethyl]-thiazolidine-2,4-dione
Phenyl [4'-(2,4-dioxothiazolidin-5-ylmethyl)biphenyl-3-yl]methylcarbamate
tert-Butyl [4'-(2,4-dioxothiazolidin-5-ylmethyl)-biphenyl-3-yl]methylcarbamate.

11. Compounds according to Claim 1 or 2,
**characterized in that** they have at least one of the following characteristics:
- R₁ represents the radical of formula (a) or the radical of formula (b) in which R₅ represents a hydroxyl radical and R₆ represents a radical OR₁₀,
- X represents a structural linkage chosen from -CH₂-N(R₁₂)-CO-, -NR₁₂-(CO)-NR₁₃- or -NR₁₂-(CO)-CH₂-, these linkages being read from left to right or vice versa.

12. Cosmetic composition, **characterized in that** it comprises, in a physiologically acceptable support, at least one of the compounds as defined in any one of Claims 1 to 11.

13. Composition according to Claim 12, **characterized in that** the concentration of compound(s) according to one of Claims 1 to 11 is between 0.001% and 3% by weight relative to the total weight of the composition.

14. Cosmetic use of a composition as defined in either of Claims 12 and 13 for body or hair hygiene.

15. Compounds according to any one of Claims 1 to 11 as medicinal products.

16. Use of a compound according to any one of Claims 1 to 11 in the manufacture of a composition for regulating and/or restoring skin lipid metabolism.

17. Use of a compound according to any one of Claims 1 to 11 in the manufacture of a composition intended for treating:
- dermatological complaints associated with a keratinization disorder relating to differentiation and to proliferation, in particular common acne, comedones, polymorphs, rosacea, nodulokystic acne, acne conglobata, senile acne and secondary acne such as solar, medicinal or occupational acne,
- ichthyosis, ichthyosiform conditions, Darrier's disease, palmoplantar keratoderma, leukoplakia and leukoplakiform conditions, and cutaneous or mucous (oral) lichen,
- dermatological complaints with an inflammatory immuno-allergic component, with or without a cellular proliferation disorder, and in particular cutaneous, mucous or ungual psoriasis, arthropathia psoriatica, or cutaneous atopy such as eczema, respiratory atopy or gingival hypertrophy,
- dermal or epidermal proliferations, whether benign or malignant, whether or not of viral origin, in particular common warts, flat warts and epidermodysplasia verruciformis, oral or florid papillomatoses, T lymphoma,
- proliferations which may be induced by ultraviolet light, in particular basal cell and prickle cell epithelioma,
- precancerous skin lesions, in particular keratoacanthomas,
- immune dermatites, in particular lupus erythematosus,
- bullous immune diseases,
- collagen diseases, in particular scleroderma,
- dermatological or systemic complaints with an immunological component,
- skin disorders due to exposure to UV radiation, light-induced or chronological ageing of the skin, actinic keratoses and pigmentations, or any pathology associated with chronological or actinic ageing, in particular xerosis,
- sebaceous function disorders, in particular the hyperseborrhoea of acne or simple seborrhoea,
- cicatrization disorders or stretch marks,
- pigmentation disorders, such as hyperpigmentation, melasma, hypopigmentation or vitiligo,
- lipid metabolism complaints, such as obesity, hyperlipidaemia or non-insulin-dependent diabetes,
- inflammatory complaints such as arthritis,
- cancerous or precancerous conditions,
- alopecia of various origins, in particular alopecia caused by chemotherapy or radiation,
- immune system disorders, such as asthma, type I sugar diabetes, multiple sclerosis or other selective dysfunctions of the immune system,
- complaints of the cardiovascular system, such as arteriosclerosis or hypertension.

18. Pharmaceutical composition, **characterized in that** it comprises, in a physiologically acceptable support, at least one of the compounds as defined in any one of Claims 1 to 11.

19. Composition according to Claim 18, **characterized in that** the concentration of compound(s) according to one of Claims 1 to 11 is between 0.001% and 10% by weight relative to the total weight of the composition.

20. Composition according to Claim 18,
**characterized in that** the concentration of compound(s) according to one of Claims 1 to 11 is between 0.01% and 1% by weight relative to the total weight of the composition.
